# EUROPÄISCHE PATENTSCHRIFT

(11) **EP 1 187 819 B1**
(45) Veröffentlichungstag und Bekanntmachung des Hinweises auf die Patenterteilung: **23.07.2003**
(21) Anmeldenummer: 00943866.4
(22) Anmeldetag: 23.06.2000
(51) Int. Cl.: C07D 273/00, C07D 285/16

(54) **N-SUBSTITUIERTE PERHYDRODIAZINE**
N-SUBSTITUTED PERHYDRO DIAZINE
PERHYDRODIAZINES A SUBSTITUTION N

(30) Priorität: 24.06.1999 DE 19928963
(43) Veröffentlichungstag der Anmeldung: 20.03.2002
(73) Patentinhaber: BASF AKTIENGESELLSCHAFT, 67056 Ludwigshafen (DE)
(72) Erfinder: HAMPRECHT, Gerhard, D-69469 Weinheim (DE); PUHL, Michael, D-68623 Lampertheim (DE); MENKE, Olaf, D-67317 Altleiningen (DE); REINHARD, Robert, D-67061 Ludwigshafen (DE); SAGASSER, Ingo, D-67125 Dannstadt (DE); ZAGAR, Cyrill, D-67061 Ludwigshafen (DE)
(74) Vertreter: Kinzebach, Werner, Dr.
(86) Internationale Anmeldenummer: EP0005792
(87) Internationale Veröffentlichungsnummer: WO01000600

(56) Entgegenhaltungen:
- WO-A-94/10173
- WO-A-95/06643
- US-A- 3 428 631
- CHEMICAL ABSTRACTS, vol. 97, no. 19, 8. November 1982 (1982-11-08) Columbus, Ohio, US; abstract no. 162944r, Seite 714; XP002149495 & Y. Y. SAMITOV ET AL.: ZH. ORG. KHIM., Bd. 18, Nr. 7, 1982, Seiten 1520-8,

## Beschreibung

Die vorliegende Erfindung betrifft in der 4-Position substituierte 1-Oxa- und 1-Thia-3,4-diazine, ein Verfahren zu ihrer Herstellung sowie ihre Verwendung in einem Verfahren zur Herstellung neuer, herbizid-wirksamer Verbindungen.

Aus der WO 94/10173 sind perhydrierte 1-Oxa-3,4-diazine der allgemeinen Formel A bekannt, worin Z für S, O, S=O oder SO₂ steht und Q einen C₆-C₁₄-aromatischen Rest bedeutet. Die Verbindungen A werden als Ausgangsstoffe für die Herstellung von Herbiziden mit Triazolin-2,5-dion-Struktur (Verbindungen B) eingesetzt.

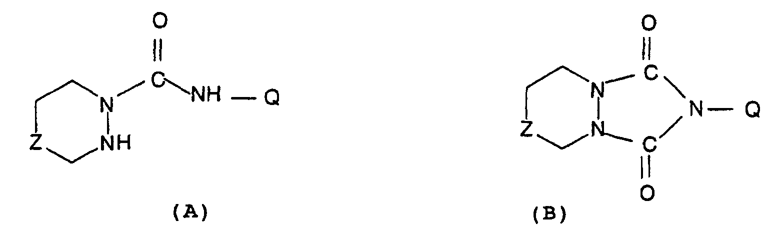

Die Verbindungen der Formel A werden ihrerseits durch Cyclisierung von substituierten N-(N'-Arylcarbamoyl)hydrazinoethanolen bzw. -thiolen mit Formaldehyd unter sauren Reaktionsbedingungen hergestellt. Die Ausbeuten derartiger Cyclisierungsreaktionen sind nicht zufriedenstellend.

In einer anderen Erfindung, die Gegenstand einer parallelen Anmeldung ist, wurde überraschenderweise gefunden, dass Verbindungen der allgemeinen Formel A', 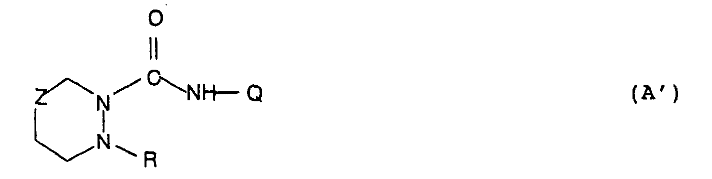 worin Z, X und Q die zuvor genannten Bedeutungen aufweisen und R für einen von Wasserstoff verschiedenen Rest steht, herbizide Wirkungen zeigen. Derartige Verbindungen sollten aus Perhydro-1-oxa- bzw. Perhydro-1-thia-3,4-diazinen, die in der 4-Position am Stickstoff substituiert sind, herstellbar sein. Der vorliegenden Erfindung lag somit die Aufgabe zugrunde, Oxa- bzw.

Thiadiazine bereitzustellen, die in der 4-Position am Stickstoff substituiert sind.

Es wurde überraschenderweise gefunden, dass 1-Oxa- und 1-Thia-3,4-diazine, die in der 4-Position am Stickstoff substituiert sind, mit guten Ausbeuten hergestellt werden können, wenn der in der 4-Position befindliche Substituent die im folgenden für R angegebenen Bedeutungen aufweist.

Die vorliegende Erfindung betrifft somit N-substituierte Perhydro-3,4-diazine der Formel I, 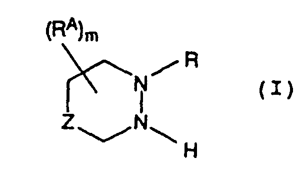 worin die Variablen Z, R, m und R^{A} die folgenden Bedeutungen haben:
- Z: O, S, S=O oder SO₂;
- R^{A}: Hydroxy, CO₂R¹, Halogen, Cyano, C(O)NR¹₂, OR², C₁-C₆-Alkyl, C₁-C₆-Halogenalkyl, C₃-C₆-Alkenyl, C₃-C₆-Alkinyl, COR¹, S(O)ₙR¹ mit n= 0, 1 oder 2, oder C(O)SR¹;
- R: CO₂H, CHO, CN, C(O)OR³, C(S)OR³, C(O)SR³, C(S)SR³, C(O)NR⁴R⁵, C(S)NR⁴R⁵, C(O)NHCO₂R⁶, C(O)NHSO₂R⁶, C(O)NHSO₃R⁶, C(O)R², P(O)R¹OR¹, P(O)(OR¹)₂, S(O)ₙR² mit n = 0, 1, oder 2 oder SO₂NHR¹;
- m: den Wert 0, 1, 2 oder 3;
und worin die Variablen R¹ bis R⁶ sowie das später genannte R⁷ die folgenden Bedeutungen aufweisen:
- R¹: Wasserstoff, C₁-C₆-Alkyl, C₁-C₆-Halogenalkyl, C₁-C₃-Alkoxy-C₁-C₃-alkyl, C₃-C₆-Alkenyl oder C₃-C₆-Alkinyl;
- R²: C₁-C₆-Alkyl, C₃-C₆-Cycloalkyl, C₃-C₆-Alkenyl, C₃-C₆-Alkinyl, C₁-C₆-Halogenalkyl, C₁-C₆-Alkoxy-C₁-C₆-alkyl, Hydroxycarbonyl-C₁-C₆-alkyl, C₁-C₆-Alkoxycarbonyl-C₁-C₆-alkyl, C₁-C₆-Akylthio-C₁-C₆-alkyl, C₁-C₆-Alkylsulfinyl-C₁-C₆-alkyl, C₁-C₆-Alkylsulfonyl-C₁-C₆-alkyl, C₃-C₆-Cycloalkyl-C₁-C₆-alkyl, C₃-C₆-Alkenyloxycarbonyl-C₁-C₆-alkyl, C₃-C₆-Alkinyloxycarbonyl-C₁-C₆-alkyl, C₃-C₆-Cycloalkoxy-C₁-C₆-alkyl, C₃-C₆-Alkenyloxy-C₁-C₆-alkyl, C₃-C₆-Alkinyloxy-C₁-C₆-alkyl, C₁-C₆-Halogenalkoxy-C₁-C₆-alkyl, C₃-C₆-Halogenalkenyloxy-C₁-C₆-alkyl, C₃-C₆-Halogenalkinyloxy-C₁-C₆-alkyl, C₃-C₆-Cycloalkyl-C₁-C₆-thioalkyl, C₃-C₆-Alkenylthio-C₁-C₆-alkyl, C₃-C₆-Alkinylthio-C₁-C₆-alkyl, Cyano-C₁-C₆-alkyl, C₃-C₆-Halogencycloalkyl-C₁-C₆-alkyl, Halogen-C₂-C₆-alkenyl, C₁-C₆-Alkoxy-C₃-C₆-alkenyl, C₁-C₆-Halogenalkoxy-C₃-C₆-alkenyl, C₁-C₆-Alkylthio-C₃-C₆-alkenyl, C₃-C₆-Halogenalkinyl, C₁-C₆-Alkoxy-C₃-C₆-alkinyl, C₁-C₆-Haloalkoxy-C₃-C₆-alkinyl, C₁-C₆-Alkylthio-C₃-C₆-alkinyl, C₁-C₆-Alkylcarbonyl, CHR¹COR⁷, CHR¹P(O)(OR⁷)₂, P(O)(OR⁷)₂, CHR¹P(S)(OR⁷)₂, CHR¹C(O)NR⁴R⁵, CHR¹C(O)NH₂,
Phenyl, Pyridyl, Benzyl, Phenoxy-C₁-C₆-alkyl, Benzyloxy-C₁-C₆-alkyl, wobei die Phenyl- bzw. Pyridylgruppen der fünf zuletzt genannten Substituenten mit Halogen, C₁-C₄-Alkyl, C₁-C₄-Alkoxy oder C₁-C₄-Halogenalkyl substituiert sein können,
- R³: C₁-C₁₅-Alkyl, C₃-C₈-Cycloalkyl, C₂-C₁₀-Alkenyl, C₃-C₁₀-Alkinyl, C₁-C₆-Halogenalkyl, C₁-C₆-Alkoxy-C₁-C₆-alkyl, C₁-C₆-Alkylthio-C₁-C₆-alkyl, C₁-C₆-Alkylsulfinyl-C₁-C₆-alkyl, C₁-C₆-Alkylsulfonyl-C₁-C₆-alkyl, C₁-C₃-Alkoxy-C₁-C₃-alkoxy-C₁-C₃-alkyl, C₃-C₆-Cycloalkyl-C₁-C₆-alkyl, Carboxyl-C₁-C₆-alkyl, C₁-C₆-Alkoxycarbonyl-C₁-C₆-alkyl, C₁-C₆-Alkoxycarbonyl-C₂-C₆-alkenyl, C₃-C₆-Alkenyloxycarbonyl-C₁-C₆-alkyl, C₃-C₆-Alkinyloxycarbonyl-C₁-C₆-alkyl, C₃-C₈-Cycloalkoxy-C₁-C₆-alkyl, C₃-C₆-Alkenyloxy-C₁-C₆-alkyl, C₃-C₆-Alkinyloxy-C₁-C₆-alkyl, C₁-C₆-Halogenalkoxy-C₁-C₆-alkyl, C₃-C₆-Halogenalkenyloxy-C₁-C₆-alkyl, C₃-C₆-Halogenalkinyloxy-C₁-C₆-alkyl, C₃-C₆-Cycloalkyl-C₁-C₆-thioalkyl, C₃-C₆-Alkenylthio-C₁-C₆-alkyl, C₃-C₆-Alkinylthio-C₁-C₆-alkyl, Cyano-C₁-C₆-alkyl, C₃-C₆-Halogencycloalkyl-C₁-C₆-alkyl, Halogen-C₃-C₆-alkenyl, C₁-C₆-Alkoxy-C₃-C₆-alkenyl, C₁-C₆-Halogenalkoxy-C₃-C₆-alkenyl, C₁-C₆-Alkylthio-C₃-C₆-alkenyl, C₃-C₆-Halogenalkinyl, C₁-C₆-Alkoxy-C₃-C₆-alkinyl, C₁-C₆-Halogenalkoxy-C₃-C₆-alkinyl, C₁-C₆-Alkylthio-C₃-C₆-alkinyl,
CHR¹COR⁷, CHR¹P(O)(OR⁷)₂, P(O)(OR⁷)₂, CHR¹P(S)(OR⁷)₂, CHR¹C(O)NR⁴R⁵, CHR¹C(O)NH₂, Phenoxy-C₁-C₆-alkyl, Benzyloxy-C₁-C₆-alkyl, wobei die Phenylgruppen der zwei zuletzt genannten Substituenten mit Halogen, C₁-C₄-Alkyl, C₁-C₄-Alk-oxy oder C₁-C₄-Halogenalkyl substituiert sein können;
Phenyl, Pyridyl, Naphthyl, Chinolyl, Chinazolyl, Chinoxalyl, 1-Methylindolyl, 1-Methylbenzimidazolyl, 2-Methylindazolyl, Benzofuranyl, Benzothienyl, Benzoxazolyl, Benzthiazolyl, Benzyl, wobei die 14 zuletzt genannten Substituenten in benachtbarten Positionen einen zweiwertigen Substituenten wie Methylendioxy, Difluormethylendioxy, Chlorfluormethylendioxy, Dichlormethylendioxy tragen können oder jeweils ein- bis fünffach durch Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Amino, C₁-C₄-Monoalkylamino, C₁-C₄-Dialkylamino, C₃-C₆-Cycloalkyl, C₁-C₄-Alkoxycarbonyl, C₁-C₄-Alkoxycarbonyl-C₁-C₄-alkyl, Hydroxy, Nitro oder Cyano substituiert sein können;
- R⁴: Wasserstoff, C₁-C₆-Alkyl oder C₁-C₆-Alkoxy;
- R⁵,: R⁶ unabhängig voneinander Wasserstoff, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy-C₁-C₆-alkyl, C₁-C₆-Alkoxycarbonyl-C₁-C₆-alkyl,
C₁-C₆-Alkoxycarbonyl-C₂-C₆-alkenyl, wobei die Alkenylkette zusätzlich ein bis drei Halogen- und/oder Cyanoreste tragen kann,
Benzyl, welches in benachbarten Positionen des Phenylrings einen zweiwertigen Substituenten wie Methylendioxy, Difluormethylendioxy, Chorfluormethylendioxy, Dichlormethylendioxy tragen kann oder ein- bis fünffach durch Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₃-C₆-Cycloalkyl, C₁-C₄-Alkoxycarbonyl, C₁-C₄-Alkoxycarbonyl-C₁-C₄-alkyl, Hydroxy, Nitro oder Cyano substitituiert sein kann, oder
- R⁴ und R⁵: zusammen mit dem Stickstoffatom, an das sie gebunden sind einen gesättigten oder ungesättigten 4- bis 7-gliedrigen Azaheterocyclus, der neben Kohlenstoffringgliedern gewünschtenfalls eines der folgenden Glieder enthalten kann: -O-, -S-, -N=, -NH- oder N-(C₁-C₆-Alkyl)-;
- R⁷: Wasserstoff, C₁-C₆-Alkyl, C₁-C₆-Halogenalkyl, C₁-C₃-Alkoxy-C₁-C₃-alkyl, C₂-C₆-Cyanoalkyl, C₃-C₆-Alkenyl oder C₃-C₆-Alkinyl.

Die bei der Definition von R, R^{A}, R¹ bis R⁷ sowie an Phenyl-, Cycloalkyl- und Heterocyclylringen genannten organischen Molekülteile stellen Sammelbegriffe für individuelle Aufzählungen der einzelnen Gruppenmitglieder dar. Sämtliche Kohlenstoffketten, also alle (gegebenenfalls substituierten) Alkyl-, Alkenyl- oder Alkinyl-Teile können geradkettig oder verzweigt sein.

Halogenierte Substituenten tragen vorzugsweise ein, zwei, drei, vier oder fünf gleiche oder verschiedene Halogenatome oder sind perhalogeniert.

Die Bedeutung Halogen steht jeweils für Fluor, Brom, Chlor oder Iod, insbesondere für Fluor oder Chlor.

Ferner stehen beispielsweise:
- C₁-C₄-Alkyl für: CH₃, C₂H₅, n-Propyl, CH(CH₃)₂, n-Butyl, CH(CH₃)-C₂H₅, 2-Methylpropyl oder C(CH₃)₃, insbesondere für CH₃, C₂H₅ oder CH(CH₃)₂;
- C₁-C₆-Alkyl für: C₁-C₄-Alkyl wie vorstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, insbesondere für CH₃, C₂H₅, n-Propyl, CH(CH₃)₂, n-Butyl, C(CH₃)₃, n-Pentyl oder n-Hexyl;
- C₁-C₁₅-Alkyl für: C₁-C₆-Alkyl wie vorstehend genannt, sowie z.B. n-Heptyl, 1-Methylhexyl, 2-Methylhexyl, 3-Methylhexyl, 4-Methylhexyl, 5-Methylhexyl, 1-Ethylpentyl, 2-Ethylpentyl, 3-Ethylpentyl, 4-Ethylpentyl, 1,1-Dimethylpentyl, 1,2-Dimethylpentyl, 1,3-Dimethylpentyl, 1,4-Dimethylpentyl, n-Octyl, 1-Methylheptyl, 2-Methylheptyl, 3-Methylheptyl, 4-Methylheptyl, 5-Methylheptyl, 6-Methylheptyl, 1-Ethylhexyl, 2-Ethylhexyl, 3-Ethylhexyl, 4-Ethylhexyl, 1,1-Dimethylhexyl, 1,2-Dimethylhexyl, 1,3-Dimethylhexyl, 1,4-Dimethylhexyl, 1,5-Dimethylhexyl, n-Nonyl, 1-Methyloctyl, 2-Methyloctyl, 3-Methyloctyl, 4-Methyloctyl, 5-Methyloctyl, 6-Methyloctyl, 7-Methyloctyl, 1-Ethylheptyl, 2-Ethylheptyl, 3-Ethylheptyl, 4-Ethylheptyl, 5-Ethylheptyl, 6-Ethylheptyl, 1,1-Dimethylheptyl, 1,2-Dimethylheptyl, 1,3-Dimethylheptyl, 1,4-Dimethylheptyl, 1,5-Dimethylheptyl, 1,6-Dimethylheptyl, n-Decyl, 1-Methylnonyl, 2-Methylnonyl, 3-Methylnonyl, 7-Methylnonyl, 8-Methylnonyl, 1-Ethyloctyl, 2-Ethyloctyl, 3-Ethyloctyl, 4-Ethyloctyl, 5-Ethyloctyl, 6-Ethyloctyl, 7-Ethyloctyl, 8-Ethyloctyl, 1,1-Dimethyloctyl, 1,2-Dimethyloctyl, 1,3-Dimethyloctyl, 1,4-Dimethyloctyl, 1,5-Dimethyloctyl, 1,6-Dimethyloctyl, 1,7-Dimethyloctyl, n-Undecyl, 1-Methyldecyl, 1-Ethylnonyl, 1,1-Dimethylnonyl, 1-Propyloctyl, 1-Butylheptyl, 6-Undecyl, n-Dodecyl, 1-Methylundecyl, 1-Ethyldecyl, 1,1-Dimethyldecyl, 1-Propylnonyl, 1-Butyloctyl, 7-Dodecyl, n-Tridecyl, 1-Methyldodecyl, 1-Ethylundecyl, 1,1-Dimethylundecyl, n-Tetradecyl, 1-Methyltridecyl, 1-Ethyldodecyl, 1,1-Dimethyldodecyl, n-Pentadecyl, 1-Mehtyltetradecyl, 1-Ethyltridecyl, 1,1-Dimethyltridecyl insbesondere für CH₃, C₂H₅, n-Propyl, CH(CH₃)₂, n-Butyl, C(CH₃)₃, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. CH₂F, CHF₂, CF₃, CH₂Cl, Dichlormethyl, Trichlormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorporpyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-brom-ethyl, 4-Fluorbutyl, 4-Chlorbutyl, 4-Brombutyl oder Nonafluorbutyl, insbesondere für CH₂F, CHF₂, CF₃, CH₂Cl, 2-Fluorethyl, 2-Chlorethyl oder 2,2,2-Trifluorethyl;
- C₁-C₆-Halogenalkyl für: C₁-C₆-Alkyl wie vorstehend genannt, das partiell oder vollständig durch Fluor, Chlor, Brom und/Iod substituiert ist, also z.B. einen der unter C₁-C₄-Halogenalkyl genannten Reste oder für 5-Fluor-1-pentyl, 5-Chlor-1-pentyl, 5-Brom-1-pentyl, 5-Iod-1-pentyl, 5,5,5-Trichlor-1-pentyl, Undecafluorpentyl, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl, insbesondere für Chlormethyl, Fluormethyl, Difluormethyl, Trifluormethyl, 2-Fluorethyl, 2-Chlorethyl oder 2,2,2-Trifluorethyl;
- Hydroxy-C₁-C₆-alkyl für: z.B. Hydroxymethyl, 2-Hydroxyeth-1-yl, 2-Hydroxy-prop-1-yl, 3-Hydroxy-prop-1-yl, 1-Hydroxyprop-2-yl, 2-Hydroxy-but-1-yl, 3-Hydroxy-but-1-yl, 4-Hydroxybut-1-yl, 1-Hydroxy-but-2-yl, 1-Hydroxy-but-3-yl, 2-Hydroxybut-3-yl, 1-Hydroxy-2-methyl-prop-3-yl, 2-Hydroxy-2-methylprop-3-yl oder 2-Hydroxymethyl-prop-2-yl, insbesondere für 2-Hydroxyethyl;
- Cyano-C₁-C₆-alkyl für: z.B. Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyanobut-3-yl, 1-Cyano-2-methyl-prop-3-yl, 2-Cyano-2-methylprop-3-yl, 3-Cyano-2-methyl-prop-3-yl oder 2-Cyanomethylprop-2-yl, insbesondere für Cyanomethyl oder 2-Cyanoethyl;
- Phenyl-C₁-C₆-alkyl für: z.B. Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Phenylmethyl)-eth-1-yl, 1-(Phenylmethyl)-1-(methyl)-eth-1-yl oder 1-(Phenylmethyl)-prop-1-yl, insbesondere für Benzyl oder 2-Phenylethyl;
- Phenyl-(C₁-C₆-alkyl)carbonyloxy für: z.B. Benzylcarbonyloxy, 1-Phenylethylcarbonyloxy, 2-Phenylethylcarbonyloxy, 1-Phenylprop-1-ylcarbonyloxy, 2-Phenylprop-1-ylcarbonyloxy, 3-Phenylprop-1-ylcarbonyloxy, 1-Phenylbut-1-ylcarbonyloxy, 2-Phenylbut-1-ylcarbonyloxy, 3-Phenylbut-1-ylcarbonyloxy, 4-Phenylbut-1-ylcarbonyloxy, 1-Phenylbut-2-ylcarbonyloxy, 2-Phenylbut-2-ylcarbonyloxy, 3-Phenylbut-2-ylcarbonyloxy, 4-Phenylbut-2-ylcarbonyloxy, 1-(Phenylmethyl)-eth-1-ylcarbonyloxy, 1-(Phenylmethyl)-1-(methyl)-eth-1-ylcarbonyloxy oder 1-(Phenylmethyl)-prop-1-ylcarbonyloxy, insbesondere für Benzylcarbonyloxy oder 2-Phenylethylcarbonyloxy;
- Phenyl-C₁-C₆-alkylsulfonyloxy für: z.B. Benzylsulfonyloxy, 1-Phenylethylsulfonyloxy, 2-Phenylethylsulfonyloxy, 1-Phenylprop-1-ylsulfonyloxy, 2-Phenylprop-1-ylsulfonyloxy, 3-Phenylprop-1-ylsulfonyloxy, 1-Phenylbut-1-ylsulfonyloxy, 2-Phenylbut-1-ylsulfonyloxy, 3-Phenylbut-1-ylsulfonyloxy, 4-Phenylbut-1-ylsulfonyloxy, 1-Phenylbut-2-ylsulfonyloxy, 2-Phenylbut-2-ylsulfonyloxy, 3-Phenylbut-2-ylsulfonyloxy, 4-Phenylbut-2-ylsulfonyloxy, 1-(Phenylmethyl)-eth-1-ylsulfonyloxy, 1-(Phenylmethyl)-1-(methyl)-eth-1-ylsulfonyloxy oder 1-(Phenylmethyl)-prop-1-ylsulfonyloxy, insbesondere für Benzylsulfonyloxy oder 2-Phenylethylsulfonyloxy;
- (C₁-C₆-Alkyl)carbonyl für: CO-CH₃, CO-C₂H₅,, n-Propylcarbonyl, 1-Methylethylcarbonyl, n-Butylcarbonyl, 1-Methylpropylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, n-Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl, insbesondere für CO-CH₃, CO-C₂H₅ oder CO-CH(CH₃)₂;
- (C₁-C₆-Alkyl)carbonyl-C₁-C₆-alkyl für: durch (C₁-C₆-Alkyl)-carbonyl wie vorstehend genannt substituiertes C₁-C₆-Alkyl, also z.B. für Methylcarbonylmethyl;
- (C₁-C₆-Halogenalkyl)carbonyl für: einen (C₁-C₆-Alkyl)carbonylrest wie vorstehend genannt, der partiell oder vollständig durch Fluor, Chlor, Brom und/oder Iod substituiert ist, also z.B. Chloracetyl, Dichloracetyl, Trichloracetyl, Fluoracetyl, Difluoracetyl, Trifluoracetyl, Chlorfluoracetyl, Dichlorfluoracetyl, Chlordifluoracetyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 2-Fluorpropylcarbonyl, 3-Fluorpropylcarbonyl, 2,2-Difluorpropylcarbonyl, 2,3-Difluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, 2,2,3,3,3-Pentafluorpropylcarbonyl, Heptafluorpropylcarbonyl, 1-(Fluormethyl)-2-fluorethylcarbonyl, 1-(Chlormethyl)-2-chlorethylcarbonyl, 1-(Brommethyl)-2-bromethylcarbonyl, 4-Fluorbutylcarbonyl, 4-Chlorbutylcarbonyl, 4-Brombutylcarbonyl, Nonafluorbutylcarbonyl, (5-Fluor-1-pentyl)carbonyl, (5-Chlor-1-pentyl)carbonyl, (5-Brom-1-pentyl)carbonyl, (5-Iod-1-pentyl)carbonyl, (5,5,5-Trichlor-1-pentyl)carbonyl, Undecafluorpentylcarbonyl, (6-Fluor-1-hexyl)carbonyl, (6-Chlor-1-hexyl)carbonyl, (6-Brom-1-hexyl)carbonyl, (6-Iod-1-hexyl)carbonyl, (6,6,6-Trichlor-1-hexyl)carbonyl oder Dodecafluorhexylcarbonyl, insbesondere für Trifluoracetyl;
- (C₁-C₆-Alkyl)carbonyloxy für: Acetyloxy, Ethylcarbonyloxy, n-Propylcarbonyloxy, 1-Methylethylcarbonyloxy, n-Butylcarbonyloxy, 1-Methylpropylcarbonyloxy, 2-Methylpropylcarbonyloxy, 1,1-Dimethylethylcarbonyloxy, n-Pentylcarbonyloxy, 1-Methylbutylcarbonyloxy, 2-Methylbutylcarbonyloxy, 3-Methylbutylcarbonyloxy, 1,1-Dimethylpropylcarbonyloxy, 1,2-Dimethylpropylcarbonyloxy, 2,2-Dimethylpropylcarbonyloxy, 1-Ethylpropylcarbonyloxy, n-Hexylcarbonyloxy, 1-Methylpentylcarbonyloxy, 2-Methylpentylcarbonyloxy, 3-Methylpentylcarbonyloxy, 4-Methylpentylcarbonyloxy, 1,1-Dimethylbutylcarbonyloxy, 1,2-Dimethylbutylcarbonyloxy, 1,3-Dimethylbutylcarbonyloxy, 2,2-Dimethylbutylcarbonyloxy, 2,3-Dimethylbutylcarbonyloxy, 3,3-Dimethylbutylcarbonyloxy, 1-Ethylbutylcarbonyloxy, 2-Ethylbutylcarbonyloxy, 1,1,2-Trimethylpropylcarbonyloxy, 1,2,2-Trimethylpropylcarbonyloxy, 1-Ethyl-1-methylpropylcarbonyloxy oder 1-Ethyl-2-methylpropylcarbonyloxy, insbesondere für Acetyloxy;
- (C₁-C₆-Halogenalkyl)carbonyloxy für: einen (C₁-C₆-Alkyl)-carbonyloxy-Rest wie vorstehend genannt, der partiell oder vollständig durch Fluor, Chlor, Brom und/oder Iod substituiert ist, also z.B. Chloracetyloxy, Dichloracetyloxy, Trichloracetyloxy, Fluoracetyloxy, Difluoracetyloxy, Trifluoracetyloxy, Chlorfluoracetyloxy, Dichlorfluoracetyloxy, Chlordifluoracetyloxy, 2-Fluorethylcarbonyloxy, 2-Chlorethylcarbonyloxy, 2-Bromethylcarbonyloxy, 2-Iodethylcarbonyloxy, 2,2-Difluorethylcarbonyloxy, 2,2,2-Trifluorethylcarbonyloxy, 2-Chlor-2-fluorethylcarbonyloxy, 2-Chlor-2,2-difluorethylcarbonyloxy, 2,2-Dichlor-2-fluorethylcarbonyloxy, 2,2,2-Trichlorethylcarbonyloxy, Pentafluorethylcarbonyloxy, 2-Fluorpropylcarbonyloxy, 3-Fluorpropylcarbonyloxy, 2,2-Difluorpropylcarbonyloxy, 2,3-Difluorpropylcarbonyloxy, 2-Chlorpropylcarbonyloxy, 3-Chlorpropylcarbonyloxy, 2,3-Dichlorpropylcarbonyloxy, 2-Brompropylcarbonyloxy, 3-Brompropylcarbonyloxy, 3,3,3-Trifluorpropylcarbonyloxy, 3,3,3-Trichlorpropylcarbonyloxy, 2,2,3,3,3-Pentafluorpropylcarbonyloxy, Heptafluorpropylcarbonyloxy, 1-(Fluormethyl)-2-fluorethylcarbonyloxy, 1-(Chlormethyl)-2-chlorethylcarbonyloxy, 1-(Brommethyl)-2-bromethylcarbonyloxy, 4-Fluorbutylcarbonyloxy, 4-Chlorbutylcarbonyloxy, 4-Brombutyl oder Nonafluorbutyl, insbesondere für Trifluoracetoxy;
- (C₁-C₆-Alkyl)carbonyloxy-C₁-C₆-alkyl für: durch (C₁-C₆-Alkyl)-carbonyloxy wie vorstehend genannt substituiertes C₁-C₆-Alkyl, also z.B. für Methylcarbonyloxymethyl, Ethylcarbonyloxymethyl, 1-(Methylcarbonyloxy)ethyl, 2-(Methylcarbonyloxy)-ethyl, 2-(Ethylcarbonyloxy)ethyl, 3-(Methylcarbonyloxy)-propyl, 4-(Methoxycarbonyloxy)butyl, 5-(Methoxycarbonyloxy)-pentyl oder 6-(Methoxycarbonyloxy)hexyl;
- (C₁-C₆-Alkyl)carbonylthio für Acetylthio, Ethylcarbonylthio, n-Propylcarbonylthio, 1-Methylethylcarbonylthio, n-Butylcarbonylthio, 1-Methylpropylcarbonylthio, 2-Methylpropylcarbonylthio, 1,1-Dimethylethylcarbonylthio, n-Pentylcarbonylthio, 1-Methylbutylcarbonylthio, 2-Methylbutylcarbonylthio, 3-Methylbutylcarbonylthio, 1,1-Dimethylpropylcarbonylthio, 1,2-Dimethylpropylcarbonylthio, 2,2-Dimethylpropylcarbonylthio, 1-Ethylpropylcarbonylthio, n-Hexylcarbonylthio, 1-Methylpentylcarbonylthio, 2-Methylpentylcarbonylthio, 3-Methylpentylcarbonylthio, 4-Methylpentylcarbonylthio, 1,1-Dimethylbutylcarbonylthio, 1,2-Dimethylbutylcarbonylthio, 1,3-Dimethylbutylcarbonylthio, 2,2-Dimethylbutylcarbonylthio, 2,3-Dimethylbutylcarbonylthio, 3,3-Dimethylbutylcarbonylthio, 1-Ethylbutylcarbonylthio, 2-Ethylbutylcarbonylthio, 1,1,2-Trimethylpropylcarbonylthio, 1,2,2-Trimethylpropylcarbonylthio, 1-Ethyl-1-methylpropylcarbonylthio oder 1-Ethyl-2-methylpropylcarbonylthio, insbesondere für Acetylthio;
- (C₁-C₆-Halogenalkyl)carbonylthio für: einen (C₁-C₆-Alkyl)-carbonylthio-Rest wie vorstehend genannt, der partiell oder vollständig durch Fluor, Chlor, Brom und/oder Iod substituiert ist, also z.B. Chloracetylthio, Dichloracetylthio, Trichloracetylthio, Fluoracetylthio, Difluoracetylthio, Trifluoracetylthio, Chlorfluoracetylthio, Dichlorfluoracetylthio, Chlordifluoracetylthio, 2-Fluorethylcarbonylthio, 2-Chlorethylcarbonylthio, 2-Bromethylcarbonylthio, 2-Iodethylcarbonylthio, 2,2-Difluorethylcarbonylthio, 2,2,2-Trifluorethylcarbonylthio, 2-Chlor-2-fluorethylcarbonylthio, 2-Chlor-2,2-difluorethylcarbonylthio, 2,2-Dichlor-2-fluorethylcarbonylthio, 2,2,2-Trichlorethylcarbonylthio, Pentafluorethylcarbonylthio, 2-Fluorpropylcarbonylthio, 3-Fluorpropylcarbonylthio, 2,2-Difluorpropylcarbonylthio, 2,3-Difluorpropylcarbonylthio, 2-Chlorpropylcarbonylthio, 3-Chlorpropylcarbonylthio, 2,3-Dichlorpropylcarbonylthio, 2-Brompropylcarbonylthio, 3-Brompropylcarbonylthio, 3,3,3-Trifluorpropylcarbonylthio, 3,3,3-Trichlorpropylcarbonylthio, 2,2,3,3,3-Pentafluorpropylcarbonylthio, Heptafluorpropylcarbonylthio, 1-(Fluormethyl)-2-fluorethylcarbonylthio, 1-(Chlormethyl)-2-chlorethylcarbonylthio, 1-(Brommethyl)-2-bromethylcarbonylthio, 4-Fluorbutylcarbonylthio, 4-Chlorbutylcarbonylthio, 4-Brombutylthio oder Nonafluorbutylthio, insbesondere für Trifluoracetylthio;
- (C₁-C₆-Alkyl)carbamoyloxy für: Methylcarbamoyloxy, Ethylcarbamoyloxy, n-Propylcarbamoyloxy, 1-Methylethylcarbamoyloxy, n-Butylcarbamoyloxy, 1-Methylpropylcarbamoyloxy, 2-Methylpropylcarbamoyloxy, 1,1-Dimethylethylcarbamoyloxy, n-Pentylcarbamoyloxy, 1-Methylbutylcarbamoyloxy, 2-Methylbutylcarbamoyloxy, 3-Methylbutylcarbamoyloxy, 1,1-Dimethylpropylcarbamoyloxy, 1,2-Dimethylpropylcarbamoyloxy, 2,2-Dimethylpropylcarbamoyloxy, 1-Ethylpropylcarbamoyloxy, n-Hexylcarbamoyloxy, 1-Methylpentylcarbamoyloxy, 2-Methylpentylcarbamoyloxy, 3-Methylpentylcarbamoyloxy, 4-Methylpentylcarbamoyloxy, 1,1-Dimethylbutylcarbamoyloxy, 1,2-Dimethylbutylcarbamoyloxy, 1,3-Dimethylbutylcarbamoyloxy, 2,2-Dimethylbutylcarbamoyloxy, 2,3-Dimethylbutylcarbamoyloxy, 3,3-Dimethylbutylcarbamoyloxy, 1-Ethylbutylcarbamoyloxy, 2-Ethylbutylcarbamoyloxy, 1,1,2-Trimethylpropylcarbamoyloxy, 1,2,2-Trimethylpropylcarbamoyioxy, 1-Ethyl-1-methylpropylcarbamoyloxy oder 1-Ethyl-2-methylpropylcarbamoyloxy, insbesondere für Methylcarbamoyloxy;
- (C₁-C₆-Halogenalkyl)carbamoyloxy für: einen (C₁-C₆-Alkyl)-carbamoyloxy-Rest wie vorstehend genannt, der partiell oder vollständig durch Fluor, Chlor, Brom und/oder Iod substituiert ist, also z.B. Chlormethylcarbamoyloxy, Dichlormethylcarbamoyloxy, Trichlormethylcarbamoyloxy, Fluormethylcarbamoyloxy, Difluormethylcarbamoyloxy, Trifluormethylcarbamoyloxy, Chlorfluormethylcarbamoyloxy, Dichlorfluormethylcarbamoyloxy, Chlordifluormethylcarbamoyloxy, 2-Fluorethylcarbamoyloxy, 2-Chlorethylcarbamoyloxy, 2-Bromethylcarbamoyloxy, 2-Iodethylcarbamoyloxy, 2,2-Difluorethylcarbamoyloxy, 2,2,2-Trifluorethylcarbamoyloxy, 2-Chlor-2-fluorethylcarbamoyloxy, 2-Chlor-2,2-difluorethylcarbamoyloxy, 2,2-Dichlor-2-fluorethylcarbamoyloxy, 2,2,2-Trichlorethylcarbamoyloxy, Pentafluorethylcarbamoyloxy, 2-Fluorpropylcarbamoyloxy, 3-Fluorpropylcarbamoyloxy, 2,2-Difluorpropylcarbamoyloxy, 2,3-Difluorpropylcarbamoyloxy, 2-Chlorpropylcarbamoyloxy, 3-Chlorpropylcarbamoyloxy, 2,3-Dichlorpropylcarbamoyloxy, 2-Brompropylcarbamoyloxy, 3-Brompropylcarbamoyloxy, 3,3,3-Trifluorpropylcarbamoyloxy, 3,3,3-Trichlorpropylcarbamoyloxy, 2,2,3,3,3-Pentafluorpropylcarbamoyloxy, Heptafluorpropylcarbamoyloxy, 1-(Fluormethyl)-2-fluorethylcarbamoyloxy, 1-(Chlormethyl)-2-chlorethylcarbamoyloxy, 1-(Brommethyl)-2-bromethylcarbamoyloxy, 4-Fluorbutylcarbamoyloxy, 4-Chlorbutylcarbamoyloxy, 4-Brombutylcarbamoyloxy oder Nonafluorbutylcarbamoyloxy, insbesondere für Trifluormethylcarbamoyloxy;
- C₁-C₆-Alkoxy für: z.B. OCH₃, OC₂H₅, OCH₂-C₂H₅, OCH(CH₃)₂, n-Butoxy, OCH(CH₃)-C₂H₅, OCH₂-CH(CH₃)₂, OC(CH₃)₃, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, insbesondere für OCH₃, OC₂H₅ oder OCH(CH₃)₂;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z.B. Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy, insbesondere für 2-Chlorethoxy oder 2,2,2-Trifluorethoxy;
- C₁-C₆-Halogenalkoxy für: einen C₁-C₆-Alkoxyrest wie vorstehend genannt, der partiell oder vollständig durch Fluor, Chlor, Brom und/oder Iod substituiert ist, also z.B. einen der unter C₁-C₄-Halogenalkoxy genannten Reste oder für 5-Fluor-1-pentoxy, 5-Chlor-1-pentoxy, 5-Brom-1-pentoxy, 5-Iod-1-pentoxy, 5,5,5-Trichlor-l-pentoxy, Undecafluorpentoxy, 6-Fluor-1-hexoxy, 6-Chlor-1-hexoxy, 6-Brom-1-hexoxy, 6-Iod-1-hexoxy, 6,6,6-Trichlor-1-hexoxy oder Dodecafluorhexoxy, insbesondere für Chlormethoxy, Fluormethoxy, Difluormethoxy, Trifluormethoxy, 2-Fluorethoxy, 2-Chlorethoxy oder 2,2,2-Trifluorethoxy;
- Hydroxy-C₁-C₆-alkoxy für: z.B. OCH₂-OH, OCH(CH₃)-OH, OCH₂-CH₂-OH, OCH(C₂H₅)-OH, OCH₂-CH(CH₃)-OH, 3-Hydroxyprop-1-yloxy, 1-Hydroxybut-1-yloxy, 2-Hydroxybut-1-yloxy, 3-Hydroxybut-1-yloxy, 4-Hydroxybut-1-yloxy, 1-Hydroxybut-2-yloxy, 2-Hydroxybut-2-yloxy, 3-Hydroxybut-2-yloxy, 4-Hydroxybut-2-yloxy, 1-(CH₂-OH)-eth-1-yloxy, 1-(CH₂-OH)-1-(CH₃)-eth-1-yloxy oder 1-(CH₂-OH)-prop-1-yloxy, insbesondere für OCH₂-OH oder OCH₂-CH₂-OH;
- Cyano-C₁-C₆-alkoxy für: z.B. OCH₂-CN, OCH(CH₃)-CN, OCH₂-CH₂-CN, OCH(C₂H₅)-OH, OCH₂-CH(CH₃)-CN, 3-Cyanoprop-1-yloxy, 1-Cyanobut-1-yloxy, 2-Cyanobut-1-yloxy, 3-Cyanobut-1-yloxy, 4-Cyanobut-1-yloxy, 1-Cyanobut-2-yloxy, 2-Cyanobut-2-yloxy, 3-Cyanobut-2-yloxy, 4-Cyanobut-2-yloxy, 1-(CH₂-CN)-eth-1-yloxy, 1-(CH₂-CN)-1-(CH₃)-eth-1-yloxy oder 1-(CH₂-CN)-prop-1-yloxy, insbesondere für OCH₂-CN oder OCH₂-CH₂-CN;
- Phenyl-C₁-C₆-alkoxy für: z.B. Benzyloxy, 1-Phenylethoxy, 2-Phenylethoxy, 1-Phenylprop-1-yloxy, 2-Phenylprop-1-yloxy, 3-Phenylprop-1-yloxy, 1-Phenylbut-1-yloxy, 2-Phenylbut-1-yloxy, 3-Phenylbut-1-yloxy, 4-Phenylbut-1-yloxy, 1-Phenylbut-2-yloxy, 2-Phenylbut-2-yloxy, 3-Phenylbut-2-yloxy, 4-Phenylbut-2-yloxy, 1-(Benzyl)-eth-1-yloxy, 1-(Benzyl)-1-(methyl)-eth-1-yloxy oder 1-(Benzyl)-prop-1-yloxy, insbesondere für Benzyloxy oder 2-Phenylethoxy;
- Phenyl-C₁-C₆-alkylthio für: z.B. Benzylthio, 1-Phenylethylthio, 2-Phenylethylthio, 1-Phenylprop-1-ylthio, 2-Phenylprop-1-ylthio, 3-Phenylprop-1-ylthio, 1-Phenylbut-1-ylthio, 2-Phenylbut-1-ylthio, 3-Phenylbut-1-ylthio, 4-Phenylbut-1-ylthio, 1-Phenylbut-2-ylthio, 2-Phenylbut-2-ylthio, 3-Phenylbut-2-ylthio, 4-Phenylbut-2-ylthio, l-(Phenylmethyl)-eth-1-ylthio, 1-(Phenylmethyl)-1-(methyl)-eth-1-ylthio oder 1-(Phenylmethyl)-prop-1-ylthio, insbesondere für Benzylthio oder 2-Phenylethylthio;
- (C₁-C₆-Alkoxy)carbonyl für: z.B. CO-OCH₃, CO-OC₂H₅, CO-OCH₂-C₂H₅, CO-OCH(CH₃)₂, n-Butoxycarbonyl, CO-OCH(CH₃)-C₂H₅, CO-OCH₂-CH(CH₃)₂, CO-OC(CH₃)₃, n-Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, n-Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl, insbesondere für CO-OCH₃, CO-OC₂H₅, CO-OCH(CH₃)₂ oder CO-OCH₂-CH(CH₃)₂;
- (C₁-C₆-Alkoxy)carbonyloxy für: Methoxycarbonyloxy, Ethoxycarbonyloxy, n-Propoxycarbonyloxy, 1-Methylethoxycarbonyloxy, n-Butoxycarbonyloxy, 1-Methylpropoxycarbonyloxy, 2-Methylpropoxycarbonyloxy, 1,1-Dimethylethoxycarbonyloxy, n-Pentoxycarbonyloxy, 1-Methylbutoxycarbonyloxy, 2-Methylbutoxycarbonyloxy, 3-Methylbutoxycarbonyloxy, 2,2-Dimethylpropoxycarbonyloxy, 1-Ethylpropoxycarbonyloxy, n-Hexoxycarbonyloxy, 1,1-Dimethylpropoxycarbonyloxy, 1,2-Dimethylpropoxycarbonyloxy, 1-Methylpentoxycarbonyloxy, 2-Methylpentoxycarbonyloxy, 3-Methylpentoxycarbonyloxy, 4-Methylpentoxycarbonyloxy, 1,1-Dimethylbutoxycarbonyloxy, 1,2-Dimethylbutoxycarbonyloxy, 1,3-Dimethylbutoxycarbonyloxy, 2,2-Dimethylbutoxycarbonyloxy, 2,3-Dimethylbutoxycarbonyloxy, 3,3-Dimethylbutoxycarbonyloxy, 1-Ethylbutoxycarbonyloxy, 2-Ethylbutoxycarbonyloxy, 1,1,2-Trimethylpropoxycarbonyloxy, 1,2,2-Trimethylpropoxycarbonyloxy, 1-Ethyl-1-methyl-propoxycarbonyloxy oder 1-Ethyl-2-methyl-propoxycarbonyloxy, insbesondere für Methoxycarbonyloxy, Ethoxycarbonyloxy oder 1-Methylethoxycarbonyloxy;
- (C₁-C₆-Alkoxy)carbonylthio für: Methoxycarbonylthio, Ethoxycarbonylthio, n-Propoxycarbonylthio, 1-Methylethoxycarbonylthio, n-Butoxycarbonylthio, 1-Methylpropoxycarbonylthio, 2-Methylpropoxycarbonylthio, 1,1-Dimethylethoxycarbonylthio, n-Pentoxycarbonylthio, 1-Methylbutoxycarbonylthio, 2-Methylbutoxycarbonylthio, 3-Methylbutoxycarbonylthio, 2,2-Dimethylpropoxycarbonylthio, 1-Ethylpropoxycarbonylthio, n-Hexoxycarbonylthio, 1,1-Dimethylpropoxycarbonylthio, 1,2-Dimethylpropoxycarbonylthio, 1-Methylpentoxycarbonylthio, 2-Methylpentoxycarbonylthio, 3-Methylpentoxycarbonylthio, 4-Methylpentoxycarbonylthio, 1,1-Dimethylbutoxycarbonylthio, 1,2-Dimethylbutoxycarbonylthio, 1,3-Dimethylbutoxycarbonylthio, 2,2-Dimethylbutoxycarbonylthio, 2,3-Dimethylbutoxycarbonylthio, 3,3-Dimethylbutoxycarbonylthio, 1-Ethylbutoxycarbonylthio, 2-Ethylbutoxycarbonylthio, 1,1,2-Trimethylpropoxycarbonylthio, 1,2,2-Trimethylpropoxycarbonylthio, 1-Ethyl-1-methyl-propoxycarbonylthio oder 1-Ethyl-2-methyl-propoxycarbonylthio, insbesondere für Methoxycarbonylthio, Ethoxycarbonylthio oder 1-Methylethoxycarbonylthio;
- C₁-C₆-Alkylthio für: SCH₃, SC₂H₅, SCH₂-C₂H₅, SCH(CH₃)₂, n-Butylthio, 1-Methylpropylthio, 2-Methylpropylthio, SC(CH₃)₃, n-Pentylthio, 1-Methylbutylthio, 2-Methylbutylthio, 3-Methylbutylthio, 2,2-Dimethylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, insbesondere für SCH₃ oder SC₂H₅;
- C₁-C₆-Halogenalkylthio für: C₁-C₆-Alkylthio wie vorstehend genannt, das partiell oder vollständig durch Fluor, Chlor, Brom und/oder Iod substituiert ist, also z.B. für SCHF₂, SCF₃, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, SC₂F₅, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio, Nonafluorbutylthio, 5-Fluorpentylthio, 5-Chlorpentylthio, 5-Brompentylthio, 5-Iodpentylthio, Undecafluorpentylthio, 6-Fluorhexylthio oder 6-Chlorhexylthio, insbesondere für SCH₂F, SCHF₂, SCF₃, SCH₂Cl, 2-Fluorethylthio, 2-Chlorethylthio oder 2,2,2-Trifluorethylthio;
- C₁-C₆-Alkylsulfinyl für: SO-CH₃, SO-C₂H₅, n-Propylsulfinyl, 1-Methylethylsulfinyl, n-Butylsulfinyl, 1-Methylpropylsulfinyl, 2-Methylpropylsulfinyl, 1,1-Dimethylethylsulfinyl, n-Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, n-Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl, insbesondere für SO-CH₃;
- C₁-C₆-Alkylsulfonyl für: SO₂-CH₃, SO₂-C₂H₅, n-Propylsulfonyl, SO₂-CH(CH₃)₂, n-Butylsulfonyl, 1-Methylpropylsulfonyl, 2-Methyipropylsulfonyl, SO₂-C(CH₃)₃, n-Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, n-Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl, insbesondere für SO₂-CH₃;
- C₁-C₆-Alkylsulfonyloxy für: O-SO₂-CH₃, O-SO₂-C₂H₅, n-Propylsulfonyloxy, O-SO₂-CH(CH₃)₂, n-Butylsulfonyloxy, 1-Methylpropylsulfonyloxy, 2-Methylpropylsulfonyloxy, O-SO₂-C(CH₃)₃, n-Pentylsulfonyloxy, 1-Methylbutylsulfonyloxy, 2-Methylbutylsulfonyloxy, 3-Methylbutylsulfonyloxy, 1,1-Dimethylpropylsulfonyloxy, 1,2-Dimethylpropylsulfonyloxy, 2,2-Dimethylpropylsulfonyloxy, 1-Ethylpropylsulfonyloxy, n-Hexylsulfonyloxy, 1-Methylpentylsulfonyloxy, 2-Methylpentylsulfonyloxy, 3-Methylpentylsulfonyloxy, 4-Methylpentylsulfonyloxy, 1,1-Dimethylbutylsulfonyloxy, 1,2-Dimethylbutylsulfonyloxy, 1,3-Dimethylbutylsulfonyloxy, 2,2-Dimethylbutylsulfonyloxy, 2,3-Dimethylbutylsulfonyloxy, 3,3-Dimethylbutylsulfonyloxy, 1-Ethylbutylsulfonyloxy, 2-Ethylbutylsulfonyloxy, 1,1,2-Trimethylpropylsulfonyloxy, 1,2,2-Trimethylpropylsulfonyloxy, 1-Ethyl-1-methylpropylsulfonyloxy oder 1-Ethyl-2-methylpropylsulfonyloxy, insbesondere für Methylsulfonyloxy;
- C₁-C₆-Halogenalkylsulfonyloxy für: C₁-C₆-Alkylsulfonyloxy wie vorstehend genannt, das partiell oder vollständig durch Fluor, Chlor, Brom und/oder Iod substituiert ist, also z.B. ClCH₂-SO₂-O-, CH(Cl)₂-SO₂-O-, C(Cl)₃-SO₂-O-, FCH₂-SO₂-O-, CHF₂-SO₂-O-, CF₃-SO₂-O-, Chlorfluormethyl-SO₂-O-, Dichlorfluormethyl-SO₂-O-, Chlordifluormethyl-SO₂-O-, 1-Fluorethyl-SO₂-O-, 2-Fluorethyl-SO₂-O-, 2-Chlorethyl-SO₂-O-, 2-Bromethyl-SO₂-O-, 2-Iodethyl-SO₂-O-, 2,2-Difluorethyl-SO₂-O-, 2,2,2-Trifluorethyl-SO₂-O-, 2-Chlor-2-fluorethyl-SO₂-O-, 2-Chlor-2,2-difluorethyl-SO₂-O-, 2,2-Dichlor-2-fluorethyl-SO₂-O-, 2,2,2-Trichlorethyl-SO₂-O-, C₂F₅-SO₂-O-, 2-Fluorpropyl-SO₂-O-, 3-Fluorpropyl-SO₂-O-, 2,2-Difluorpropyl-SO₂-O-, 2,3-Difluorpropyl-SO₂-O-, 2-Chlorpropyl-SO₂-O-, 3-Chlorpropyl-SO₂-O-, 2,3-Dichlorpropyl-SO₂-O-, 2-Brompropyl-SO₂-O-, 3-Brompropyl-SO₂-O-, 3,3,3-Trifluorpropyl-SO₂-O-, 3,3,3-Trichlorpropyl-SO₂-O-, 2,2,3,3,3-Pentafluorpropyl-SO₂-O-, C₂F₅-CF₂-SO₂-O-, 1-(Fluormethyl)-2-fluorethyl-SO₂-O-, 1-(Chlormethyl)-2-chlorethyl-SO₂-O-, 1-(Brommethyl)-2-bromethyl-SO₂-O-, 4-Fluorbutyl-SO₂-O-, 4-Chlorbutyl-SO₂-O-, 4-Brombutyl-SO₂-O-, C₂F₅-CF₂-CF₂-SO₂-O-, 5-Fluorpentyl-SO₂-O-, 5-Chlorpentyl-SO₂-O-, 5-Brompentyl-SO₂-O-, 5-Iodpentyl-SO₂-O-, 5,5,5-Trichlorpentyl-SO₂-O-, C₂F₅-CF₂-CF₂-CF₂-SO₂-O-, 6-Fluorhexyl-SO₂-O-, 6-Chlorhexyl-SO₂-O-, 6-Bromhexyl-SO₂-O-, 6-Iodhexyl-SO₂-O-, 6,6,6-Trichlorhexyl-SO₂-O- oder Dodecafluorhexyl-SO₂-O-, insbesondere für CF₃-SO₂-O-;
- (C₁-C₆-Alkyl)aminocarbonyl für: (C₁-C₄-Alkyl)aminocarbonyl wie vorstehend genannt sowie z.B. n-Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, n-Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl, insbesondere für CO-NH-CH₃, CO-NH-C₂H₅ oder CO-NH-CH(CH₃)₂;
- Di(C₁-C₆-alkyl)aminocarbonyl für: z.B. N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)-aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)-aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl, insbesondere für N,N-Dimethylaminocarbonyl oder N,N-Diethylaminocarbonyl;
- (C₁-C₆-Alkyl)iminooxycarbonyl für: Methyliminooxycarbonyl, Ethyliminooxycarbonyl, n-Propyliminooxycarbonyl, 1-Methylethyliminooxycarbonyl, n-Butyliminooxycarbonyl, 1-Methylpropyliminooxycarbonyl, 2-Methylpropyliminooxycarbonyl, 1,1-Dimethylethyliminooxycarbonyl, n-Pentyliminooxycarbonyl, 1-Methylbutyliminooxycarbonyl, 2-Methylbutyliminooxycarbonyl, 3-Methylbutyliminooxycarbonyl, 1,1-Dimethylpropyliminooxycarbonyl, 1,2-Dimethylpropyliminooxycarbonyl, 2,2-Dimethylpropyliminooxycarbonyl, 1-Ethylpropyliminooxycarbonyl, n-Hexyliminooxycarbonyl, 1-Methylpentyliminooxycarbonyl, 2-Methylpentyliminooxycarbonyl, 3-Methylpentyliminooxycarbonyl, 4-Methylpentyliminooxycarbonyl, 1,1-Dimethylbutyliminooxycarbonyl, 1,2-Dimethylbutyliminooxycarbonyl, 1,3-Dimethylbutyliminooxycarbonyl, 2,2-Dimethylbutyliminooxycarbonyl, 2,3-Dimethylbutyliminooxycarbonyl, 3,3-Dimethylbutyliminooxycarbonyl, 1-Ethylbutyliminooxycarbonyl, 2-Ethylbutyliminooxycarbonyl, 1,1,2-Trimethylpropyliminooxycarbonyl, 1,2,2-Trimethylpropyliminooxycarbonyl, 1-Ethyl-1-methylpropyliminooxycarbonyl oder 1-Ethyl-2-methylpropyliminooxycarbonyl, insbesondere für Methyliminooxycarbonyl, Ethyliminooxycarbonyl oder 1-Methylethyliminooxycarbonyl;
- C₁-C₆-Alkylidenaminoxy für: Acetylidenaminoxy, 1-Propylidenaminoxy, 2-Propylidenaminoxy, 1-Butylidenaminoxy, 2-Butylidenaminoxy oder 2-Hexylidenaminoxy, insbesondere für Acetylidenaminoxy oder 2-Propylidenaminoxy;
- C₁-C₆-Alkyliminooxy für: Methyliminooxy, Ethyliminooxy, n-Propyliminooxy, 1-Methylethyliminooxy, n-Butyliminooxy, 1-Methylpropyliminooxy, 2-Methylpropyliminooxy, n-Pentyliminooxy, n-Hexyliminooxy, 1-Methylpentyliminooxy, 2-Methylpentyliminooxy, 3-Methylpentyliminooxy oder 4-Methylpentyliminooxy, insbesondere für Methyliminooxy, Ethyliminooxy oder 1-Methylethyliminooxy;
- C₁-C₆-Alkoxy-(C₁-C₆-alkyl)aminocarbonyl für: (C₁-C₆-Alkyl)-aminocarbonyl wie CO-NH-CH₃, CO-NH-C₂H₅, CO-NH-CH₂-C₂H₅, CO-NH-CH(CH₃)₂, CO-NH-(CH₂)₃-CH₃, CO-NH-CH(CH₃)-C₂H₅, CO-NH-CH₂-CH(CH₃)₂, CO-NH-C(CH₃)₃, CO-NH-(CH₂)₄-CH₃, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, n-Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl und 1-Ethyl-2-methylpropylaminocarbonyl, vorzugsweise (C₁-C₄-Alkyl)aminocarbonyl, das durch C₁-C₆-Alkoxy - wie vorstehend genannt - substituiert ist, also z.B. für CO-NH-CH₂-OCH₃ oder CO-NH-CH₂-OC₂H₅;
- C₁-C₆-Alkoxyamino-C₁-C₆-alkyl für: z.B. CH₂-NH-OCH₃, CH₂-NH-OC₂H₅, CH₂-NH-OCH₂-C₂H₅, CH₂-NH-OCH(CH₃)₂, CH₂-NH-OCH₂-CH₂-C₂H₅, CH₂-NH-OCH(CH₃)-C₂H₅, CH₂-NH-OCH₂-CH(CH₃)₂, CH₂-NH-OC(CH₃)₃, CH₂-NH-OCH₂-(CH₂)₃-CH₃, (1-Methylbutoxyamino)methyl, (2-Methylbutoxyamino)methyl, (3-Methylbutoxyamino)methyl, (2,2-Dimethylpropoxyamino)methyl, (1-Ethylpropoxyamino)methyl, n-Hexoxyamino-methyl, (1,1-Dimethylpropoxyamino)methyl, (1,2-Dimethylpropoxyamino)-methyl, (1-Methylpentoxyamino)methyl, (2-Methylpentoxyamino)methyl, (3-Methylpentoxyamino)methyl, (4-Methylpentoxyamino)methyl, (1,1-Dimethylbutoxyamino)methyl, (1,2-Dimethylbutoxyamino)methyl, (1,3-Dimethylbutoxyamino)methyl, (2,2-Dimethylbutoxyamino)methyl, (2,3-Dimethylbutoxyamino)methyl, (3,3-Dimethylbutoxyamino)methyl, (1-Ethylbutoxyamino)methyl, (2-Ethylbutoxyamino)methyl, (1,1,2-Trimethylpropoxyamino)-methyl, (1,2,2-Trimethylpropoxyamino)methyl, (1-Ethyl-1-methylpropoxyamino)methyl, (1-Ethyl-2-methylpropoxyamino)-methyl, Methoxyamino-ethyl, Ethoxyamino-ethyl, n-Propoxyamino-ethyl, (1-Methylethoxyamino)ethyl, n-Butoxyamino-ethyl, (1-Methylpropoxyamino)ethyl, (2-Methylpropoxyamino)ethyl, (1,1-Dimethylethoxyamino)ethyl, n-Pentoxyaminoethyl, (1-Methylbutoxyamino)ethyl, (2-Methylbutoxyamino)ethyl, (3-Methylbutoxyamino)ethyl, (2,2-Dimethylpropoxyamino)ethyl, (1-Ethylpropoxyamino)ethyl, n-Hexoxyamino-ethyl, (1,1-Dimethylpropoxyamino)ethyl, (1,2-Dimethylpropoxyamino)ethyl, (1-Methylpentoxyamino)ethyl, (2-Methylpentoxyamino)ethyl, (3-Methylpentoxyamino)ethyl, (4-Methylpentoxyamino)ethyl, (1,1-Dimethylbutoxyamino)ethyl, (1,2-Dimethylbutoxyamino)-ethyl, (1,3-Dimethylbutoxyamino)ethyl, (2,2-Dimethylbutoxyamino)ethyl, (2,3-Dimethylbutoxyamino)ethyl, (3,3-Dimethylbutoxyamino)ethyl, (1-Ethylbutoxyamino)ethyl, (2-Ethylbutoxyamino)ethyl, (1,1,2-Trimethylpropoxyamino)ethyl, (1,2,2-Trimethylpropoxyamino)ethyl, (1-Ethyl-1-methylpropoxyamino)-ethyl, (1-Ethyl-2-methylpropoxyamino)ethyl, 2-(Methoxyamino)-propyl, 3-(Methoxyamino)propyl oder 2-(Ethoxyamino)propyl, vorzugsweise C₁-C₆-Alkoxyamino-C₁-C₂-alkyl;
- C₁-C₆-Alkoxy-C₁-C₆-alkylamino-C₁-C₆-alkyl für: C₁-C₆-Alkylamino-C₁-C₆-alkyl wie CH₂-NH-CH₃, CH₂-NH-C₂H₅, CH₂-NH-CH₂-C₂H₅, CH₂-NH-CH(CH₃)₂, CH₂-NH-(CH₂)₃-CH₃, CH₂-NH-CH(CH₃)-C₂H₅, CH₂-NH-CH₂-CH(CH₃)₂, CH₂-NH-C(CH₃)₃, CH₂-NH-(CH₂)₄-CH₃, (1-Methylbutylamino)methyl, (2-Methylbutylamino)methyl, (3-Methylbutylamino)methyl, (2,2-Dimethylpropylamino)methyl, (1-Ethylpropylamino)methyl, n-Hexylamino-methyl, (1,1-Dimethylpropylamino)methyl, (1,2-Dimethylpropylamino)methyl, (1-Methylpentylamino)methyl, (2-Methylpentylamino)methyl, (3-Methylpentylamino)methyl, (4-Methylpentylamino)methyl, (1,1-Dimethylbutylamino)methyl, (1,2-Dimethylbutylamino)-methyl, (1,3-Dimethylbutylamino)methyl, (2,2-Dimethylbutylamino)methyl, (2,3-Dimethylbutylamino)methyl, (3,3-Dimethylbutylamino)methyl, (1-Ethylbutylamino)methyl, (2-Ethylbutylamino)methyl, (1,1,2-Trimethylpropylamino)methyl, (1,2,2-Trimethylpropylamino)methyl, (1-Ethyl-1-methylpropylamino)-methyl, (1-Ethyl-2-methylpropylamino)methyl, Methylaminoethyl, Ethylamino-ethyl, n-Propylamino-ethyl, (1-Methylethylamino)ethyl, n-Butylamino-ethyl, (1-Methylpropylamino)ethyl, (2-Methylpropylamino)ethyl, (1,1-Dimethylethylamino)ethyl, n-Pentylaminoethyl, (1-Methylbutylamino)ethyl, (2-Methylbutylamino)ethyl, (3-Methylbutylamino)ethyl, (2,2-Dimethylpropylamino)ethyl, (1-Ethylpropylamino)ethyl, n-Hexylaminoethyl, (1,1-Dimethylpropylamino)ethyl, (1,2-Dimethylpropylamino)ethyl, (1-Methylpentylamino)ethyl, (2-Methylpentylamino)ethyl, (3-Methylpentylamino)ethyl, (4-Methylpentylamino)ethyl, (1,1-Dimethylbutylamino)ethyl, (1,2-Dimethylbutylamino)ethyl, (1,3-Dimethylbutylamino)ethyl, (2,2-Dimethylbutylamino)ethyl, (2,3-Dimethylbutylamino)ethyl, (3,3-Dimethylbutylamino)ethyl, (1-Ethylbutylamino)ethyl, (2-Ethylbutylamino)ethyl, (1,1,2-Trimethylpropylamino)ethyl, (1,2,2-Trimethylpropylamino)ethyl, (1-Ethyl-1-methylpropylamino)ethyl, (1-Ethyl-2-methylpropylamino)ethyl, 2-(Methylamino)propyl, 3-(Methylamino)propyl und 2-(Ethylamino)propyl, vorzugsweise C₁-C₆-Alkylamino-C₁-C₂-alkyl, das durch C₁-C₆-Alkoxy - wie vorstehend genannt - substituiert ist, also z.B. für CH₂-NH-CH₂-OCH₃ oder CH₂-NH-CH₂-OC₂H₅;
- C₁-C₆-Alkyloximino-C₁-C₆-alkyl für: durch C₁-C₆-Alkyloximino wie Methoxyimino, Ethoxyimino, 1-Propoxyimino, 2-Propoxyimino, 1-Methylethoxyimino, n-Butoxyimino, sec.-Butoxyimino, tert.-Butoxyimino, 1-Methyl-1-propoxyimino, 2-Methyl-1-propoxyimino, 1-Methyl-2-propoxyimino, 2-Methyl-2-propoxyimino, n-Pentoxyimino, 2-Pentoxyimino, 3-Pentoxyimino, 4-Pentoxyimino, 1-Methyl-1-butoxyimino, 2-Methyl-1-butoxyimino, 3-Methyl-1-butoxyimino, 1-Methyl-2-butoxyimino, 2-Methyl-2-butoxyimino, 3-Methyl-2-butoxyimino, 1-Methyl-3-butoxyimino, 2-Methyl-3-butoxyimino, 3-Methyl-3-butoxyimino, 1,1-Dimethyl-2-propoxyimino, 1,2-Dimethyl-1-propoxyimino, 1,2-Dimethyl-2-propoxyimino, 1-Ethyl-1-propoxyimino, 1-Ethyl-2-propoxyimino, n-Hexoxyimino, 2-Hexoxyimino, 3-Hexoxyimino, 4-Hexoxyimino, 5-Hexoxyimino, 1-Methyl-1-pentoxyimino, 2-Methyl-1-pentoxyimino, 3-Methyl-1-pentoxyimino, 4-Methyl-1-pentoxyimino, 1-Methyl-2-pentoxyimino, 2-Methyl-2-pentoxyimino, 3-Methyl-2-pentoxyimino, 4-Methyl-2-pentoxyimino, 1-Methyl-3-pentoxyimino, 2-Methyl-3-pentoxyimino, 3-Methyl-3-pentoxyimino, 4-Methyl-3-pentoxyimino, 1-Methyl-4-pentoxyimino, 2-Methyl-4-pentoxyimino, 3-Methyl-4-pentoxyimino, 4-Methyl-4-pentoxyimino, 1,1-Dimethyl-2-butoxyimino, 1,1-Dimethyl-3-butoxyimino, 1,2-Dimethyl-1-butoxyimino, 1,2-Dimethyl-2-butoxyimino, 1,2-Dimethyl-3-butoxyimino, 1,3-Dimethyl-1-butoxyimino, 1,3-Dimethyl-2-butoxyimino, 1,3-Dimethyl-3-butoxyimino, 2,2-Dimethyl-3-butoxyimino, 2,3-Dimethyl-1-butoxyimino, 2,3-Dimethyl-2-butoxyimino, 2,3-Dimethyl-3-butoxyimino, 3,3-Dimethyl-1-butoxyimino, 3,3-Dimethyl-2-butoxyimino, 1-Ethyl-1-butoxyimino, 1-Ethyl-2-butoxyimino, 1-Ethyl-3-butoxyimino, 2-Ethyl-1-butoxyimino, 2-Ethyl-2-butoxyimino, 2-Ethyl-3-butoxyimino, 1,1,2-Trimethyl-2-propoxyimino, 1-Ethyl-1-methyl-2-propoxyimino, 1-Ethyl-2-methyl-1-propoxyimino und 1-Ethyl-2-methyl-2-propoxyimino, substituiertes C₁-C₆-Alkyl, also z.B. für Methoxyiminomethyl;
- C₁-C₆-Alkoxy-C₁-C₆-alkyl für: durch C₁-C₆-Alkoxy - wie vorstehend genannt - substituiertes C₁-C₆-Alkyl, also z.B. für CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)-propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)-propyl, 3-(Ethoxy)-propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, insbesondere für CH₂-OCH₃ oder 2-Methoxyethyl;
- Di(C₁-C₆-Alkoxy)-C₁-C₆-alkyl für: z.B. 2,2-Dimethoxyethyl oder 2,2-Diethoxyethyl;
- C₁-C₆-Alkoxy-C₁-C₆-alkoxy für: durch C₁-C₆-Alkoxy wie vorstehend genannt substituiertes C₁-C₆-Alkoxy, also z.B. für OCH₂-OCH₃, OCH₂-OC₂H₅, n-Propoxymethoxy, OCH₂-OCH(CH₃)₂, n-Butoxymethoxy, (1-Methylpropoxy)methoxy, (2-Methylpropoxy)methoxy, OCH₂-OC(CH₃)₃, 2-(Methoxy)ethoxy, 2-(Ethoxy)ethoxy, 2-(n-Propoxy)ethoxy, 2-(1-Methylethoxy)ethoxy, 2-(n-Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(n-Propoxy)propoxy, 2-(1-Methylethoxy)propoxy, 2-(n-Butoxy)propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(n-Propoxy)propoxy, 3-(1-Methylethoxy)propoxy, 3-(n-Butoxy)propoxy, 3-(1-Methylpropoxy)propoxy, 3-(2-Methylpropoxy)propoxy, 3-(1,1-Dimethylethoxy)propoxy, 2-(Methoxy)-butoxy, 2-(Ethoxy)butoxy, 2-(n-Propoxy)butoxy, 2-(1-Methylethoxy)butoxy, 2-(n-Butoxy)-butoxy, 2-(1-Methylpropoxy)-butoxy, 2-(2-Methylpropoxy)butoxy, 2-(1,1-Dimethylethoxy)-butoxy, 3-(Methoxy)butoxy, 3-(Ethoxy)butoxy, 3-(n-Propoxy)-butoxy, 3-(1-Methylethoxy)butoxy, 3-(n-Butoxy)butoxy, 3-(1-Methylpropoxy)butoxy, 3-(2-Methylpropoxy)butoxy, 3-(1,1-Dimethylethoxy)butoxy, 4-(Methoxy)butoxy, 4-(Ethoxy)-butoxy, 4-(n-Propoxy)butoxy, 4-(1-Methylethoxy)butoxy, 4-(n-Butoxy)butoxy, 4-(1-Methylpropoxy)butoxy, 4-(2-Methylpropoxy)butoxy, 4-(1,1-Dimethylethoxy)butoxy, 5-(Methoxy)-pentoxy, 5-(Ethoxy)pentoxy, 5-(n-Propoxy)pentoxy, 5-(1-Methylethoxy)pentoxy, 5-(n-Butoxy)pentoxy, 5-(1-Methylpropoxy)pentoxy, 5-(2-Methylpropoxy)pentoxy, 5-(1,1-Dimethylethoxy)pentoxy, 6-(Methoxy)hexoxy, 6-(Ethoxy)hexoxy, 6-(n-Propoxy)hexoxy, 6-(1-Methylethoxy)hexoxy, 6-(n-Butoxy)-hexoxy, 6-(1-Methylpropoxy)hexoxy, 6-(2-Methylpropoxy)hexoxy oder 6-(1,1-Dimethylethoxy)hexoxy, insbesondere für OCH₂-OCH₃ oder OCH₂-OC₂H₅;
- (C₁-C₆-Alkyl)carbonyl-C₁-C₆-alkoxy für: durch (C₁-C₆-Alkyl)-carbonyl wie vorstehend genannt substituiertes C₁-C₆-Alkoxy, also z.B. für OCH₂-CO-CH₃, OCH₂-CO-C₂H₅, OCH₂-CO-CH₂-C₂H₅, OCH₂-CO-CH(CH₃)₂, n-Butylcarbonyl-methoxy, 1-(CO-CH₃)ethoxy, 2-(CO-CH₃)ethoxy, 2-(CO-C₂H₅)ethoxy, 2-(CO-CH₂-C₂H₅)ethoxy, 2-(n-Butylcarbonyl)ethoxy, 3-(CO-CH₃)propoxy, 3-(CO-C₂H₅)-propoxy, 3-(CO-CH₂-C₂H₅)propoxy, 3-(n-Butylcarbonyl)propoxy, 4-(CO-CH₃)butoxy, 4-(CO-C₂H₅)butoxy, 4-(CO-CH₂-C₂H₅)butoxy, 4-(n-Butylcarbonyl)butoxy, 5-(CO-CH₃)pentoxy, 5-(CO-C₂H₅)-pentoxy, 5-(CO-CH₂-C₂H₅)pentoxy, 5-(n-Butylcarbonyl)butoxy, 6-(CO-CH₃)hexoxy, 6-(CO-C₂H₅)hexoxy, 6-(CO-CH₂-C₂H₅)hexoxy oder 6-(n-Butylcarbonyl)hexoxy, insbesondere für OCH₂-CO-OCH₃ oder 1-(CO-CH₃)ethoxy;
- (C₁-C₆-Alkoxy)carbonyl-C₁-C₆-alkoxy für: durch (C₁-C₆-Alkoxy)-carbonyl wie vorstehend genannt substituiertes C₁-C₆-Alkoxy, also z.B. für OCH₂-CO-OCH₃, OCH₂-CO-OC₂H₅, OCH₂-CO-OCH₂-C₂H₅, OCH₂-CO-OCH(CH₃)₂, n-Butoxycarbonyl-methoxy, 1-(Methoxycarbonyl)ethoxy, 2-(Methoxycarbonyl)ethoxy, 2-(Ethoxycarbonyl)ethoxy, 2-(n-Propoxycarbonyl)ethoxy, 2-(n-Butoxycarbonyl)ethoxy, 3-(Methoxycarbonyl)propoxy, 3-(Ethoxycarbonyl)propoxy, 3-(n-Propoxycarbonyl)propoxy, 3-(n-Butoxycarbonyl)propoxy, 4-(Methoxycarbonyl)butoxy, 4-(Ethoxycarbonyl)butoxy, 4-(n-Propoxycarbonyl)butoxy, 4-(n-Butoxycarbonyl)butoxy, 5-(Methoxycarbonyl)pentoxy, 5-(Ethoxycarbonyl)pentoxy, 5-(n-Propoxycarbonyl)pentoxy, 5-(n-Butoxycarbonyl)butoxy, 6-(Methoxycarbonyl)hexoxy, 6-(Ethoxycarbonyl)hexoxy, 6-(n-Propoxycarbonyl)hexoxy oder 6-(n-Butoxycarbonyl)hexoxy, insbesondere für OCH₂-CO-OCH₃ oder 1-(Methoxycarbonyl)ethoxy;
- (C₁-C₆-Alkoxy)carbonyl-C₁-C₆-alkyl für: durch (C₁-C₆-Alkoxy)-carbonyl wie vorstehend genannt substituiertes C₁-C₆-Alkyl, also z.B. für Methoxycarbonylmethyl, Ethoxycarbonylmethyl, 1-(Methoxycarbonyl)ethyl, 2-(Methoxycarbonyl)ethyl, 2-(Ethoxycarbonyl)ethyl, 3-(Methoxycarbonyl)propyl, 4-(Methoxycarbonyl)butyl, 5-(Methoxycarbonyl)pentyl oder 6-(Methoxycarbonyl)hexyl;
- (C₁-C₆-Alkoxy)carbonyl-C₁-C₆-alkylsulfonyl für: durch (C₁-C₆-Alkoxy)carbonyl wie vorstehend genannt substituiertes C₁-C₆-Alkylsulfonyl, also z.B. für Methoxycarbonylmethylsulfonyl, Ethoxycarbonylmethylsulfonyl, 1-(Methoxycarbonyl)-ethylsulfonyl, 2-(Methoxycarbonyl)ethylsulfonyl, 2-(Ethoxycarbonyl)ethylsulfonyl, 3-(Methoxycarbonyl)propylsulfonyl, 4-(Methoxycarbonyl)butylsulfonyl, 5-(Methoxycarbonyl)pentylsulfonyl oder 6-(Methoxycarbonyl)hexylsulfonyl;
- C₁-C₆-Alkylthio-C₁-C₆-alkyl für: durch C₁-C₆-Alkylthio wie vorstehend genannt substituiertes C₁-C₆-Alkyl, also z.B. für CH₂-SCH₃, CH₂-SC₂H₅, CH₂-SCH₂-C₂H₅, CH₂-SCH(CH₃)₂, n-Butylthiomethyl, CH₂-SCH(CH₃)-C₂H₅, CH₂-SCH₂-CH(CH₃)₂, CH₂-SC(CH₃)₃, 2-(SCH₃)ethyl, 2-(SC₂H₅)ethyl, 2-(SCH₂-C₂H₅)ethyl, 2-[SCH(CH₃)₂]ethyl, 2-(n-Butylthio)ethyl, 2-[SCH(CH₃)-C₂H₅]-ethyl, 2-(2-Methylpropylthio)ethyl, 2-[SC(CH₃)₃]ethyl, 2-(SCH₃)propyl, 3-(SCH₃)propyl, 2-(SC₂H₅)propyl, 3-(SC₂H₅)-propyl, 3-(SCH₂-C₂H₅)propyl, 3-(Butylthio)propyl, 4-(SCH₃)-butyl, 4-(SC₂H₅)butyl, 4-(SCH₂-C₂H₅)butyl oder 4-(n-Butylthio)butyl, insbesondere für 2-(SCH₃)ethyl;
- C₁-C₆-Alkylthio-C₁-C₆-alkoxy für: durch C₁-C₆-Alkylthio wie vorstehend genannt substituiertes C₁-C₆-Alkoxy, also z.B. für OCH₂-SCH₃, OCH₂-SC₂H₅, OCH₂-SCH₂-C₂H₅, OCH₂-SCH(CH₃)₂, n-Butylthiomethoxy, OCH₂-SCH(CH₃)-C₂H₅, OCH₂-SCH₂-CH(CH₃)₂, OCH₂-SC(CH₃)₃, 2-(SCH₃)ethoxy, 2-(SC₂Hₛ)ethoxy, 2-(SCH₂-C₂H₅)-ethoxy, 2-[SCH(CH₃)₂]ethoxy, 2-(n-Butylthio)ethoxy, 2-[SCH(CH₃]-C₂H₅]ethoxy, 2-(2-Methylpropylthio)ethoxy, 2-[SC(CH₃)₃]ethoxy, 2-(SCH₃)propoxy, 3-(SCH₃)propoxy, 2-(SC₂H₅)propoxy, 3-(SC₂H₅)propoxy, 3-(SCH₂-C₂H₅)propoxy, 3-(Butylthio)propoxy, 4-(SCH₃)butoxy, 4-(SC₂H₅)butoxy, 4-(CH₂-C₂H₅)butoxy oder 4-(n-Butylthio)butoxy, insbesondere für 2-(SCH₃)ethoxy;
- C₁-C₆-Alkylthio-(C₁-C₆-alkyl)carbonyl für: durch C₁-C₆-Alkylthio wie vorstehend genannt, vorzugsweise SCH₃ oder SC₂H₅, substituiertes (C₁-C₆-Alkyl)carbonyl, also z.B. für Methylthiomethylcarbonyl, Ethylthiomethylcarbonyl, 1-(Methylthio)ethylcarbonyl, 2-(Methylthio)ethylcarbonyl, 3-(Methylthio)propylcarbonyl, 4-(Methylthio)butylcarbonyl, 5(Methylthio)pentylcarbonyl oder 6-(Methylthio)hexylcarbonyl, insbesondere für CO-CH₂-SCH₃ oder CO-CH(CH₃)-SCH₃;
- Di(C₁-C₆-alkyl)amino-C₁-C₆-alkoxy: durch Di-(C₁-C₆-alkyl)-amino wie N(CH₃)₂, N(C₂H₅)₂, N,N-Dipropylamino, N,N-Di-(1-methylethyl)amino, N,N-Dibutylamino, N,N-Di-(1-methylpropyl)amino, N,N-Di-(2-methylpropyl)amino, N[C(CH₃)₃]₂, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)-amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino, vorzugsweise N,N-Dimethylamino oder N,N-Diethylamino, substituiertes C₁-C₆-Alkoxy, also z.B. für OCH₂-N(CH₃)₂, OCH₂-N(C₂H₅)₂, OCH(CH₃)-N(CH₃)₂, 2-(Dimethylamino)ethoxy, OCH(CH₃)-N(C₂H₅)₂, 3-(Dimethylamino)propoxy, 4-(Dimethylamino)butoxy, 5-(Dimethylamino)pentoxy oder 6-(Dimethylamino)hexoxy, insbesondere für OCH₂-N(CH₃)₂ oder OCH(CH₃)-N(CH₃)₂;
- C₃-C₆-Alkenyl für: z.B. Prop-2-en-1-yl, n-Buten-4-yl, 1-Methyl-prop-2-en-1-yl, 2-Methyl-prop-2-en-1-yl, 2-Buten-1-yl, n-Penten-3-yl, n-Penten-4-yl, 1-Methyl-but-2-en-1-yl, 2-Methyl-but-2-en-1-yl, 3-Methyl-but-2-en-1-yl, 1-Methylbut-3-en-1-yl, 2-Methyl-but-3-en-1-yl, 3-Methyl-but-3-en-1-yl, 1,1-Dimethyl-prop-2-en-1-yl, 1,2-Dimethyl-prop-2-en-1-yl, 1-Ethyl-prop-2-en-1-yl, n-Hex-3-en-1-yl, n-Hex-4-en-1-yl, n-Hex-5-en-1-yl, 1-Methyl-pent-3-en-1-yl, 2-Methylpent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-2-en-1-yl, 1,2-Dimethyl-but-3-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-2-en-1-yl, 2,3-Dimethyl-but-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl oder 1-Ethyl-2-methylprop-2-en-1-yl, insbesondere für Prop-2-en-1-yl oder n-Buten-4-yl;
- C₂-C₆-Alkenyl für: Ethenyl oder einen der unter C₃-C₆-Alkenyl genannten Reste, insbesondere für Ethenyl oder Prop-2-en-1-yl;
- C₃-C₆-Alkenyloxy für: Prop-1-en-1-yloxy, Prop-2-en-1-yloxy, 1-Methylethenyloxy, n-Buten-1-yloxy, n-Buten-2-yloxy, n-Buten-3-yloxy, 1-Methyl-prop-1-en-1-yloxy, 2-Methylprop-1-en-1-yloxy, 1-Methyl-prop-2-en-1-yloxy, 2-Methylprop-2-en-1-yloxy, n-Penten-1-yloxy, n-Penten-2-yloxy, n-Penten-3-yloxy, n-Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, n-Hex-1-en-1-yloxy, n-Hex-2-en-1-yloxy, n-Hex-3-en-1-yloxy, n-Hex-4-en-1-yloxy, n-Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methyl-pent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethylbut-3-en-1-yloxy, 2-Ethyl-but-1-en-1-yloxy, 2-Ethyl-but-2-en-1-yloxy, 2-Ethyl-but-3-en-1-yloxy, 1,1,2-Trimethyl-prop-2-en-1-yloxy, 1-Ethyl-1-methyl-prop-2-en-1-yloxy, 1-Ethyl-2-methyl-prop-1-en-1-yloxy oder 1-Ethyl-2-methyl-prop-2-en-1-yloxy, insbesondere für Prop-2-en-1-yloxy;
- C₂-C₆-Alkenyloxy für: Ethenyloxy oder einen der unter C₃-C₆-Alkenyloxy genannten Reste, insbesondere für Ethenyloxy oder Prop-2-en-1-yloxy;
- C₃-C₆-Halogenalkenyloxy für: C₃-C₆-Alkenyloxy wie vorstehend genannt, das partiell oder vollständig durch Fluor, Chlor und/oder Brom substituiert ist, also z.B. 2-Chlorallyloxy, 3-Chlorallyloxy, 2,3-Dichlorallyloxy, 3,3-Dichlorallyloxy, 2,3,3-Trichlorallyloxy, 2,3-Dichlorbut-2-enyloxy, 2-Bromallyloxy, 3-Bromallyloxy, 2,3-Dibromallyloxy, 3,3-Dibromallyloxy, 2,3,3-Tribromallyloxy oder 2,3-Dibrombut-2-enyloxy, insbesondere für 2-Chlorallyloxy oder 3,3-Dichlorallyloxy;
- C₂-C₆-Alkenylthio für: Ethenylthio, Prop-1-en-1-ylthio, Prop-2-en-1-ylthio, 1-Methylethenylthio, n-Buten-1-ylthio, n-Buten-2-ylthio, n-Buten-3-ylthio, 1-Methyl-prop-1-en-1-ylthio, 2-Methyl-prop-1-en-1-ylthio, 1-Methyl-prop-2-en-1-ylthio, 2-Methyl-prop-2-en-1-ylthio, n-Penten-1-ylthio, n-Penten-2-ylthio, n-Penten-3-ylthio, n-Penten-4-ylthio, 1-Methyl-but-1-en-1-ylthio, 2-Methyl-but-1-en-1-ylthio, 3-Methyl-but-1-en-1-ylthio, 1-Methyl-but-2-en-1-ylthio, 2-Methyl-but-2-en-1-ylthio, 3-Methyl-but-2-en-1-ylthio, 1-Methyl-but-3-en-1-ylthio, 2-Methyl-but-3-en-1-ylthio, 3-Methyl-but-3-en-1-ylthio, 1,1-Dimethyl-prop-2-en-1-ylthio, 1,2-Dimethyl-prop-1-en-1-ylthio, 1,2-Dimethyl-prop-2-en-1-ylthio, 1-Ethyl-prop-1-en-2-ylthio, 1-Ethyl-prop-2-en-1-ylthio, n-Hex-1-en-1-ylthio, n-Hex-2-en-1-ylthio, n-Hex-3-en-1-ylthio, n-Hex-4-en-1-ylthio, n-Hex-5-en-1-ylthio, 1-Methyl-pent-1-en-1-ylthio, 2-Methyl-pent-1-en-1-ylthio, 3-Methyl-pent-1-en-1-ylthio, 4-Methyl-pent-1-en-1-ylthio, 1-Methyl-pent-2-en-1-ylthio, 2-Methyl-pent-2-en-1-ylthio, 3-Methyl-pent-2-en-1-ylthio, 4-Methyl-pent-2-en-1-ylthio, 1-Methyl-pent-3-en-1-ylthio, 2-Methyl-pent-3-en-1-ylthio, 3-Methyl-pent-3-en-1-ylthio, 4-Methyl-pent-3-en-1-ylthio, 1-Methyl-pent-4-en-1-ylthio, 2-Methyl-pent-4-en-1-ylthio, 3-Methyl-pent-4-en-1-ylthio, 4-Methyl-pent-4-en-1-ylthio, 1,1-Dimethyl-but-2-en-1-ylthio, 1,1-Dimethyl-but-3-en-1-ylthio, 1,2-Dimethyl-but-1-en-1-ylthio, 1,2-Dimethyl-but-2-en-1-ylthio, 1,2-Dimethyl-but-3-en-1-ylthio, 1,3-Dimethylbut-1-en-1-ylthio, 1,3-Dimethyl-but-2-en-1-ylthio, 1,3-Dimethyl-but-3-en-1-ylthio, 2,2-Dimethyl-but-3-en-1-ylthio, 2,3-Dimethyl-but-1-en-1-ylthio, 2,3-Dimethyl-but-2-en-1-ylthio, 2,3-Dimethyl-but-3-en-1-ylthio, 3,3-Dimethyl-but-1-en-1-ylthio, 3,3-Dimethyl-but-2-en-1-ylthio, 1-Ethyl-but-1-en-1-ylthio, 1-Ethyl-but-2-en-1-ylthio, 1-Ethyl-but-3-en-1-ylthio, 2-Ethyl-but-1-en-1-ylthio, 2-Ethyl-but-2-en-1-ylthio, 2-Ethyl-but-3-en-1-ylthio, 1,1,2-Trimethyl-prop-2-en-1-ylthio, 1-Ethyl-1-methyl-prop-2-en-1-ylthio, 1-Ethyl-2-methylprop-1-en-1-ylthio oder 1-Ethyl-2-methyl-prop-2-en-1-ylthio, insbesondere für Ethenylthio oder Prop-2-en-1-ylthio;
- C₃-C₆-Alkinyl für: Prop-1-in-1-yl, Prop-2-in-1-yl, n-But-1-in-1-yl n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methylpent-1-in-1-yl, 3-Methyl-pent-1-in-3-yl, 3-Methylpent-1-in-4-yl, 3-Methyl-pent-1-in-5-yl, 4-Methylpent-1-in-1-yl, 4-Methyl-pent-2-in-4-yl oder 4-Methylpent-2-in-5-yl, insbesondere für Prop-2-in-1-yl;
- C₂-C₆-Alkinyl für: Ethinyl oder einen der unter C₃-C₆-Alkinyl genannten Reste, insbesondere für Ethinyl oder Prop-2-in1-yl;
- C₃-C₆-Alkinyloxy für: Prop-1-in-1-yloxy, Prop-2-in-1-yloxy, n-But-1-in-1-yloxy, n-But-1-in-3-yloxy, n-But-1-in-4-yloxy, n-But-2-in-1-yloxy, n-Pent-1-in-1-yloxy, n-Pent-1-in-3-yloxy, n-Pent-1-in-4-yloxy, n-Pent-1-in-5-yloxy, n-Pent-2-in-1-yloxy, n-Pent-2-in-4-yloxy, n-Pent-2-in-5-yloxy, 3-Methyl-but-1-in-3-yloxy, 3-Methylbut-1-in-4-yloxy, n-Hex-1-in-1-yloxy, n-Hex-1-in-3-yloxy, n-Hex-1-in-4-yloxy, n-Hex-1-in-5-yloxy, n-Hex-1-in-6-yloxy, n-Hex-2-in-1-yloxy, n-Hex-2-in-4-yloxy, n-Hex-2-in-5-yloxy, n-Hex-2-in-6-yloxy, n-Hex-3-in-1-yloxy, n-Hex-3-in-2-yloxy, 3-Methylpent-1-in-1-yloxy, 3-Methyl-pent-1-in-3-yloxy, 3-Methyl-pent-1-in-4-yloxy, 3-Methyl-pent-1-in-5-yloxy, 4-Methylpent-1-in-1-yloxy, 4-Methyl-pent-2-in-4-yloxy oder 4-Methylpent-2-in-5-yloxy, insbesondere für Prop-2-in-1-yloxy;
- C₂-C₆-Alkinyloxy für: Ethinyloxy oder einen der unter C₃-C₆-Alkinyloxy genannten Reste, insbesondere für Ethinyloxy oder Prop-2-in-1-yloxy;
- C₃-C₆-Alkinylthio für: Prop-1-in-1-ylthio, Prop-2-in-1-ylthio, n-But-1-in-1-ylthio, n-But-1-in-3-ylthio, n-But-1-in-4-ylthio, n-But-2-in-1-ylthio, n-Pent-1-in-1-ylthio, n-Pent-1-in-3-ylthio, n-Pent-1-in-4-ylthio, n-Pent-1-in-5-ylthio, n-Pent-2-in-1-ylthio, n-Pent-2-in-4-ylthio, n-Pent-2-in-5-ylthio, 3-Methylbut-1-in-3-ylthio, 3-Methylbut-1-in-4-ylthio, n-Hex-1-in-1-ylthio, n-Hex-1-in-3-ylthio, n-Hex-1-in-4-ylthio, n-Hex-1-in-5-ylthio, n-Hex-1-in-6-ylthio, n-Hex-2-in-1-ylthio, n-Hex-2-in-4-ylthio, n-Hex-2-in-5-ylthio, n-Hex-2-in-6-ylthio, n-Hex-3-in-1-ylthio, n-Hex-3-in-2-ylthio, 3-Methylpent-1-in-1-ylthio, 3-Methylpent-1-in-3-ylthio, 3-Methyl-pent-1-in-4-ylthio, 3-Methylpent-1-in-5-ylthio, 4-Methyl-pent-1-in-1-ylthio, 4-Methylpent-2-in-4-ylthio oder 4-Methyl-pent-2-in-5-ylthio, insbesondere für Prop-2-in-1-ylthio;
- C₂-C₆-Alkinylthio für: Ethinylthio oder einen der unter C₃-C₆-Alkinylthio genannten Reste, insbesondere für Ethinylthio oder Prop-2-in-1-ylthio;
- (C₃-C₆-Alkenyloxy)carbonyl für: Prop-1-en-1-yloxycarbonyl, Prop-2-en-1-yloxycarbonyl, 1-Methylethenyloxycarbonyl, n-Buten-1-yloxycarbonyl, n-Buten-2-yloxycarbonyl, n-Buten-3-yloxycarbonyl, 1-Methyl-prop-1-en-1-yloxycarbonyl, 2-Methylprop-1-en-1-yloxycarbonyl, 1-Methyl-prop-2-en-1-yloxycarbonyl, 2-Methyl-prop-2-en-1-yloxycarbonyl, n-Penten-1-yloxycarbonyl, n-Penten-2-yloxycarbonyl, n-Penten-3-yloxycarbonyl, n-Penten-4-yloxycarbonyl, 1-Methyl-but-1-en-1-yloxycarbonyl, 2-Methyl-but-1-en-1-yloxycarbonyl, 3-Methyl-but-1-en-1-yloxycarbonyl, 1-Methyl-but-2-en-1-yloxycarbonyl, 2-Methylbut-2-en-1-yloxycarbonyl, 3-Methyl-but-2-en-1-yloxycarbonyl, 1-Methyl-but-3-en-1-yloxycarbonyl, 2-Methyl-but-3-en-1-yloxycarbonyl, 3-Methyl-but-3-en-1-yloxycarbonyl, 1,1-Dimethylprop-2-en-1-yloxycarbonyl, 1,2-Dimethyl-prop-1-en-1-yloxycarbonyl, 1,2-Dimethyl-prop-2-en-1-yloxycarbonyl, 1-Ethylprop-1-en-2-yloxycarbonyl, 1-Ethyl-prop-2-en-1-yloxycarbonyl, n-Hex-1-en-1-yloxycarbonyl, n-Hex-2-en-1-yloxycarbonyl, n-Hex-3-en-1-yloxycarbonyl, n-Hex-4-en-1-yloxycarbonyl, n-Hex-5-en-1-yloxycarbonyl, 1-Methyl-pent-1-en-1-yloxycarbonyl, 2-Methyl-pent-1-en-1-yloxycarbonyl, 3-Methylpent-1-en-1-yloxycarbonyl, 4-Methyl-pent-1-en-1-yloxycarbonyl, 1-Methyl-pent-2-en-1-yloxycarbonyl, 2-Methylpent-2-en-1-yloxycarbonyl, 3-Methyl-pent-2-en-1-yloxycarbonyl, 4-Methyl-pent-2-en-1-yloxycarbonyl, 1-Methylpent-3-en-1-yloxycarbonyl, 2-Methyl-pent-3-en-1-yloxycarbonyl, 3-Methyl-pent-3-en-1-yloxycarbonyl, 4-Methylpent-3-en-1-yloxycarbonyl, 1-Methyl-pent-4-en-1-yloxycarbonyl, 2-Methyl-pent-4-en-1-yloxycarbonyl, 3-Methylpent-4-en-1-yloxycarbonyl, 4-Methyl-pent-4-en-1-yloxycarbonyl, 1,1-Dimethyl-but-2-en-1-yloxycarbonyl, 1,1-Dimethylbut-3-en-1-yloxycarbonyl, 1,2-Dimethyl-but-1-en-1-yloxycarbonyl, 1,2-Dimethyl-but-2-en-1-yloxycarbonyl, 1,2-Dimethylbut-3-en-1-yloxycarbonyl, 1,3-Dimethyl-but-1-en-1-yloxycarbonyl, 1,3-Dimethyl-but-2-en-1-yloxycarbonyl, 1,3-Dimethylbut-3-en-1-yloxycarbonyl, 2,2-Dimethyl-but-3-en-1-yloxycarbonyl, 2,3-Dimethyl-but-1-en-1-yloxycarbonyl, 2,3-Dimethylbut-2-en-1-yloxycarbonyl, 2,3-Dimethyl-but-3-en-1-yloxycarbonyl, 3,3-Dimethyl-but-1-en-1-yloxycarbonyl, 3,3-Dimethylbut-2-en-1-yloxycarbonyl, 1-Ethyl-but-1-en-1-yloxycarbonyl, 1-Ethyl-but-2-en-1-yloxycarbonyl, 1-Ethyl-but-3-en-1-yloxycarbonyl, 2-Ethyl-but-1-en-1-yloxycarbonyl, 2-Ethylbut-2-en-1-yloxycarbonyl, 2-Ethyl-but-3-en-1-yloxycarbonyl, 1,1,2-Trimethyl-prop-2-en-1-yloxycarbonyl, 1-Ethyl-1-methyl-prop-2-en-1-yloxycarbonyl, 1-Ethyl-2-methyl-prop-1-en-1-yloxycarbonyl oder 1-Ethyl-2-methyl-prop-2-en-1-yloxycarbonyl, insbesondere für Prop-2-en-1-yloxycarbonyl;
- (C₃-C₆-Alkenyloxy)carbonyl-C₁-C₆-alkyl für: durch (C₃-C₆-Alkenyloxy)carbonyl wie vorstehend genannt, vorzugsweise Prop-2-en-1-yl-oxycarbonyl, substituiertes C₁-C₆-Alkyl, also beispielsweise Prop-2-en-1-yl-oxycarbonyl-methyl;
- (C₂-C₆-Alkenyl)carbonyloxy für: Ethenylcarbonyloxy, Prop-1-en-1-ylcarbonyloxy, Prop-2-en-1-ylcarbonyloxy, 1-Methylethenylcarbonyloxy, n-Buten-1-ylcarbonyloxy, n-Buten-2-ylcarbonyloxy, n-Buten-3-ylcarbonyloxy, 1-Methylprop-1-en-1-ylcarbonyloxy, 2-Methyl-prop-1-en-1-ylcarbonyloxy, 1-Methyl-prop-2-en-1-ylcarbonyloxy, 2-Methylprop-2-en-1-ylcarbonyloxy, n-Penten-1-ylcarbonyloxy, n-Penten-2-ylcarbonyloxy, n-Penten-3-ylcarbonyloxy, n-Penten-4-ylcarbonyloxy, 1-Methyl-but-1-en-1-ylcarbonyloxy, 2-Methylbut-1-en-1-ylcarbonyloxy, 3-Methyl-but-1-en-1-ylcarbonyloxy, 1-Methyl-but-2-en-1-ylcarbonyloxy, 2-Methyl-but-2-en-1-ylcarbonyloxy, 3-Methyl-but-2-en-1-ylcarbonyloxy, 1-Methylbut-3-en-1-ylcarbonyloxy, 2-Methyl-but-3-en-1-ylcarbonyloxy, 3-Methyl-but-3-en-1-ylcarbonyloxy, 1,1-Dimethylprop-2-en-1-ylcarbonyloxy, 1,2-Dimethyl-prop-1-en-1-ylcarbonyloxy, 1,2-Dimethyl-prop-2-en-1-ylcarbonyloxy, 1-Ethylprop-1-en-2-ylcarbonyloxy, 1-Ethyl-prop-2-en-1-ylcarbonyloxy, n-Hex-1-en-1-ylcarbonyloxy, n-Hex-2-en-1-ylcarbonyloxy, n-Hex-3-en-1-ylcarbonyloxy, n-Hex-4-en-1-ylcarbonyloxy, n-Hex-5-en-1-ylcarbonyloxy, 1-Methyl-pent-1-en-1-ylcarbonyloxy, 2-Methyl-pent-1-en-1-ylcarbonyloxy, 3-Methylpent-1-en-1-ylcarbonyloxy, 4-Methyl-pent-1-en-1-ylcarbonyloxy, 1-Methyl-pent-2-en-1-ylcarbonyloxy, 2-Methylpent-2-en-1-ylcarbonyloxy, 3-Methyl-pent-2-en-1-ylcarbonyloxy, 4-Methyl-pent-2-en-1-ylcarbonyloxy, 1-Methylpent-3-en-1-ylcarbonyloxy, 2-Methyl-pent-3-en-1-ylcarbonyloxy, 3-Methyl-pent-3-en-1-ylcarbonyloxy, 4-Methylpent-3-en-1-ylcarbonyloxy, 1-Methyl-pent-4-en-1-ylcarbonyloxy, 2-Methyl-pent-4-en-1-ylcarbonyloxy, 3-Methylpent-4-en-1-ylcarbonyloxy, 4-Methyl-pent-4-en-1-ylcarbonyloxy, 1,1-Dimethyl-but-2-en-1-ylcarbonyloxy, 1,1-Dimethylbut-3-en-1-ylcarbonyloxy, 1,2-Dimethyl-but-1-en-1-ylcarbonyloxy, 1,2-Dimethyl-but-2-en-1-ylcarbonyloxy, 1,2-Dimethylbut-3-en-1-ylcarbonyloxy, 1,3-Dimethyl-but-1-en-1-ylcarbonyloxy, 1,3-Dimethyl-but-2-en-1-ylcarbonyloxy, 1,3-Dimethylbut-3-en-1-ylcarbonyloxy, 2,2-Dimethyl-but-3-en-1-ylcarbonyloxy, 2,3-Dimethyl-but-1-en-1-ylcarbonyloxy, 2,3-Dimethylbut-2-en-1-ylcarbonyloxy, 2,3-Dimethyl-but-3-en-1-ylcarbonyloxy, 3,3-Dimethyl-but-1-en-1-ylcarbonyloxy, 3,3-Dimethylbut-2-en-1-ylcarbonyloxy, 1-Ethyl-but-1-en-1-ylcarbonyloxy, 1-Ethyl-but-2-en-1-ylcarbonyloxy, 1-Ethyl-but-3-en-1-ylcarbonyloxy, 2-Ethyl-but-1-en-1-ylcarbonyloxy, 2-Ethylbut-2-en-1-ylcarbonyloxy, 2-Ethyl-but-3-en-1-ylcarbonyloxy, 1,1,2-Trimethyl-prop-2-en-1-ylcarbonyloxy, 1-Ethyl-1-methylprop-2-en-1-ylcarbonyloxy, 1-Ethyl-2-methyl-prop-1-en-1-ylcarbonyloxy oder 1-Ethyl-2-methyl-prop-2-en-1-ylcarbonyloxy, insbesondere für Ethenylcarbonyloxy oder Prop-2-en-1-ylcarbonyloxy;
- (C₁-C₆-Alkoxy)carbonyl-C₂-C₆-alkenyl für: durch (C₁-C₆-Alkoxy)carbonyl wie vorstehend genannt substituiertes C₂-C₆-Alkenyl, also beispielsweise für Methoxycarbonylprop-2-en-1-yl;
- C₁-C₆-Alkoxy-C₃-C₆-alkenyloxy für: durch C₁-C₆-Alkoxy wie vorstehend genannt substituiertes C₃-C₆-Alkenyloxy, also beispielsweise für Methylprop-2-en-1-yloxy;
- C₃-C₆-Alkenyloxy-C₁-C₆-alkyl für: durch C₃-C₆-Alkenyloxy wie vorstehend genannt, vorzugsweise Allyloxy, 2-Methylprop-2-en-1-yloxy, But-1-en-3-yloxy, But-1-en-4-yloxy oder But-2-en-1-yloxy substituiertes C₁-C₆-Alkyl, also beispielsweise für Allyloxymethyl, 2-Allyloxyethyl oder But-1-en-4-yloxymethyl;
- C₃-C₆-Alkinyloxy-C₁-C₆-alkyl für: durch C₃-C₆-Alkinyloxy wie vorstehend genannt, vorzugsweise Propargyloxy, But-1-in-3-yloxy, But-1-in-4-yloxy oder But-2-in-1-yloxy, substituiertes C₁-C₆-Alkyl, also beispielsweise für Propargyloxymethyl oder 2-Propargyloxyethyl;
- C₃-C₆-Halogenalkenyl für: C₃-C₆-Alkenyl wie vorstehend genannt, das partiell oder vollständig durch Fluor, Chlor und/oder Brom substituiert ist, also z. B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl, insbesondere für 2-Chlorallyl oder 3,3-Dichlorallyl;
- C₃-C₆-Halogenalkinyl für: C₃-C₆-Alkinyl wie vorstehend genannt, das partiell oder vollständig durch Fluor, Chlor und/oder Brom substituiert ist, also z. B. 3-Chlorpropargyl, 3-Brompropargyl, 3-Fluorpropargyl, 3,3,3-Trifluorpropargyl, 4-Chlor-but-2-inyl, 4-Brom-but-2-inyl, 4,4,4-Trifluorbut-2-inyl, 1,4-Dichlor-but-2-inyl, 5-Chlor-pent-3-inyl, 5-Fluor-pent-3-inyl, 5,5,5-Trifluor-pent-3-inyl, 6-Chlorhex-2-inyl, vorzugsweise 3-Chlorpropargyl, 3,3,3-Trifluorpropargyl, 4,4,4-Trifluor-but-2-inyl;
- C₃-C₆-Cycloalkyl-C₁-C₆-alkyl für: Cyclopropylmethyl, Cyclobutylmethyl, Cyclopentylmethyl, Cyclohexylmethyl, Cycloheptylmethyl, Cyclooctylmethyl, 1-(Cyclopropyl)ethyl, 1-(Cyclobutyl)ethyl, 1-(Cyclopentyl)ethyl, 1-(Cyclohexyl)ethyl, 2-(Cyclopropyl)ethyl, 2-(Cyclobutyl)ethyl, 2-(Cyclopentyl)ethyl, 2-(Cyclohexyl)ethyl, 2-(Cyclopropyl)propyl, 3-(Cyclopropyl)propyl, 3-(Cyclobutyl)propyl, 3-(Cyclopentyl)propyl, 3-(Cyclohexyl)propyl, 2-(Cyclopropyl)butyl, 3-(Cyclopropyl)butyl, 4-(Cyclopropyl)butyl, 4-(Cyclobutyl)butyl, 4-(Cyclopentyl)butyl, 4-(Cyclohexyl)butyl, 2-(Cyclopropyl)pentyl, 3-(Cyclopropyl)pentyl, 4-(Cyclopropyl)pentyl, 5-(Cyclopropyl)pentyl, 2-(Cyclobutyl)pentyl, 3-(Cyclobutyl)pentyl, 5-(Cyclobutyl)pentyl, 2-(Cyclopropyl)hexyl, 3-(Cyclopropyl)hexyl, 6-(Cyclopropyl)hexyl insbesondere für Cyclopentylmethyl oder Cyclohexylmethyl;
- C₃-C₆-Halogencycloalkyl-C₁-C₆-alkyl: für C₃-C₆-Cycloalkyl-C₁-C₆-alkyl, wie vorstehend genannt, das am Cycloalkylring teilweise oder vollständig halogeniert ist und insbesondere 1, 2, 3, 4 oder 5 Halogenatome speziell Fluor oder Chlor trägt, z. B. für Perchlorcyclopropyl, Perfluorcyclopropyl, 1-Fluor- oder 1-Chlorcyclopropyl, Hexachlorcyclohexyl und vergleichbare;
- C₃-C₆-Cycloalkoxy für: Cyclopropoxy, Cyclobutoxy, Cyclopentoxy, oder Cyclohexoxy;
- C₃-C₆-Cycloalkoxy-C₁-C₄-alkyl für: Cyclopropyloxymethyl, Cyclobutyloxymethyl, Cyclopentyloxymethyl, Cyclohexyloxymethyl, 1-(Cyclopropyloxy)ethyl, 1-(Cyclobutyloxy)ethyl, 1-(Cyclopentyloxy)ethyl, 1-(Cyclohexyloxy)ethyl, 2-(Cyclopropyloxy)ethyl, 2-(Cyclobutyloxy)ethyl, 2-(Cyclopentyloxy)ethyl, 2-(Cyclohexyloxy)ethyl, 3-(Cyclopropyloxy)propyl, 3-(Cyclobutyloxy)propyl, 3-(Cyclopentyloxy)propyl, 3-(Cyclohexyloxy)propyl, 4-(Cyclopropyloxy)butyl, 4-(Cyclobutyloxy)butyl, 4-(Cyclopentyloxy)butyl oder 4-(Cyclohexyloxy)butyl, insbesondere für Cyclopentyloxymethyl, Cyclohexyloxymethyl oder 2-(Cyclopentyloxy)ethyl;
- C₆-C₁₄-aromatische Reste für: Phenyl, Naphthyl oder Anthracenyl oder für heteroaromatische Reste (aromatisches Heterocyclyl), die 6 bis 14 Ringkohlenstoffatome aufweisen, die teilweise d.h. wenigstens einfach, zweifach, dreifach, vierfach oder fünffach durch Heteroatome, ausgewählt unter N, O oder S, ersetzt sind. Daneben können C₆-C₁₄-aromatische Reste über zwei benachbarte Kohlenstoffatome mit einem 4 bis 7 gliedrigen Carbo- oder Heterocyclus verbunden sein, der gesättigt oder teilweise ungesättigt sein kann und neben den Kohlenstoffringgliedern ein, zwei oder drei der folgenden Hetero-Ringglieder: -O-, -S-, -S(O)₂-, -N=, -NH- oder -N(C₁-C₆-Alkyl)- aufweisen kann. Weiterhin können die C₆-C₁₄-aromatischen Reste einfach, zweifach, dreifach, vierfach oder fünffach mit den vorstehend definierten Atomgruppen substituiert sein.

Die im erfindungsgemäßen Verfahren als Einsatzstoffe verwendeten N'-substituierten N-Aminoharnstoffderivate der Formel II wie auch Verfahren zu deren Herstellung sind an sich bekannt und in der Literatur, z.B. in der WO 94/10173 beschrieben, so dass hier wegen näherer Einzelheiten auf diese entsprechende Literatur verwiesen werden soll.

Im Hinblick auf ihre Verwendung für die Herstellung von Verbindungen mit herbizider Wirkung bedeutet R vorzugsweise eine Gruppe der allgemeinen Formeln:
- CHO, CN, C(O)OR³, C(S)SR³, C(S)OR³, C(O)SR³, C(O)R², P(O)R¹OR¹, P(O)(OR¹)₂, S(O)₂R² oder SO₂NHR¹
insbesondere
- C(O)OR³, C(S)SR³, C(S)OR³, C(O)SR^{3,}
und speziell C(O)OR³.

Die Variablen R¹ bis R⁷ haben unabhängig voneinander vorzugsweise die folgenden Bedeutungen:
- R¹: Wasserstoff, C₁-C₆-Alkyl, C₁-C₆-Halogenalkyl, C₁-C₃-Alkoxy-C₁-C₃-alkyl, C₃-C₆-Alkenyl oder C₃-C₆-Alkinyl;
- R²: C₁-C₆-Alkyl, C₃-C₆-Cycloalkyl, C₃-C₆-Alkenyl, C₃-C₆-Alkinyl, C₁-C₆-Halogenalkyl, C₁-C₆-Alkoxy-C₂-C₆-alkyl, C₁-C₆-Alkoxycarbonyl-C₁-C₆-alkyl, C₁-C₆-Alkylthio-C₁-C₆-alkyl, C₃-C₆-Alkenyloxycarbonyl-C₁-C₆-alkyl, C₃-C₆-Alkinyloxycarbonyl-C₁-C₆-alkyl, C₃-C₆-Alkenyloxy-C₁-C₆-alkyl, C₃-C₆-Alkinyloxy-C₁-C₆-alkyl, C₁-C₆-Halogenalkoxy-C₁-C₆-alkyl, Cyano-C₁-C₆-alkyl, Halogen-C₂-C₆-alkenyl, Halogen-C₃-C₆-alkinyl, C₁-C₆-Alkylcarbonyl, CHR¹COR⁷, P(O)(OR⁷)₂, CHR¹P(S)(OR⁷)₂, CHR¹C(O)NR⁴R⁵, CHR¹C(O)NH₂,
Benzyl, Phenyl, Phenoxy-C₁-C₆-alkyl oder Benzyloxy-C₁-C₆-alkyl, wobei die vier letztgenannten Substituenten mit Halogen, C₁-C₄-Alkyl oder C₁-C₄-Halogenalkyl substituiert sein können;
- R³: C₁-C₁₅-Alkyl, C₃-C₈-Cycloalkyl, C₂-C₁₀-Alkenyl, C₃-C₁₀-Alkinyl, C₁-C₆-Halogenalkyl, C₁-C₆-Alkoxy-C₁-C₆-alkyl, C₁-C₆-Alkylthioalkyl, C₁-C₆-Alkoxycarbonyl-C₁-C₆-alkyl, C₃-C₆-Alkenyloxycarbonyl-C₁-C₆-alkyl, C₃-C₆-Alkinyloxycarbonyl-C₁-C₆-alkyl, C₃-C₆-Alkenyloxy-C₁-C₆-alkyl, C₁-C₆-Halogenalkoxy-C₁-C₆-alkyl, Cyano-C₁-C₆-alkyl, Halogen-C₃-C₆-alkenyl, C₁-C₆-Alkoxy-C₃-C₆-alkenyl, C₁-C₆-Alkoxy-C₃-C₆-alkinyl, CHR¹COR⁷, P(O)(OR⁷)₂, CHR¹P(S)(OR⁷)₂, CHR¹C(O)NR⁴R⁵, CHR¹C(O)NH₂,
Benzyl, Phenyl, Phenoxy-C₁-C₆-alkyl oder Benzyloxy-C₁-C₆-alkyl, wobei die vier letztgenannten Substituenten mit Halogen, C₁-C₄-Alkyl oder C₁-C₄-Halogenalkyl substituiert sein können;
- R⁴: Wasserstoff, C₁-C₆-Alkyl oder C₁-C₆-Alkoxy;
- R⁵,: R⁶ unabhängig voneinander Wasserstoff, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy-C₁-C₆-alkyl, C₁-C₆-Alkoxycarbonyl-C₁-C₆-alkyl, C₁-C₆-Alkoxycarbonyl-C₂-C₆-alkenyl,
Benzyl, welche ein- bis fünffach durch Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxycarbonyl, C₁-C₄-Alkoxycarbonyl-C₁-C₄-alkyl, Nitro oder Cyano substituiert sein können, oder
- R⁴ und R⁵: zusammen mit dem gemeinsamen Stickstoffatom für einen gesättigten oder ungesättigten 4- bis 7gliedrigen Azaheterocyclus stehen, der neben den Kohlenstoffringgliedern gewünschtenfalls eines der folgenden Glieder enthalten kann: -O-, -S-, -N=, -NH- oder -N(C₁-C₆-Alkyl)-;
- R⁷: Wasserstoff, C₁-C₆-Alkyl, C₁-C₆-Halogenalkyl, C₁-C₃-Alkoxy-C₁-C₃-alkyl, C₂-C₆-Cyanoalkyl, C₃-C₆-Alkenyl oder C₃-C₆-Alkinyl;
R¹ steht insbesondere für: C₁-C₃-Alkyl, C₁-C₃-Haloalkyl, C₁-C₃-Alkoxy-C₁-C₃-alkyl;
R² steht insbesondere für: C₁-C₆-Alkyl, C₃-C₆-Cycloalkyl, C₃-C₄-Alkenyl, C₃-C₄-Alkinyl, C₁-C₃-Haloalkyl, C₁-C₃-Alkoxy-C₁-C₃-alkyl, C₁-C₃-Alkoxycarbonyl-C₁-C₃-alkyl, Cyano-C₁-C₃-alkyl, Halo-C₂-C₄-alkenyl, Halo-C₃-C₄-alkinyl, C₁-C₃-Alkylcarbonyl, CHR¹COR⁷, P(O)(OR⁷)₂, CHR¹P(S)(OR⁷)₂, CHR¹C(O)NR⁴R⁵, CHR¹C(O)NH₂, C₁-C₃-Alkyl, welches mit Phenoxy oder Benzyloxy substituiert ist, Benzyl oder Phenyl, welche mit Halogen, Methyl oder CF₃ substituiert sein können;
R³ steht insbesondere für: C₁-C₁₅-Alkyl, C₃-C₈-Cycloalkyl, C₂-C₆-Alkenyl, C₃-C₆-Alkinyl, C₁-C₆-Haloalkyl, C₁-C₃-Alkoxy-C₁-C₃-alkyl, C₁-C₃-Alkoxycarbonyl-C₁-C₃-alkyl, C₃-C₄-Alkenyloxycarbonyl-C₁-C₃-alkyl, C₃-C₄-Alkinyloxycarbonyl-C₁-C₃-alkyl, Cyano-C₁-C₆-alkyl, Halo-C₃-C₄-alkenyl, CHR¹COR⁷, P(O)(OR⁷)₂, CHR¹C(O)NR⁴R⁵, CHR¹C(O)NH₂, C₁-C₃-Alkyl, welches mit Phenoxy oder Benzyloxy substituiert ist, Benzyl oder Phenyl welche mit Halogen, Methyl oder CF₃ substituiert sein können;

Die Variable m hat insbesondere den Wert 0; Z steht vorzugsweise für Sauerstoff oder Schwefel, insbesondere für Sauerstoff.

Beispiele für bevorzugte Perhydrodiazine der Formel I sind die Verbindungen der allgemeinen Formel Ia, worin R die in Tabelle 1 angegebenen Bedeutungen aufweist.

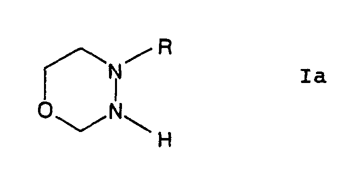

**Tabelle 1**

| Nr. | R |
|---|---|
| 1 | CO₂H |
| 2 | CHO |
| 3 | CN |
| 4 | C(O)NHCH₃ |
| 5 | C(O)NHC₂H₅ |
| 6 | C(O)NH-N-C₃H₇ |
| 7 | C(O)NH-i-C₃H₇ |
| 8 | C(O)NH-c-C₃H₅ |
| 9 | C(O)NHCH₂CH=CH₂ |
| 10 | C(O)NHCH₂-C≡CH |
| 11 | C(O)NHCH₂CH₂OCH₃ |
| 12 | C(O)NHCH₂CO₂CH₃ |
| 13 | C(O)NHCH(CH₃)CO₂CH₃ |
| 14 | C(O)N(CH₃)₂ |
| 15 | C(O)N(CH₃)C₂H₅ |
| 16 | C(O)NHCH₂C₆H₅ |
| 17 | C(O)N(CH₃)CH₂C₆H₅ |
| 18 | C(O)NH-2-Cl-benzyl |
| 19 | C(O)NH-3-Cl-benzyl |
| 20 | C(O)NH-4-Cl-benzyl |
| 21 | C(O)NH-2-CH₃-benzyl |
| 22 | C(O)NH-3-CH₃-benzyl |
| 23 | C(O)NH-4-CH₃-benzyl |
| 24 | C(O)NH-2,3-Cl₂-benzyl |
| 25 | C(O)NH-3,4-Cl₂-benzyl |
| 26 | C(O)NH-2,4-Cl₂-benzyl |
| 27 | C(O)NH-3-CF₃-benzyl |
| 28 | C(O)NH-4-CF₃-benzyl |
| 29 | C(O)NH-3-NO₂-benzyl |
| 30 | C(O)NH-4-NO₂-benzyl |
| 31 | C(O)N(CH₂)₅ |
| 32 | C(O)N(CH₂CH₂OCH₂CH₂) |
| 33 | C(O)N(CH₂CH₂N(CH₃)CH₂CH₂) |
| 34 | C(O)NHSO₂CH₃ |
| 35 | C(O)NHSO₂C₂H₅ |
| 36 | C(O)NHSO₂-n-C₃H₇ |
| 37 | C(O)NHSO₂-i-C₃H₇ |
| 38 | C(O)NHCO₂CH₃ |
| 39 | C(O)NHCO₂C₂H₅ |
| 40 | C(O)NHCO₂-n-C₃H₇ |
| 41 | C(O)NHCO₂-i-C₃H₇ |
| 42 | C(O)NHCO₂-c-C₃H₅ |
| 43 | C(O)NHSO₃CH₃ |
| 44 | C(O)NHSO₃C₂H₅ |
| 45 | C(O)NHSO₃-n-C₃H₇ |
| 46 | C(O)NHSO₃-i-C₃H₇ |
| 47 | C(O)NHSO₃-c-C₃H₅ |
| 48 | C(O)CH₃ |
| 49 | C(O)C₂H₅ |
| 50 | C(O)-n-C₃H₇ |
| 51 | C(O)-i-C₃H₇ |
| 52 | C(O)-c-C₃H₅ |
| 53 | C(O)-n-C₄H₉ |
| 54 | C(O)-sek.-C₄H₉ |
| 55 | C(O)-i-C₄H₉ |
| 56 | C(O)-t-C₄H₉ |
| 57 | C(O)CH₂CH=CH₂ |
| 58 | C(O)CH₂C≡CH |
| 59 | C(O)-c-C₃H₅ |
| 60 | C(O)-c-C₅H₉ |
| 61 | C(O)CH₂Cl |
| 62 | C(O)CF₃ |
| 63 | C(O)CH₂CF₃ |
| 64 | C(O)CH₂OCH₃ |
| 65 | C(O)CH₂OC₂H₅ |
| 66 | C(O)CH₂CO₂CH₃ |
| 67 | C(O)CH₂CH₂CO₂CH₃ |
| 68 | C(O)CH₂CH₂CO₂CH₂CH=CH₂ |
| 69 | C(O)CH₂CH₂CO₂CH₂C≡CH |
| 70 | C(O)CH₂CN |
| 71 | C(O)CH₂CH₂CN |
| 72 | C(O)CH₂-C(Cl)=CH₂ |
| 73 | C(O)CH=CH₂ |
| 74 | C(O)CH₂C(O)CH₃ |
| 75 | C(O)CH₂P(O)(OCH₃)₂ |
| 76 | C(O)CH(CH₃)P(O)(OCH₃)₂ |
| 77 | C(O)CH₂C(O)N(CH₃)₂ |
| 78 | C(O)-benzyl |
| 79 | C(O)-3-CH₃-benzyl |
| 80 | C(O)-4-CH₃-benzyl |
| 81 | C(O)-3-Cl-benzyl |
| 82 | C(O)-4-Cl-benzyl |
| 83 | C(O)-3-CF₃-benzyl |
| 84 | C(O)-4-CF₃-benzyl |
| 85 | C(O)-phenyl |
| 86 | C(O)-3-CH₃-phenyl |
| 87 | C(O)-4-CH₃-phenyl |
| 88 | C(O)-3-Cl-phenyl |
| 89 | C(O)-4-Cl-phenyl |
| 90 | C(O)-3-CF₃-phenyl |
| 91 | C(O)-4-CF₃-phenyl |
| 92 | C(O)-2-pyridyl |
| 93 | C(O)-3-pyridyl |
| 94 | C(O)-4-pyridyl |
| 95 | P(O)(CH₃)OCH₃ |
| 96 | P(O)(C₂H₅)OCH₃ |
| 97 | P(O)(CH₃)OC₂H₅ |
| 98 | P(O)(CH₃)O-n-C₃H₇ |
| 99 | P(O)(OCH₃)₂ |
| 100 | P(O)(OC₂H₅)₂ |
| 101 | P(O)(OCH₃)OC₂H₅ |
| 102 | SOCH₃ |
| 103 | SOC₂H₅ |
| 104 | SO₂CH₃ |
| 105 | S0₂C₂H₅ |
| 106 | SO₂-n-C₃H₇ |
| 107 | SO₂-i-C₃H₇ |
| 108 | SO₂-c-C₃H₅ |
| 109 | SO₂-sek.-C₄H₉ |
| 110 | SO₂-CH₂CH=CH₂ |
| 111 | SO₂CH₂Cl |
| 112 | SO₂CF₃ |
| 113 | SO₂CH₂CF₃ |
| 114 | SO₂CH₂OCH₃ |
| 115 | SO₂CH₂OC₂H₅ |
| 116 | SO₂CH₂CO₂CH₃ |
| 117 | SO₂CH₂CN |
| 118 | SO₂CH₂CH₂CN |
| 119 | SO₂CH=CH₂ |
| 120 | SO₂-benzyl |
| 121 | SO₂-3-CH₃-benzyl |
| 122 | SO₂-4-CH₃-benzyl |
| 123 | SO₂-3-Cl-benzyl |
| 124 | SO₂-4-Cl-benzyl |
| 125 | SO₂-3-CF₃-benzyl |
| 126 | SO₂-4-CF₃-benzyl |
| 127 | S(O)-phenyl |
| 128 | S(O)-3-Cl-phenyl |
| 129 | S(O)-4-Cl-phenyl |
| 130 | SO₂-3-Cl-phenyl |
| 131 | SO₂-4-Cl-phenyl |
| 132 | SO₂-3-CF₃-phenyl |
| 133 | SO₂-4-CF₃-phenyl |
| 134 | SO₂-3-CH₃-phenyl |
| 135 | SO₂-4-CH₃-phenyl |
| 136 | SO₂-3-pyridyl |
| 137 | SO₂-4-pyridyl |
| 138 | SO₂NH₂ |
| 139 | SO₂NHCH₃ |
| 140 | SO₂NHC₂H₅ |
| 141 | SO₂NH-i-C₃H₇ |
| 142 | SO₂NH-n-C₃H₇ |
| 143 | SO₂NHCH₂CH₂Cl |
| 144 | SO₂NH-t-C₄H₉ |
| 145 | SO₂NH-n-C₄H₉ |

In der Tabelle 1 steht i für "iso", t für "tertiär" und c für "cyclo".

Beispiele für bevorzugte Perhydrodiazine der Formel I sind außerdem die Verbindungen der allgemeinen Formel Ib, worin R die in der Tabelle 1 angegebenen Bedeutungen hat. 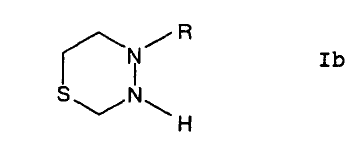 (Verbindungen Ib Nr.1 - Ib Nr.145).

Ganz besonders bevorzugte Verbindungen der Formel I sind die Verbindungen der allgemeinen Formel Ib.1, worin R für COOR³ steht, insbesondere solche worin R³ die in Tabelle 2 angegebenen Bedeutungen aufweist.

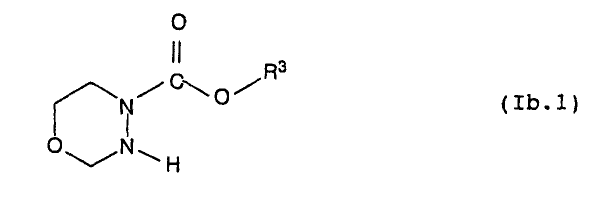

**Tabelle 2**

| Nr. | R³ |
|---|---|
| 1 | CH₃ |
| 2 | C₂H₅ |
| 3 | n-C₃H₇ |
| 4 | i-C₃H₇ |
| 5 | c-C₃H₅ |
| 6 | n-C₄H₉ |
| 7 | i-C₄H₉ |
| 8 | sek.-C₄H₉ |
| 9 | c-C₄H₇ |
| 10 | t-C₄H₉ |
| 11 | n-C₅H₁₁ |
| 12 | sek.-C₅H₁₁ |
| 13 | 2,2-dimethylpropyl |
| 14 | 2-methylbutyl |
| 15 | 3-methylbutyl |
| 16 | 1,1-dimethylpropyl |
| 17 | 1,2-dimethylpropyl |
| 18 | c-C₅H₉ |
| 19 | n-C₆H₁₃ |
| 20 | 1,1-dimethylbutyl |
| 21 | 1,2-dimethylbutyl |
| 22 | 1,3-dimethylbutyl |
| 23 | 2,2-dimethylbutyl |
| 24 | 2,3-dimethylbutyl |
| 25 | 3,3-dimethylbutyl |
| 26 | 1-ethylpropyl |
| 27 | 1-ethylbutyl |
| 28 | 2-ethylbutyl |
| 29 | 1,1,2-trimethylpropyl |
| 30 | 1,2,2-trimethylpropyl |
| 31 | 1-methylpentyl |
| 32 | 1-ethyl-1-methylpropyl |
| 33 | 1-ethyl-2-methylpropyl |
| 34 | c-C₆H₁₁ |
| 35 | n-C₇H₁₅ |
| 36 | 1-methylhexyl |
| 37 | 2-methylhexyl |
| 38 | 3-methylhexyl |
| 39 | 4-methylhexyl |
| 40 | 5-methylhexyl |
| 41 | 1-ethylpentyl |
| 42 | 2-ethylpentyl |
| 43 | 3-ethylpentyl |
| 44 | 4-ethylpentyl |
| 45 | 1,1-dimethylpentyl |
| 46 | 1,2-dimethylpentyl |
| 47 | 1,3-dimethylpentyl |
| 48 | 1,4-dimethylpentyl |
| 49 | c-C₇H₁₃ |
| 50 | n-C₈H₁₇ |
| 51 | 1-methylheptyl |
| 52 | 2-methylheptyl |
| 53 | 3-methylheptyl |
| 54 | 4-methylheptyl |
| 55 | 5-methylheptyl |
| 56 | 6-methylheptyl |
| 57 | 1-ethylhexyl |
| 58 | 2-ethylhexyl |
| 59 | 3-ethylhexyl |
| 60 | 4-ethylhexyl |
| 61 | 1,1-dimethylhexyl |
| 62 | 1,2-dimethylhexyl |
| 63 | 1,3-dimethylhexyl |
| 64 | 1,4-dimethylhexyl |
| 65 | 1,5-dimethylhexyl |
| 66 | c-C₈H₁₅ |
| 67 | n-C₉H₁₉ |
| 68 | 1-methyloctyl |
| 69 | 2-methyloctyl |
| 70 | 3-methyloctyl |
| 71 | 4-methyloctyl |
| 72 | 5-methyloctyl |
| 73 | 6-methyloctyl |
| 74 | 7-methyloctyl |
| 75 | 1-ethylheptyl |
| 76 | 2-ethylheptyl |
| 77 | 3-ethylheptyl |
| 78 | 4-ethylheptyl |
| 79 | 5-ethylheptyl |
| 80 | 6-ethylheptyl |
| 81 | 1,1-dimethylheptyl |
| 82 | 1,2-dimethylheptyl |
| 83 | 1,3-dimethylheptyl |
| 84 | 1,4-dimethylheptyl |
| 85 | 1,5-dimethylheptyl |
| 86 | 1,6-dimethylheptyl |
| 87 | c-C₉H₁₇ |
| 88 | n-C₁₀H₂₁ |
| 89 | 1-methylnonyl |
| 90 | 2-methylnonyl |
| 91 | 3-methylnonyl |
| 92 | 4-methylnonyl |
| 93 | 5-methylnonyl |
| 94 | 1-ethyloctyl |
| 95 | 2-ethyloctyl |
| 96 | 3-ethyloctyl |
| 97 | 4-ethyloctyl |
| 98 | 5-ethyloctyl |
| 99 | 6-ethyloctyl |
| 100 | 7-ethyloctyl |
| 101 | 8-ethyloctyl |
| 102 | 1,1-dimethyloctyl |
| 103 | 1,2-dimethyloctyl |
| 104 | 1,3-dimethyloctyl |
| 105 | 1,4-dimethyloctyl |
| 106 | 1,5-dimethyloctyl |
| 107 | 1,6-dimethyloctyl |
| 108 | 1,7-dimethyloctyl |
| 109 | c-C₁₀H₁₉ |
| 110 | n-C₁₁H₂₃ |
| 111 | 1-methyldecyl |
| 112 | 1-ethylnonyl |
| 113 | 1,1-dimethylnonyl |
| 114 | n-dodecyl |
| 115 | 1-methylundecyl |
| 116 | 1-ethyldecyl |
| 117 | 1,1-dimethyldecyl |
| 118 | 1-propylnonyl |
| 119 | n-tridecyl |
| 120 | 1-methyldodecyl |
| 121 | 1-ethylundecyl |
| 122 | 1,1-dimethylundecyl |
| 123 | n-tetradecyl |
| 124 | 1-methyltridecyl |
| 125 | 1-ethyldodecyl |
| 126 | 1,1-dimethyldodecyl |
| 127 | n-Pentadecyl |
| 128 | CH₂CH=CH₂ |
| 129 | CH₂C≡CH |
| 130 | CH₂C(CH₃)=CH₂ |
| 131 | CH(CH₃)CH=CH₂ |
| 132 | CH(CH₃)C≡CH |
| 133 | C(CH₃)₂CH=CH₂ |
| 134 | C(CH₃)₂C≡CH |
| 135 | CH₂CH=CH-CH₃ |
| 136 | CH₂-C≡C-CH₃ |
| 137 | (CH₂)₂CH=CH-CH₃ |
| 138 | (CH₂)₂C≡C-CH₃ |
| 139 | (CH₂)₃CH=CH₂ |
| 140 | (CH₂)₃C≡CH |
| 141 | (CH₂)₃CH=CHCH₃ |
| 142 | (CH₂)₃C≡C-CH₃ |
| 143 | CH₂CH₂Cl |
| 144 | CH₂CH₂Br |
| 145 | (CH₂)₃Cl |
| 146 | CH(CH₃)CH₂Cl |
| 147 | (CH₂)₂F |
| 148 | (CH₂)₃F |
| 149 | (CH₂)₄Cl |
| 150 | (CH₂)₅Cl |
| 151 | C(CH₃)₂CH₂Cl |
| 152 | CH₂CH₂OCH₃ |
| 153 | CH(CH₃)CH₂OCH₃ |
| 154 | C(CH₃)₂CH₂OCH₃ |
| 155 | (CH₂)₃OCH₃ |
| 156 | (CH₂)₂OC₂H₅ |
| 157 | CH(CH₃)CH₂OC₂H₅ |
| 158 | CH₂CH₂SCH₃ |
| 159 | CH(CH₃)CH₂SCH₃ |
| 160 | C(CH₃)₂CH₂SCH₃ |
| 161 | (CH₂)₂SC₂H₅ |
| 162 | CH(CH₃)CH₂SC₂H₅ |
| 163 | CH₂CH₂S(O)CH₃ |
| 164 | CH₂CH₂SO₂CH₃ |
| 165 | CH₂CH₂S(O)C₂H₅ |
| 166 | CH₂CH₂SO₂C₂H₅ |
| 167 | CH₂CH₂OCH₂CH₂OCH₃ |
| 168 | CH₂CH₂OCH₂CH₂OC₂H₅ |
| 169 | CH(CH₃)CH₂OCH₂CH₂OCH₃ |
| 170 | CH₂-c-C₃H₅ |
| 171 | CH₂-C-C₄H₇ |
| 172 | CH₂-c-C₅H₉ |
| 173 | CH₂-c-C₆H₁₁ |
| 174 | CH₂CO₂H |
| 175 | CH(CH₃)CO₂H |
| 176 | (CH₂)₂CO₂H |
| 177 | CH₂CO₂CH₃ |
| 178 | CH(CH₃)CO₂CH₃ |
| 179 | CH₂CH₂-O-CH₂CH=CH₂ |
| 180 | CH₂CH₂O-CH₂C≡CH |
| 181 | CH₂CH₂O-CH₂CH₂Cl |
| 182 | C(CH₃)₂CO₂CH₂CH=CH₂ |
| 183 | C(CH₃)₂CO₂CH₂C≡CH |
| 184 | (CH₂)₂O-c-C₃H₅ |
| 185 | (CH₂)₂O-c-C₅H₇ |
| 186 | (CH₂)₂O-CH₂-CH=CHCl |
| 187 | (CH₂)₂S-CH₂CH=CHCl |
| 188 | (CH₂)₂S-CH₂CH=CH₂ |
| 189 | (CH₂)₂S-CH₂C≡CH |
| 190 | (CH₂)₂CN |
| 191 | (CH₂)₃CN |
| 192 | CH(CH₃)CN |
| 193 | (CH₂)₄CN |
| 194 | (CH₂)₅CN |
| 195 | (CH₂)₆CN |
| 196 | CH₂C(O)H |
| 197 | CH₂C(O)CH₃ |
| 198 | CH₂C(O)C₂H₅ |
| 199 | CH₂C(O)CH₂OCH₃ |
| 200 | CH₂C(O)(CH₂)₂OCH₃ |
| 201 | CH₂C(O)CH₂CN |
| 202 | CH₂C(O)CH=CH₂ |
| 203 | CH₂P(O)(OCH₃)₂ |
| 204 | (CH₂)₂P(O)(OCH₃)₂ |
| 205 | CH₂P(S)(OCH₃)₂ |
| 206 | CH₂C(O)NHCH₃ |
| 207 | CH₂C(O)N(CH₃)₂ |
| 208 | (CH₂)₂C(O)N(CH₃)₂ |
| 209 | CH₂OC₆H₅ |
| 210 | CH₂O-p-Cl-phenyl |
| 211 | CH₂O-p-tolyl |
| 212 | (CH₂)₂O-C₆H₅ |
| 213 | (CH₂)₂O-p-Cl-phenyl |
| 214 | (CH₂)₂O-p-tolyl |
| 215 | C₆H₅ |
| 216 | p-Cl-phenyl |
| 217 | m-Cl-phenyl |
| 218 | o-Cl-phenyl |
| 219 | o-tolyl |
| 220 | m-tolyl |
| 221 | p-tolyl |
| 222 | p-CH₃O-phenyl |
| 223 | m-CH₃O-phenyl |
| 224 | o-CH₃O-phenyl |
| 225 | 2,3-dichlorphenyl |
| 226 | 2,4-dichlorphenyl |
| 227 | 3-CH₃-4-chlorphenyl |
| 228 | 3-CH₃O-4-chlorphenyl |
| 229 | 2-CF₃-phenyl |
| 230 | 3-CF₃-phenyl |
| 231 | 4-CF₃-phenyl |
| 232 | 2-Cl-4-CF₃-phenyl |
| 233 | 3-Cl-4-CH₃O-phenyl |
| 234 | 3-F₂CH-O-phenyl |
| 235 | 4-F₂CH-O-phenyl |
| 236 | 2-NO₂-phenyl |
| 237 | 3-NO₂-phenyl |
| 238 | 4-NO₂-phenyl |
| 239 | 2-CN-phenyl |
| 240 | 3-CN-phenyl |
| 241 | 4-CN-phenyl |
| 242 | 2-CH₃OC(O)-phenyl |
| 243 | 3-CH₃OC(O)-phenyl |
| 244 | 4-CH₃OC(O)-phenyl |
| 245 | 3-CH₃O-C(O)CH₂O-phenyl |
| 246 | 3-CH₃O-C(O)CH(CH₃)O-phenyl |
| 247 | 4-CH₃O-C(O)CH₂O-phenyl |
| 248 | 4-CH₃O-C(O)C(CH₃)₂O-phenyl |
| 249 | 2-pyridyl |
| 250 | 3-pyridyl |
| 251 | 4-pyridyl |
| 252 | 1-naphthyl |
| 253 | 2-naphthyl |
| 254 | 2-chlor-1-naphthyl |
| 255 | 3-chlor-1-naphthyl |
| 256 | 4-chlor-1-naphthyl |
| 257 | 5-chlor-1-naphthyl |
| 258 | 2-chlor-4-nitro-1-naphthyl |
| 259 | 2-methyl-4-nitro-1-naphthyl |
| 260 | 5-chlor-8-methyl-1-naphthyl |
| 261 | 2-chinolyl |
| 262 | 3-chinolyl |
| 263 | 4-chinolyl |
| 264 | 5-chinolyl |
| 265 | 8-chinolyl |
| 266 | 2-chinazolyl |
| 267 | 4-chinazolyl |
| 268 | 1-chinoxalyl |
| 269 | 1-methylindolyl-2 |
| 270 | 1-methylindolyl-3 |
| 271 | 1-methylbenzimidazolyl-2 |
| 272 | benzofuran-2-yl |
| 273 | benzofuran-3-yl |
| 274 | benzofuran-4-yl |
| 275 | benzofuran-5-yl |
| 276 | benzofuran-6-yl |
| 277 | 3-methylbenzofuran-5-yl |
| 278 | benzothienyl-2 |
| 279 | benzothienyl-3 |
| 280 | benzoxazolyl-3 |
| 281 | benzoxazolyl-4 |
| 282 | benzoxazolyl-5 |
| 283 | benzthiazolyl-3 |
| 284 | benzthiazolyl-4 |
| 285 | benzthiazolyl-5 |
| 286 | benzyl |
| 287 | 2-CH₃-benzyl |
| 288 | 3-CH₃-benzyl |
| 289 | 4-CH₃-benzyl |
| 290 | 2-Cl-benzyl |
| 291 | 3-Cl-benzyl |
| 292 | 4-Cl-benzyl |
| 293 | 2,3-dichlorbenzyl |
| 294 | 2,4-dichlorbenzyl |
| 295 | 2-CH₃-4-Cl-benzyl |
| 296 | 2-Cl-4-CH₃-benzyl |
| 297 | 2,6-Cl₂-4-CH₃benzyl |
| 298 | 2-CH₃O-benzyl |
| 299 | 3-CH₃O-benzyl |
| 300 | 4-CH₃O-benzyl |
| 301 | 2-NO₂-benzyl |
| 302 | 3-NO₂-benzyl |
| 303 | 4-NO₂-benzyl |
| 304 | 2-CN-benzyl |
| 305 | 3-CN-benzyl |
| 306 | 4-CN-benzyl |

In Tabelle 2 steht "o" für ortho, "m" für meta und "p" für para und bezeichnet die Position des nachfolgend aufgeführten Substituenten am Phenylring.

Ganz besonders bevorzugt sind auch die Verbindungen der allgemeinen Formel Ib.2, 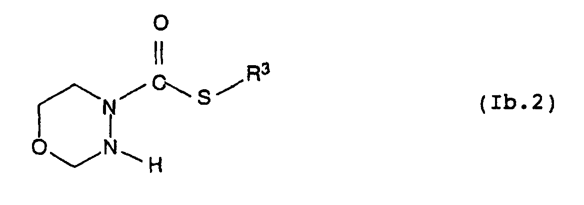 worin R³ die in den Zeilen 1 bis 306 der Tabelle 2 genannten Bedeutungen aufweist.

Ganz besonders bevorzugt sind weiterhin die Verbindungen der allgemeinen Formel Ib.3, 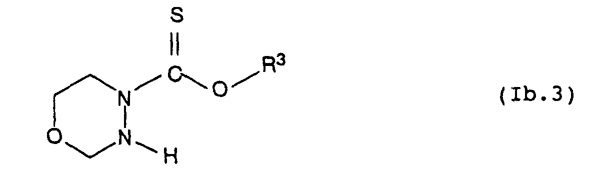 worin R³ die in den Zeilen 1 bis 306 der Tabelle 2 genannten Bedeutungen aufweist.

Ganz besonders bevorzugt sind weiterhin die Verbindungen der allgemeinen Formel Ib.4, 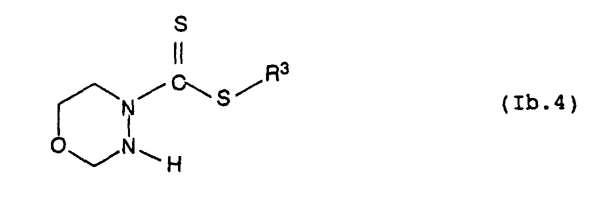 worin R³ die in den Zeilen 1 bis 306 der Tabelle 2 genannten Bedeutungen aufweist.

Ganz besonders bevorzugt sind weiterhin die Verbindungen der allgemeinen Formel Ib.5, 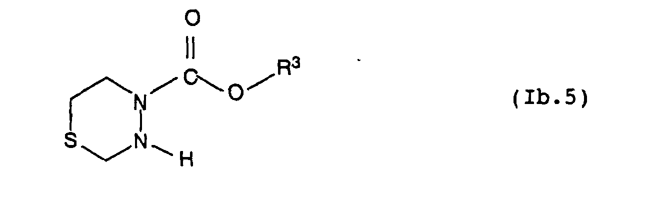 worin R³ die in den Zeilen 1 bis 306 der Tabelle 2 genannten Bedeutungen aufweist.

Ganz besonders bevorzugt sind weiterhin die Verbindungen der allgemeinen Formel Ib.6, 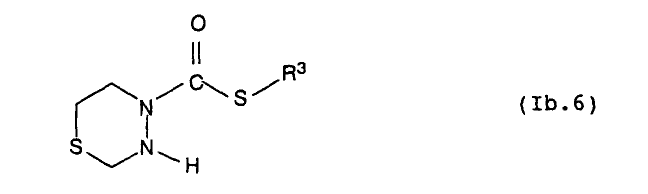 worin R³ die in den Zeilen 1 bis 306 der Tabelle 2 genannten Bedeutungen aufweist.

Ganz besonders bevorzugt sind weiterhin die Verbindungen der allgemeinen Formel Ib.7, 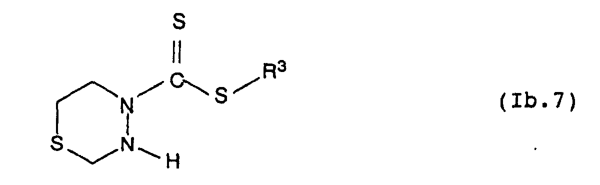 worin R³ die in den Zeilen 1 bis 306 der Tabelle 2 genannten Bedeutungen aufweist.

Ganz besonders bevorzugt sind weiterhin die Verbindungen der allgemeinen Formel Ib.8, 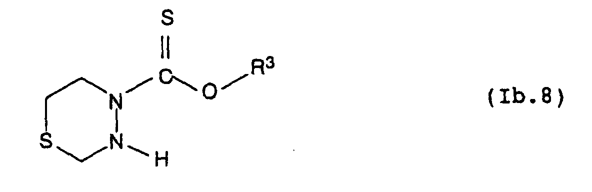 worin R³ die in den Zeilen 1 bis 306 der Tabelle 2 genannten Bedeutungen aufweist.

Die Perhydrodiazine der allgemeinen Formel 1 werden erfindungsgemäß hergestellt, indem man zunächst ein substituiertes Hydrazin der Formel II 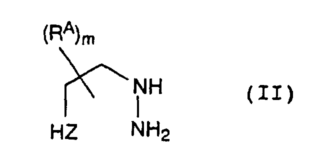 worin Z für O oder S steht und R^{A} und m die vorgenannte Bedeutung haben, mit einem Säurederivat der Formel III,

R-G (III)

worin R die vorgenannte Bedeutung hat und G für eine nucleophil verdrängbare Abgangsgruppe steht, oder R-G ein Isocyanat der allgemeinen Formel R⁵-N=C=X oder ein Keten der allgemeinen Formel R^{2a}(R^{2b})C=C=X bedeutet, worin X für O oder S steht und R^{2a}(R^{2b})C-H den Rest R² bedeutet, wobei R² und R⁵ die obengenannten Bedeutungen aufweisen kann, umsetzt, und das so erhaltene Hydrazinderivat der Formel IV 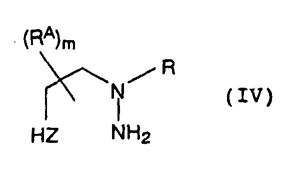 in einem zweiten Schritt mit Formaldehyd in Gegenwart einer Säure zu den substituierten Perhydrodiazinen der allgemeinen Formel I mit Z = O oder S cyclisiert. Gegebenenfalls kann man die Reste R in den Verbindungen der Formel I anschließend nach Standardverfahren in andere Reste R umwandeln bzw. derivatisieren; und/oder man kann in einem weiteren Reaktionsschritt Verbindungen I mit Z = S zu den Sulfoxiden (Z = SO) oder Sulfonen (Z = SO₂) oxidieren.

Beispiele für geeignete, nucleophil verdrängbare Abgangsgruppen sind Halogen, vorzugsweise Chlor oder Brom, C₁-C₆-Alkoxy wie Methoxy, Ethoxy, n-Propoxy, n-Butoxy, C₁-C₄-Halogenalkoxy wie Trichlormethoxy, Trifluormethoxy, Pentafluorethoxy, N-gebundenes Heterocyclyl wie Imidazolyl, C₁-C₆-Alkylcarbonyloxy (bzw. C₁-C₆-Alkanoat) wie Acetat, Propionat, n-Butyrat, Isobutyrat, Pivalat und Capronat, C₁-C₆-Halogenalkylcarbonyloxy wie Mono-, Di- und Trichloracetat, C₁-C₆-Alkylsulfonyloxy wie Methylsulfonyloxy,
C₁-C₆-Halogenalkylsulfonyloxy wie Trifluormethylsulfonyloxy, Phenylsulfonyloxy, worin der Phenylrest gegebenenfalls mit Halogen oder C₁-C₆-Alkyl ein- oder zweifach substituiert sein kann, wie Phenylsulfonyloxy, p-Toluolsulfonyloxy und p-Cl-Phenylsulfonyloxy, N-gebundenes Stickstoff-C₅-C₆-Heterocyclyl wie N-Imidazolyl.

Bevorzugte Abgangsgruppe ist Halogen, insbesondere Chlor oder Brom, sowie weiterhin Acetat oder Trifluoracetat insbesondere wenn R für eine Gruppe C(O)OR³, C(S)OR³, C(S)SR³, C(O)SR³, P(O)R¹(OR¹), P(O)(OR¹)₂, S(O)₂R² oder SO₂HNR¹ steht.

Erfindungsgemäß kann man die Verbindungen der allgemeinen Formel I auch herstellen, indem man im ersten Reaktionsschritt ein substituiertes Hydrazin mit einem Isocyanat oder einem Isothiocyanat der allgemeinen Formel R'-N=C=X, , worin R' für R⁵ steht, oder R' eine Gruppe R⁶-W mit W = CO₂, S(O)₂ oder S(O)₂O bedeutet, umsetzt. R⁵ und R⁶ haben hierbei die obengenannten Bedeutungen. In den so erhaltenen Hydraziden der allgemeinen Formel IV weist R dann die Bedeutungen C(O)NR⁴R⁵ mit R⁴ = H, C(S)NR⁴R⁵ mit R⁴ = H, C(O)NHCO₂R⁶, C(O)NHS(O)₂R⁶ oder C(O)NHS(O)₂OR⁶ auf.

Erfindungsgemäß kann man die Verbindungen der allgemeinen Formel I, worin R für C(O)R² steht, auch herstellen, indem man die substituierten Hydrazine mit einem Keten der allgemeinen Formel (R^{2a})(R^{2b})C=C=O umsetzt. R steht dann für einen Rest C(O)R² mit R² = HC(R^{2a})(R^{2b}).

In einem zweiten Reaktionsschritt setzt man dann die Hydrazide der allgemeinen Formel (IV) mit Formaldehyd zu den erfindungsgemäßen 1-Oxa-3,4-diazinen bzw. 1-Thia-3,4-diazinen der allgemeinen Formel I mit Z = O oder S um. Die Umsetzung kann sowohl mit Formaldehyd oder einer Verbindung, die unter sauren Bedingungen Formaldehyd freisetzt, wie Paraformaldehyd oder 1,3,5-Trioxan, in Gegenwart einer Säure erfolgen.

Man kann jedoch auch die Hydrazide IV mit Formaldehyd zu den Verbindungen der allgemeinen Formel IVa umsetzen und diese dann zu den erfindungsgemäßen 1-Oxa- bzw. 1-Thiadiazinen cyclisieren.

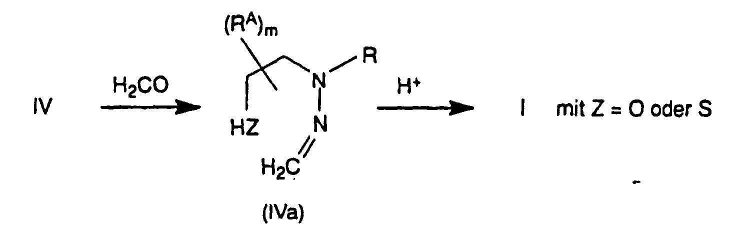

Die Cyclisierung erfolgt üblicherweise unter sauren Bedingungen.

Für die Darstellung der Verbindungen I steht beispielhaft die in folgendem Schema beschriebene Umsetzung, wobei ausgehend von 2-Hydrazinoethanol und Methylchlorformiat als Säurederivat zunächst das N-Amino-N-methoxycarbonyl-2-hydrazinoethanol hergestellt wird, das in einer Folgereaktion mit Formaldehyd zum Tetrahydro-4-methoxycarbonyl-4H-1-oxa-3,4-diazin cyclisiert wird.

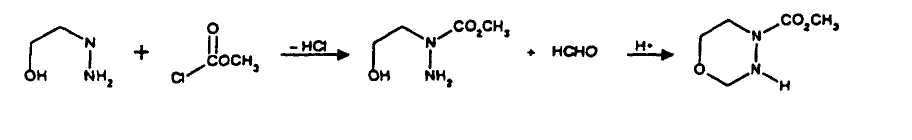

Bevorzugte Ausführungsformen des Verfahrens sind im folgenden genannt:

Im Folgenden wird der erste Reaktionsschritt näher erläutert: Die Umsetzung der Hydrazinoethanole/thiole II mit den Verbindungen der Formel III wird vorteilhaft in Gegenwart eines Lösungsmittels bei Temperaturen im Bereich von -30 bis 100°C, vorzugsweise -10 bis 80°C, besonders bevorzugt 0 bis 60°C durchgeführt.

Als Lösungsmittel verwendet man für diese Umsetzungen - je nach Temperaturbereich - Kohlenwasserstoffe wie Pentan, Hexan, Cyclopentan, Cyclohexan, Toluol, Xylol, chlorierte Kohlenwasserstoffe wie Methylenchlorid, Chloroform, 1,2-Dichlorethan, 1,1,2,2-Tetrachlorethan, Chlorbenzol, 1,2-, 1,3- oder 1,4-Dichlorbenzol, Ether wie 1,4-Dioxan, Anisol, Glykolether wie Dimethylglykolether, Diethylglykolether, Diethylenglykoldimethylether, Ester wie Ethylacetat, Propylacetat, Methylisobutyrat, Isobutylacetat, Carbonsäureamide wie DMF, N-Methylpyrrolidon, Nitrokohlenwasserstoffe wie Nitrobenzol, Harnstoffe wie Tetraethylharnstoff, Tetrabutylharnstoff, Dimethylethylenharnstoff, Dimethylpropylenharnstoff, Sulfoxide wie Dimethylsulfoxid, Sulfone wie Dimethylsulfon, Diethylsulfon, Tetramethylensulfon, Nitrile wie Acetonitril, Propionitril, Butyronitril oder Isobutyronitril; Wasser oder auch Gemische einzelner Lösungsmittel.

Die molaren Verhältnisse, in denen die Ausgangsverbindungen II und III miteinander umgesetzt werden, betragen im Allgemeinen 0,9 bis 1,2, vorzugsweise 0,95 bis 1,1, besonders bevorzugt 0,98 bis 1,04 für das Verhältnis von Säurederivat der allgemeinen Formel III zu Hydrazinoethanol/thiol II.

Vorteilhaft arbeitet man im ersten Reaktionsschritt unter neutralen Bedingungen. Sofern bei der Reaktion ein saures Reaktionsprodukt entsteht, z.B. Halogenwasserstoff, wenn G in Formel III für Halogen steht, entfernt man diesen durch Zugabe basischer Verbindungen, z. B. Alkali- oder Erdalkalihydroxide bzw. -hydrogencarbonate oder -carbonate. Man kann die Reaktion jedoch auch in Gegenwart einer organischen Base, z. B. Triethylamin, Tri-n-propylamin, N-Ethyldiisopropylamin, Pyridin, α-, β-, γ-Picolin, 2,4-, 2,6-Lutidin, N-Methylpyrrolidin, Dimethylanilin, N,N-Dimethylcyclohexylamin, Chinolin oder Acridin durchführen.

Schließlich kann man die Reaktion auch in einem wäßrigen Zweiphasensystem durchführen, vorzugsweise in Gegenwart von Phasentransferkatalysatoren wie quartären Ammonium- oder Phosphoniumsalzen.
Für die Zweiphasen-Reaktion sind die in der EP-A 556737 beschriebenen Reaktionsbedingungen geeignet.

Als Phasentransferkatalysatoren können quartäre Ammonium- oder Phosphoniumsalze verwendet werden. An geeigneten Verbindungen seien folgende genannt: Tetraalkyl-(C₁-C₁₈)-ammoniumchloride, -bromide oder fluoride, N-Benzyltrialkyl-(C₁-C₁₈)-ammoniumchloride, -bromide oder -fluoride, Tetraalkyl-(C₁-C₁₈)-phosphoniumchloride oder -bromide, Tetraphenylphosphoniumchlorid oder -bromid, (Phenyl)ₒ(alkyl-(C₁-C₁₈)ₚ-phosphoniumchloride oder -bromide, wobei o = 1 bis 3, p = 3 bis 1 und o + p = 4 ist. Besonders bevorzugt sind Tetraethylammoniumchlorid und N-Benzyltriethylammoniumchlorid. Die Menge an Phasentransferkatalysator beträgt im Allgemeinen bis zu 20 Gew.-%, bevorzugt zwischen 1 und 15 Gew.-% und besonders bevorzugt zwischen 2 bis 8 Gew.-%, bezogen auf das Hydrazinoethanol/thiol II.

Vorteilhaft gibt man das Säurederivat III während 0,25 bis 2 Stunden zu einer Mischung des Hydrazinoethanols/thiols II und der Base in einem der vorgenannten Lösungsmittel bei 0 bis 60°C und rührt zur Vervollständigung der Reaktion noch 0,5 bis 16 Stunden, vorzugsweise 2 bis 8 Stunden bis 0 bis 60°C nach.

Bei Verwendung eines wäßrigen Zweiphasensystems kann man in beliebiger Reihenfolge die Ausgangsstoffe II und III zu einer Mischung des Phasentransferkatalysators in den beiden Phasen unter Rühren zugeben und dann im genannten Temperaturbereich unter Zugabe von Base die Umsetzung zu Ende bringen.

Handelt es sich bei der Verbindung der Formel III um ein Isocyanat, Isothiocyanat oder ein Keten kann auf die Zugabe einer Base verzichtet werden. Die Umsetzung erfolgt dann vorzugsweise unter wasserfreien Bedingungen.

Die Reaktion kann drucklos oder unter Druck kontinuierlich oder diskontinuierlich durchgeführt werden.

Zur Aufarbeitung trennt man von den gegebenenfalls ausgefallenen Salzen ab, oder vervollständigt deren Abscheidung durch Zugabe unpolarer Lösungsmittel und reichert so die Hydrazide IV in dem Filtrat an.

Im folgenden wird der zweite Reaktionsschritt erläutert: Vorteilhaft setzt man dann die Hydrazide IV unter sauren Bedingungen mit einer Formaldehydlösung oder Paraformaldehyd in einem der vorgenannten Lösungsmittel um.

Für den Folgeschritt werden zweckmäßig 0,9 bis 1,2, vorzugsweise 0,95 bis 1,1, besonders bevorzugt 0,98 bis 1.04 Moläquivalente Formaldehyd oder Paraformaldehyd pro Mol Hydrazidderivat IV eingesetzt. Die Konzentration der Edukte im Lösungsmittel beträgt 0,1 bis 5 Mol/l, bevorzugt 0,2 bis 2 Mol/l.

Als Säure kann man aromatische Sulfonsäuren, z.B. Benzolsulfonsäure, p-Chlor- oder p-Toluolsulfonsäure, aliphatische Sulfonsäuren wie Methansulfonsäure, Trifluormethansulfonsäure, Ethansulfonsäure und n-Propylsulfonsäure, Sulfaminsäuren wie Methylsulfaminsäure, Ethylsulfaminsäure oder Isopropylsulfaminsäure, aliphatische Carbonsäure wie Essigsäure, Trifluoressigsäure, Propionsäure, Buttersäure oder Isobuttersäure sowie anorganische Säuren wie Chlorwasserstoffsäure, Schwefelsäure, Salpetersäure oder Borsäure verwenden. Zweckmäßig kann man eine Säure wie Essigsäure oder Propionsäure direkt auch als Reaktionsmedium verwenden. Den sauren Katalysator setzt man zweckmäßig in einer Menge von 1 bis 20 Mol.-%, bevorzugt 3 bis 15 Mol.-%, besonders bevorzugt 5 bis 10 Mol.-% Säure pro Mol Hydrazid IV ein.

Vorzugsweise gibt man eine Formaldehydlösung oder Paraformaldehyd während 2 bis 60 min zu einer Mischung von Hydrazid IV und dem sauren Katalysator in einem der vorgenannten Lösungsmittel bei 0 bis 100°C, vorteilhaft 10 bis 80°C, besonders bevorzugt 20 bis 50°C und rührt zur Vervollständigung der Reaktion noch 10 bis 50 Stunden, vorzugsweise 15 bis 30 Stunden bei 40 bis 50°C nach.

Setzt man eine wäßrige Formalinlösung ein, entfernt man zweckmäßig das Wasser, z.B. am Wasserabscheider.

Man kann jedoch auch den sauren Katalysator zu einer Mischung von Hydrazid IV und Paraformaldehyd in einem der vorgenannten Lösungsmittel geben und dann wie beschrieben die Reaktion zu Ende führen.

Die Reaktion kann drucklos oder unter Druck, kontinuierlich oder diskontinuierlich betrieben werden.

Die sich gegebenenfalls anschließende Oxidation der Perhydrodiazine I mit Z = S zu den Sulfoxiden wird bevorzugt mit Wasserstoffsuperoxid durchgeführt, wobei mit etwa äquivalenten Mengen an Oxidationsmittel die Sulfoxide und mit etwa doppelt molaren Mengen die Sulfone erhalten werden.

Die Oxidation mit Wasserstoffperoxid kann durch geeignete Metallverbindungen katalysiert werden, z.B. Übergangsmetalloxide wie Vanadinpentoxid, Natriumwolframat, Kaliumdichromat, Eisenoxidwolframat, Natriumwolframat-Molybdänsäure, Osmiumsäure, Titantrichlorid, Selendioxid, Phenylenselensäure, Oxovanadinyl-2,4-pentandionat. Die Katalysatoren werden im allgemeinen in einer Menge von 0,5 bis 10% eingesetzt, wegen der leichten Filtrierbarkeit und Wiedergewinnung der anorganischen Katalysatoren können jedoch auch stöchiometrische Mengen eingesetzt werden.

Als Lösungsmittel für die Oxidation mit Wasserstoffperoxid können beispielsweise Wasser, Acetonitril, Alkohole wie Methanol, Ethanol, Isopropanol, tert.-Butanol, chlorierte Kohlenwasserstoffe wie Methylenchlorid, 1,1,2,2-Tetrachlorethan oder Ketone wie Aceton oder Methylethylketon verwendet werden.

Neben Wasserstoffperoxid können als Oxidationsmittel auch Persäuren, wie Perbenzoesäure, Monoperphthalsäure oder 3-Chlorperbenzoesäure eingesetzt werden. Die Umsetzung mit Persäuren erfolgt zweckmäßig in chlorierten Kohlenwasserstoffen wie Methylenchlorid oder 1,2-Dichlorethan.

Sehr geeignet zur Oxidation der Thiole zu Sulfoxiden oder Sulfonen sind ferner Chlor und Brom. Diese Oxidation wird zweckmäßigerweise in polaren Lösungsmitteln wie Wasser, Acetonitril, Dioxan, oder in Zweiphasensystemen wie wäßriger Kaliumhydrogencarbonatlösung/Dichlormethan ferner auch Essigsäure durchgeführt. Als Quelle für aktives Halogen können ferner tert.-Butylhypochlorit, unterchlorige sowie unterbromige Säure, deren Salze, ferner N-Halogenverbindungen wie N-Brom- und N-Chlorsuccinimid oder auch Sulfurylchlorid eingesetzt werden.

Geeignet für die Oxidation ist auch die photosensibilisierte Sauerstoffübertragung, wobei üblicherweise als Photosensibilisatoren organische Farbstoffe, z.B. Porphyrine wie Tetraphenylporphyrin, Chlorophyll, Protoporphyrin, Xanthenfarbstoffe, wie Bengalrosa oder Phenothiazin-Farbstoffe wie Methylenblau eingesetzt werden.

Als inerte Lösungsmittel sind Kohlenwasserstoffe wie Pentan, Hexan, Heptan, Cyclohexan, chlorierte Kohlenwasserstoffe wie Methylenchlorid, 1,2-Dichlorethan, 1,1,2,2-Tetrachlorethan, Alkohole wie Methanol, Ethanol, n-Propanol oder Isopropanol, Ketone wie Aceton, Methylethylketon, polare aprotische Lösungsmittel wie Acetonitril, Propionitril oder aromatische Kohlenwasserstoffe wie Benzol, Toluol, Chlorbenzol oder Xylol geeignet. An Stelle von Sauerstoff kann man auch Ozon verwenden in den obengenannten Lösungsmitteln, zusätzlich noch Ether, 1,4-Dioxan oder THF.

Neben der Photosensibilisierung eignen sich für die Sauerstoffoxidation auch Katalysatoren z. b. Oxide und Sulfide vom Nickel, Kupfer, Aluminium, Wolfram, Chrom, Vanadium, Ruthenium, Titan, Mangan, Molybdän, Magnesium und Eisen.

Je nach Stöchiometrie der verwendeten Oxidationsmittel gelangt man entweder zu den Sulfoxiden I B oder deren Sulfonen I C. Die molaren Verhältnisse, in denen die Ausgangsverbindungen miteinander umgesetzt werden, betragen im Allgemeinen 0,9 bis 1,8, vorzugsweise 1,05 bis 1,3 für das Verhältnis von Thiadiazin I A zu Oxidationsmittel im Falle der Oxidation zum Sulfoxid und im allgemeinen 1,9 bis 3,5, vorzugsweise 2,05 bis 2,9 im Falle der Oxidation zum Sulfon.

Die Konzentration der Edukte im Lösungsmittel beträgt im Allgemeinen 0,1 bis 5 Mol/l, bevorzugt 0,2 bis 2 Mol/l.

Vorteilhaft legt man das 1-Thiadiazin der Formel I mit Z = S oder das Sulfoxid ggf. mit einem der vorgenannten Katalysatoren in einem der vorgenannten Lösungsmittel vor und gibt dann das Oxidationsmittel während 0,25 bis 20 Stunden unter Rühren hinzu. Die Zugabe und Reaktionstemperatur richtet sich nach der optimalen Effizienz der jeweiligen Oxidationsmittel und der Vermeidung von Nebenreaktionen. Im Falle der Verwendung von photosensibilisiertem Sauerstoff arbeitet man im allgemeinen bei -20 bis 80°C, metallkatalysiert jedoch im allgemeinen bei 50 bis 140°C und bei Verwendung von Ozon im allgemeinen bei -78 bis 60°C. Wegen der begrenzten Löslichkeit der Sauerstoffderivate werden diese vorzugsweise über einen längeren Zeitraum (bis zu 20 h) kontinuierlich in das Reaktionsgemisch eingeführt, bis die Oxidation auf der Sulfoxid- oder Sulfon-Stufe abgeschlossen ist. Flüssige oder leicht lösliche Oxidationsmittel wie Wasserstoffsuperoxid, unterchlorige oder unterbromige Säure, tert.-Butylhypochlorit, Chlor oder Brom, ferner N-Chlor-, bzw. N-Bromsuccinimid können je nach exothermen Charakter der Reaktion in kürzeren Zeitspannen während 0,25 bis 6 h zu der Reaktionsmischung des Thiadiazins oder -sulfoxids zugegeben werden, um die Reaktion nach weiteren 1 bis 60 h zum Abschluß zu bringen. Bevorzugt ist ferner eine gestaffelte Zugabe des flüssigen oder gelösten Oxidationsmittels. Im Falle von Wasserstoffsuperoxid arbeitet man im allgemeinen bei 0 bis 90°C, mit tert.-Butylpypochlorit im allgemeinen bei -78 bis 30°C und mit N-Halogenverbindungen im allgemeinen bei 0 bis 30°C. Im Falle von Chlor oder Brom ist eine Reaktionstemperatur von 0 bis 40°C zu empfehlen.

Die Oxidationen können drucklos, unter Druck, kontinuierlich oder diskontinuierlich betrieben werden.

Vorteilhaft kann man die mehrstufige Reaktion auch als Eintopfverfahren durchführen, wobei man die Thiadiazine I (Z = S) ohne Isolierung und Reinigung direkt zu den Sulfoxiden I (Z = SO) oder den Sulfonen I (Z = SO₂) umsetzt. Dementsprechend läßt man das Umsetzungsprodukt Ia gegebenenfalls auf 90 bis 20°C abkühlen, gibt gegebenenfalls ein Lösungsmittel, z.B. Methylenchlorid und/oder Wasser hinzu und fügt nun das Oxidationsmittel nach Maßgabe seines Verbrauches hinzu. Als Oxidationsmittel sind Wasserstoffperoxid oder Natriumhypochlorit besonders bevorzugt.

Zur Aufarbeitung der Oxidationsmischung nimmt man im Allgemeinen die Endstoffe I in einem mit Wasser nicht mischbaren Lösungsmittel auf, extrahiert saure Verunreinigungen bzw. Oxidationsmittel mit verdünntem Alkali- bzw. Wasser, trocknet und entfernt das Lösungsmittel unter reduziertem Druck.

Grundsätzlich sind die substituierten Perhydrodiazine I nach dem vorstehend genannten erfindungsgemäßen Syntheseverfahren herstellbar. Aus wirtschaftlichen oder verfahrenstechnischen Gründen kann es jedoch zweckmäßiger sein, einige Verbindungen I aus ähnlichen substituierten Perhydrodiazinen, die sich jedoch in der Bedeutung eines Restes unterscheiden, herzustellen.

Die Aufarbeitung der Reaktionsgemische erfolgt in der Regel nach an sich bekannten Methoden, beispielsweise durch Verdünnen der Reaktionslösung mit Wasser und anschließender Isolierung des Produktes mittels Filtration, Kristallisation oder Lösungsmittelextraktion, oder durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die erfindungsgemäßen Verbindungen der allgemeinen Formel I sind in guten Ausbeuten zugänglich. Sie lassen sich auch in größerem Maßstab herstellen. Sie sind daher in besonderem Maße als Ausgangsprodukte für die Herstellung von Verbindungen mit 1-Hetero-3,4-diazinstruktur der allgemeinen Formel, 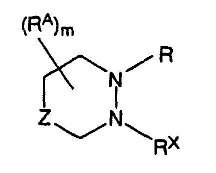 worin R, Z, R^{A} und m die zuvor genannten Bedeutungen aufweisen und R^{X} für einen beliebigen organischen Rest steht, geeignet.

Es wurde nun gefunden, dass Verbindungen der allgemeinen Formel V 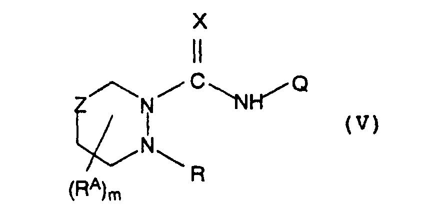 worin R, R^{A}, Z und m die in den Ansprüchen 1 bis 4 genannten Bedeutungen aufweisen,
- X: für O oder S steht, und
- Q: einen C₆-C₁₄-aromatischen Rest bedeutet, der wenigstens einfach substituiert ist und/oder über zwei benachbarte Kohlenstoffatome mit einen gesättigten oder ungesättigten 4- bis 7-gliedrigen Cyclus verbunden ist, der neben den Kohlenstoffringgliedern gewünschtenfalls eines, zwei oder drei der folgenden Glieder enthalten kann: -O-, -S-, -S(O)₂- -N=, -NH- oder -N(C₁-C₆-Alkyl)-;
eine besonders gute herbizide Wirkung haben. Derartige Verbindungen sind neu und Gegenstand einer weiteren Patentanmeldung.

Es wurde gefunden, dass die Verbindungen der allgemeinen Formel V ausgehend von den Verbindungen der allgemeinen Formel I durch Umsetzung mit Isocyanaten oder Isothiocyanaten der allgemeinen Formel VI,

Q-N=C=X (VI)

worin Q und X die zuvor genannten Bedeutungen haben, herstellbar sind.

Demnach betrifft die vorliegende Erfindung auch ein Verfahren zur Herstellung von Verbindungen der allgemeinen Formel V (substituierte Harnstoffe), das dadurch gekennzeichnet ist, dass man die vorstehend definierten Verbindungen der allgemeinen Formel I mit einem Isocyanat oder Isothiocyanat der allgemeinen Formel VI umsetzt.

Q steht im Hinblick auf die herbizide Wirkung der substituierten Harnstoffe der allgemeinen Formel V beispielsweise für einen Rest Q1 bis Q6: 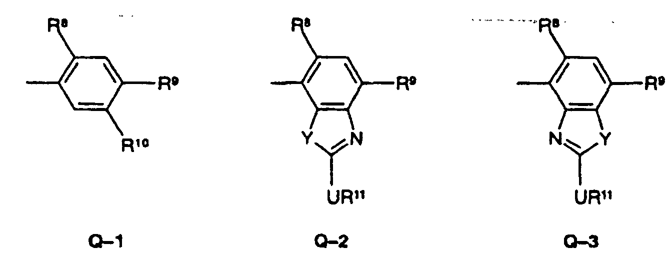 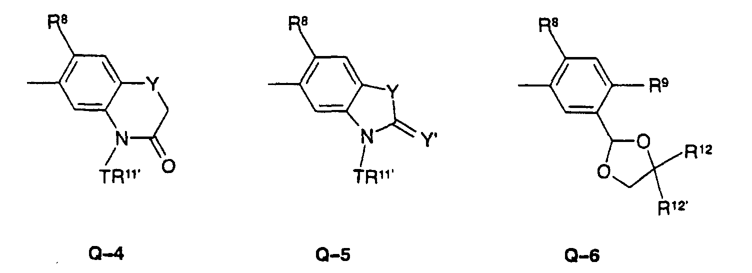 worin
- Y und Y': unabhängig voneinander für O oder S;
- T: für eine chemische Bindung oder O;
- U: für eine chemische Bindung, C₁-C₄-Alkylen, O, S, SO oder SO₂;
stehen und die Reste R⁸ bis R^{12'} die folgenden Bedeutungen haben:
- R⁸: Wasserstoff oder Halogen;
- R⁹: C₁-C₆-Akyl, C₁-C₆-Haloalkyl, OCH₃, SCH₃, OCHF₂, Halogen, Cyano oder NO₂;
- R¹⁰: Wasserstoff, Hydroxy, Mercapto, Cyano, Nitro, Halogen, C₁-C₆-Alkyl, C₂-C₆-Alkenyl, C₂-C₆-Alkinyl, C₁-C₆-Halogenalkyl, C₁-C₆-Halogenalkoxy, C₁-C₆-Halogenalkylthio, C₁-C₆-Alkoxy-(C₁-C₆-alkyl)carbonyl, C₁-C₆-Alkylthio-(C₁-C₆-alkyl)carbonyl, (C₁-C₆-Alkyl)-iminooxycarbonyl, C₁-C₆-Alkoxy-C₁-C₆-alkyl, C₁-C₆-Alkoxyamino-C₁-C₆-alkyl, C₁-C₆-Alkoxy-C₁-C₆-alkylamino-C₁-C₆-alkyl,
C₁-C₆-Alkoxy, C₁-C₆-Alkylthio, C₃-C₆-Cycloalkoxy, C₃-C₆-Cycloalkylthio, C₂-C₆-Alkenyloxy, C₂-C₆-Alkenylthio, C₂-C₆-Alkinyloxy, C₂-C₆-Alkinylthio, (C₁-C₆-Alkyl)carbonyloxy, (C₁-C₆-Alkyl)carbonylthio, (C₁-C₆-Alkoxy)carbonyloxy, (C₂-C₆-Alkenyl)carbonyloxy, (C₂-C₆-Alkenyl)carbonylthio, (C₂-C₆-Alkinyl)carbonyloxy, (C₂-C₆-Alkinyl)carbonylthio, C₁-C₆-Alkylsulfonyloxy oder C₁-C₆-Alkylsulfonyl, wobei jeder der zuletzt genannten 17 Reste gewünschtenfalls einen, zwei oder drei Substituenten tragen kann, die ausgewählt sind unter:
- Halogen, Nitro, Cyano, Hydroxy, C₃-C₆-Cycloalkyl, C₁-C₆-Alkoxy, C₃-C₆-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy, Oxo, =N-OR¹³
- Phenyl, Phenoxy oder Phenylsulfonyl, wobei die drei letztgenannten Gruppen gegebenenfalls einen, zwei oder drei Substituenten tragen können, ausgewählt unter Halogen, Nitro, Cyano, C₁-C₆-Akyl, C₁-C₆-Halogenalkyl, C₁-C₆-Alkoxy und (C₁-C₆-Alkoxy)carbonyl;
- -CO-R¹⁴, -CO-OR¹⁴, -CO-SR¹⁴, -CO-N(R¹⁴)-R¹⁵, -OCO-R¹⁴, -OCO-OR^{14'}, -OCO-SR^{14'}, -OCO-N(R¹⁴)-R¹⁵, -N(R¹⁴)-R¹⁵, und -C(R¹⁶)=N-OR¹³;
C(Z¹)-R¹⁷, -C(=NR¹⁸)R¹⁷, C(R¹⁷)(Z²R¹⁹)(Z³R²⁰), C(R¹⁷)=C(R²¹)-CN, C(R¹⁷)=C(R²¹)-CO-R²², -CH(R¹⁷)-CH(R²¹)-COR²², -C(R¹⁷)=C(R²¹)-CH₂-CO-R²², -C(R¹⁷)=C(R²¹)-C(R²³)=C(R²⁴)-CO-R²², -C(R¹⁷)=C(R²¹)-CH₂-CH(R²⁵)-CO-R²², -CO-OR²⁶, -CO-SR²⁶, -CON(R²⁶)-OR¹³, -C≡C-CO-NHOR¹³, -C≡C-CO-N(R²⁶)-OR¹³, -C≡C-CS-NH-OR¹³, -C≡C-CS-N(R²⁶)-OR¹³, -C(R¹⁷)=C(R²¹)-CO-NHOR¹³, -C(R¹⁷)=C(R²¹)-CO-N(R²⁶)-OR¹³, -C(R¹⁷)=C(R²¹)-CS-NHOR¹³, -C(R¹⁷)=C(R²¹)-CS-N(R²⁶)-OR¹³, -C(R¹⁷)=C(R²¹)-C(R¹⁶)=N-OR¹³, C(R¹⁶)=N-OR¹³, -C≡C-C(R¹⁶)=NOR¹³, C(Z²R¹⁹)(Z³R²⁰)-OR²⁶, -C(Z²R¹⁹)(Z²R²⁰)SR²⁶, C(Z²R¹⁹)(Z³R²⁰)-N(R²⁷)R²⁸, -N(R²⁷)-R²⁸, -CO-N(R²⁷)-R²⁸ oder -C(R¹⁷)=C(R²¹)CO-N(R²⁷)R²⁸; wobei Z¹, Z², Z³ unabhängig voneinander für Sauerstoff oder Schwefel stehen;
- R¹¹: Wasserstoff, C₁-C₆-Alkyl, C₁-C₆-Haloalkyl, C₁-C₃-Alkoxy-C₁-C₃-alkyl, C₃-C₆-Alkenyl, C₁-C₃-Alkoxy-C₃-C₆-alkenyl, C₃-C₆-Haloalkenyl, C₃-C₆-Alkinyl, C₁-C₃-Alkoxy-C₃-C₆-alkinyl, C₃-C₆-Haloalkinyl, C₃-C₇-Cycloalkyl, 3- bis 7-gliedriges gesättigtes Heterocyclyl, wobei jeder Cycloalkyl- und jeder Heterocyclyl-Ring ein Carbonyl- oder Thiocarbonyl-Ringlied enthalten kann und wobei jeder Cycloalkyl- und Heterocyclyl-Ring unsubstituiert sein oder ein zwei, drei oder vier Substituenten tragen kann, ausgewählt unter Cyano, Nitro, Amino, Hydroxy, Halogen, C₁-C₄-Alkyl, C₁-C₄-Halogenalkyl, C₁-C₄-Cyanoalkyl, C₁-C₄-Hydroxyalkyl, C₁-C₄-Aminoalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfonyl, (C₁-C₄-Alkoxy)carbonyl, (C₁-C₄-Alkyl)carbonyl, (C₁-C₄-Halogenalkyl)carbonyl, (C₁-C₄-Alkyl)carbonyloxy, (C₁-C₄-Halogenalkyl)carbonyloxy, Di(C₁-C₄-alkyl)amino, C₃-C₆-Alkenyl, C₃-C₆-Alkinyl, C₃-C₄-Alkenyloxy, C₃-C₄-Alkenylthio, C₃-C₄-Alkinyloxy und C₃-C₄-Alkinylthio;
- R^{11'}: Wasserstoff, C₁-C₆-Alkyl, C₁-C₆-Haloalkyl, C₁-C₃-Alkoxy-C₁-C₃-alkyl, C₂-C₆-Cyanoalkyl, C₃-C₆-Alkenyl, C₃-C₆-Alkinyl;
- R¹²: Wasserstoff, C₁-C₃-Alkyl, C₁-C₃-Haloalkyl oder Halogen;
- R^{12'}: Wasserstoff, C₁-C₃-Alkyl, C₁-C₃-Haloalkyl; oder
- R¹²: und R^{12'} gemeinsam C=O;

Die bei R¹⁰ aufgeführten Variablen R¹³ bis R³⁰ haben beispielsweise die folgenden Bedeutungen:
- R¹³: Wasserstoff, C₁-C₆-Alkyl, C₁-C₆-Halogenalkyl, C₃-C₆-Cycloalkyl, C₃-C₆-Alkenyl, C₃-C₆-Alkinyl, Hydroxy-C₁-C₆-alkyl, C₁-C₆-Alkoxy-C₁-C₆-alkyl, C₁-C₆-Alkylthio-C₁-C₆-alkyl, Cyano-C₁-C₆-alkyl, (C₁-C₆-Alkyl)carbonyl-C₁-C₆-alkyl, (C₁-C₆-Alkoxy)carbonyl-C₁-C₆-alkyl, (C₁-C₆-Alkoxy)-carbonyl-C₂-C₆-alkenyl, (C₁-C₆-Alkyl)carbonyloxy-C₁-C₆-alkyl oder Phenyl-C₁-C₆-alkyl, worin der Phenylring gewünschtenfalls ein zwei oder drei Substituenten tragen kann, ausgewählt unter Cyano, Nitro, Halogen, C₁-C₆-Alkyl, C₁-C₆-Halogenalkyl, C₁-C₆-Alkoxy und (C₁-C₆-Alkoxy)carbonyl;
- R¹⁴: Wasserstoff, C₁-C₆-Alkyl, C₃-C₆-Cycloalkyl, C₃-C₆-Alkenyl, C₃-C₆-Alkinyl, C₁-C₆-Alkoxy-C₁-C₆-alkyl, (C₁-C₆-Alkoxy)-carbonyl-C₁-C₆-alkyl, (C₃-C₆-Alkenyloxy)carbonyl-C₁-C₆-alkyl, Phenyl oder Phenyl-C₁-C₆-alkyl, wobei der Phenyl-Ring der zwei zuletzt genannten Gruppen unsubstituiert sein oder einen zwei oder drei Reste tragen kann, ausgewählt unter Halogen, Nitro, Cyano, C₁-C₆-Alkyl, C₁-C₆-Halogenalkyl, C₁-C₆-Alkoxy und (C₁-C₆-Alkyl)carbonyl;
- R^{14'}: die für R¹⁴ genannten Bedeutungen, ausgenommen Wasserstoff;
- R¹⁵: Wasserstoff, Hydroxy, C₁-C₆-Alkyl, C₃-C₆-Cycloalkyl, C₁-C₆-Alkoxy, (C₁-C₆-Alkoxy)carbonyl-C₁-C₆-alkoxy, C₃-C₆-Alkenyl oder C₃-C₆-Alkenyloxy;
- R¹⁶: Wasserstoff, Halogen, C₁-C₆-Alkyl, C₁-C₆-Halogenalkyl, C₁-C₆-Alkoxy, C₁-C₆-Halogenalkoxy, C₃-C₆-Alkenyloxy, C₃-C₆-Alkinyloxy, C₁-C₆-Alkylthio, C₁-C₆-Halogenalkylthio, (C₁-C₆-Alkyl)carbonyloxy, (C₁-C₆-Halogenalkyl)carbonyloxy, C₁-C₆-Alkylsulfonyloxy oder C₁-C₆-Halogenalkylsulfonyloxy, wobei die letztgenannten 12 Reste einen der folgenden Substituenten tragen können: Hydroxy, Cyano, Hydroxycarbonyl, C₁-C₆-Alkoxy, C₁-C₆-Alkylthio, (C₁-C₆-Alkyl)carbonyl, (C₁-C₆-Alkoxy)carbonyl, (C₁-C₆-Alkyl)aminocarbonyl, Di(C₁-C₆-alkyl)aminocarbonyl, (C₁-C₆-Alkyl)carbonyloxy, C₁-C₆-Alkoxy-(C₁-C₆-alkyl)aminocarbonyl;
(C₁-C₆-Alkyl)carbonyl, (C₁-C₆-Halogenalkyl)carbonyl, (C₁-C₆-Alkoxy)carbonyl, (C₁-C₆-Alkoxy)carbonyloxy, (C₁-C₆-Alkyl)carbonylthio, (C₁-C₆-Halogenalkyl)carbonylthio, (C₁-C₆-Alkoxy)carbonylthio, C₂-C₆-Alkenyl, (C₂-C₆-Alkenyl)carbonyloxy, C₂-C₆-Alkenylthio, C₃-C₆-Alkinyl, C₃-C₆-Alkinyloxy, C₃-C₆-Alkinylthio, (C₂-C₆-Alkinyl)carbonyloxy, C₃-C₆-Alkinylsulfonyoxy, C₃-C₆-Cycloalkyl, C₃-C₆-Cycloalkyloxy, C₃-C₆-Cycloalkylthio, (C₃-C₆-Cycloalkyl)carbonyloxy, C₃-C₆-Cycloalkylsulfonyloxy;
Phenyl, Phenoxy, Phenylthio, Benzoyloxy, Phenylsulfonyloxy, Phenyl-C₁-C₆-alkyl, Phenyl-C₁-C₆-alkoxy, Phenyl-C₁-C₆-alkylthio, Phenyl-(C₁-C₆-alkyl)-carbonyloxy oder Phenyl-(C₁-C₆-alkyl)sulfonyloxy, wobei die Phenylringe der letzgenannten 10 Reste unsubstituiert sein oder ihrerseits ein bis drei Stubstituenten tragen können, jeweils ausgewählt aus der Gruppe bestehen aus Cyano, Nitro, Halogen, C₁-C₆-Alkyl, C₁-C₆-Halogenalkyl, C₁-C₆-Alkoxy und (C₁-C₆-Alkoxy)carbonyl;
- R¹⁷: Wasserstoff, Cyano, C₁-C₆-Alkyl, C₁-C₆-Halogenalkyl, C₂-C₆-Alkenyl, C₂-C₆-Alkinyl, C₃-C₆-Cycloalkyl, C₁-C₆-Alkoxy-C₁-C₆-alkyl oder (C₁-C₆-Alkoxy)carbonyl;
- R¹⁸: Wasserstoff, Hydroxy, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkoxy-C₁-C₆-alkyl, C₁-C₆-Alkoxy, C₃-C₆-Alkenyloxy, C₃-C₆-Alkinyloxy, C₃-C₆-Cycloalkoxy, C₅-C₇-Cycloalkenyloxy, C₁-C₆-Halogenalkoxy, C₃-C₆-Halogenalkenyloxy, Hydroxy-C₁-C₆-alkoxy, Cyano-C₁-C₆-alkoxy, C₃-C₆-Cycloalkyl-C₁-C₆-alkoxy, C₁-C₆-Alkoxy-C₁-C₆-alkoxy, C₁-C₆-Alkoxy-C₃-C₆-alkenyloxy, (C₁-C₆-Alkyl)carbonyloxy, (C₁-C₆-Halogenalkyl)carbonyloxy, (C₁-C₆-Alkyl)carbamoyloxy, (C₁-C₆-Halogenalkyl)carbamoyloxy, (C₁-C₆-Alkyl)carbonyl-C₁-C₆-alkyl, (C₁-C₆-Alkyl)carbonyl-C₁-C₆-alkoxy, (C₁-C₆-Alkoxy)carbonyl-C₁-C₆-alkyl, (C₁-C₆-Alkoxy)carbonyl-C₁-C₆-alkoxy, C₁-C₆-Alkylthio-C₁-C₆-alkoxy, Di(C₁-C₆-alkyl)amino-C₁-C₆-alkoxy, -N(R²⁹)R³⁰, Phenyl, das seinerseits noch einen zwei oder drei Substituenten tragen kann, jeweils ausgewählt unter Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl;
Phenyl-C₁-C₆-alkoxy, Phenyl-C₁-C₆-alkyl, Phenyl-C₃-C₆-alkenyloxy oder Phenyl-C₃-C₆-alkinyloxy, wobei jeweils eine oder zwei Methylengruppen der Kohlenstoffketten in den vier zuletzt genannten Gruppen durch -O-, -S-, oder -N(C₁-C₆-Alkyl)- ersetzt sein können und wobei Phenylringe in den vier zuletzt genannten Gruppen unsubstituiert oder ihrerseits einen bis drei Substituenten tragen können, ausgewählt unter Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl;
C₃-C₇-Heterocyclyl, C₃-C₇-Heterocyclyl-C₁-C₆-alkyl, C₃-C₇-Heterocyclyl-C₁-C₆-alkoxy, C₃-C₇-Heterocyclyl-C₃-C₆-alkenyloxy oder C₃-C₇-Heterocyclyl-C₃-C₆-alkinyloxy, wobei jeweils eine oder zwei Methylengruppen der Kohlenstoffketten in den vier zuletzt genannten Gruppen durch -O-, -S- oder -N(C₁-C₆-Alkyl)- ersetzt sein können und wobei jeder Heterocyclus gesättigt, ungesättigt oder aromatisch sein kann und entweder unsubstituiert ist oder seinerseits einen bis drei Substituenten trägt, ausgewählt unter Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl;
- R¹⁹,: R²⁰ unabhängig voneinander C₁-C₆-Alkyl, C₁-C₆-Halogenalkyl, C₃-C₆-Alkenyl, C₃-C₆-Alkinyl, C₁-C₆-Alkoxy-C₁-C₆-alkyl bedeuten oder zusammen für eine gesättigte oder ungesättigte, 2- bis 4-gliedrige Kohlenstoffkette stehen, die einen Oxosubstituenten tragen kann, wobei ein den Variablen Z² und Z³ nicht benachbartes Glied dieser Kette durch -O-, -S-, -N=, -NH- oder -N(C₁-C₆-Alkyl)- ersetzt sein kann, und wobei die Kohlenstoffkette noch ein bis drei Reste tragen kann, ausgewählt unter Cyano, Nitro, Amino, Halogen, C₁-C₆-Alkyl, C₂-C₆-Alkenyl, C₁-C₆-Alkoxy, C₂-C₆-Alkenyloxy, C₂-C₆-Alkinyloxy, C₁-C₆-Halogenalkyl, Cyano-C₁-C₆-alkyl, Hydroxy-C₁-C₆-alkyl, C₁-C₆-Alkoxy-C₁-C₆-alkyl, C₃-C₆-Alkenyloxy-C₁-C₆-alkyl, C₃-C₆-Alkinyloxy-C₁-C₆-alkyl, C₃-C₆-Cycloalkyl, C₃-C₆-Cycloalkoxy, Carboxy, (C₁-C₆-Alkoxy)carbonyl, (C₁-C₆-Alkyl)carbonyloxy-C₁-C₆-alkyl und Phenyl; gegebenenfalls substituiertes Phenyl, wobei die Kohlenstoffkette auch durch einen ankondensierten oder spiroverknüpften 3- bis 7-gliedrigen Ring substituiert sein kann, der ein oder zwei Heteroatome als Ringglieder enthalten kann, ausgewählt unter Sauerstoff, Schwefel, Stickstoff und durch C₁-C₆-Alkyl substituiertem Stickstoff, und der gewünschtenfalls seinerseits einen oder zwei der folgenden Substituenten tragen kann: Cyano, C₁-C₆-Alkyl, C₂-C₆-Alkenyl, C₁-C₆-Alkoxy, Cyano-C₁-C₆-alkyl, C₁-C₆-Halogenalkyl und (C₁-C₆-Alkoxy)carbonyl;
- R²¹: Wasserstoff, Cyano, Halogen, C₁-C₆-Alkyl, C₁-C₆-Halogenalkyl, C₁-C₆-Alkoxy, (C₁-C₆-Alkyl)carbonyl oder (C₁-C₆-Alkoxy)carbonyl;
- R²²: Wasserstoff, O-R³¹, S-R³¹, C₁-C₆-Alkyl, das noch einen oder zwei C₁-C₆-Alkoxysubstituenten tragen kann, C₂-C₆-Alkenyl, C₂-C₆-Alkinyl, C₁-C₆-Halogenalkyl, C₃-C₆-Cycloalkyl, C₁-C₆-Alkylthio-C₁-C₆-alkyl, C₁-C₆-Alkyliminooxy, -N(R²⁷)R²⁸ oder Phenyl, das unsubstituiert sein oder ein bis drei Substituenten tragen kann, jeweils ausgewählt aus der Gruppe bestehend aus Cyano, Nitro, Halogen, C₁-C₆-Alkyl, C₂-C₆-Alkenyl, C₁-C₆-Halogenalkyl, C₁-C₆-Alkoxy und (C₁-C₆-Alkoxy)carbonyl;
- R²³: Wasserstoff, Cyano, Halogen, C₁-C₆-Alkyl, C₃-C₆-Alkenyl, C₃-C₆-Alkinyl, C₁-C₆-Alkoxy-C₁-C₆-alkyl, (C₁-C₆-Alkyl)carbonyl, (C₁-C₆-Alkoxy)carbonyl, -N(R²⁷)R²⁸ oder Phenyl, das seinerseits noch einen bis drei Substituenten tragen kann, ausgewählt unter Cyano, Nitro, Halogen, C₁-C₆-Alkyl, C₁-C₆-Halogenalkyl, C₃-C₆-Alkenyl, C₁-C₆-Alkoxy und (C₁-C₆-Alkoxy)carbonyl;
- R²⁴: Wasserstoff, Cyano, Halogen, C₁-C₆-Alkyl, C₁-C₆-Alkoxy, C₁-C₆-Halogenalkyl, (C₁-C₆-Alkyl)carbonyl oder (C₁-C₆-Alkoxy)carbonyl;
- R²⁵: Wasserstoff, Cyano, C₁-C₆-Alkyl oder (C₁-C₆-Alkoxy)carbonyl;
- R²⁶, R³¹: unabhängig voneinander Wasserstoff, C₁-C₆-Alkyl, C₁-C₆-Halogenalkyl, C₂-C₆-Alkenyl oder C₂-C₆-Alkinyl, wobei die letztgenannten 4 Gruppen jeweils einen oder zwei der folgenden Reste tragen können: Cyano, Halogen, Hydroxy, Hydroxycarbonyl, C₁-C₆-Alkoxy, C₁-C₆-Alkylthio, (C₁-C₆-Alkyl)carbonyl, (C₁-C₆-Alkoxy)carbonyl, (C₁-C₆-Alkyl)carbonyloxy, (C₃-C₆-Alkenyloxy)carbonyl;
(C₁-C₆-Halogenalkyl)carbonyl, (C₁-C₆-Alkoxy)carbonyl, C₁-C₆-Alkylaminocarbonyl, Di(C₁-C₆-alkyl)aminocarbonyl, C₁-C₆-Alkyloximino-C₁-C₆-alkyl, C₃-C₆-Cycloalkyl;
Phenyl oder Phenyl-C₁-C₆-alkyl, worin die Phenylringe unsubstituiert oder ihrerseits ein bis drei Substituenten tragen können, jeweils ausgewählt unter Cyano, Nitro, Halogen, C₁-C₆-Alkyl, C₁-C₆-Halogenalkyl, C₁-C₆-Alkoxy und (C₁-C₆-Alkoxy)carbonyl;
- R²⁷,: R²⁸, R²⁹, R³⁰ unabhängig voneinander Wasserstoff, C₁-C₆-Alkyl, C₃-C₆-Alkenyl, C₂-C₆-Alkinyl, C₃-C₆-Cycloalkyl, C₁-C₆-Halogenalkyl, C₁-C₆-Alkoxy-C₁-C₆-alkyl, (C₁-C₆-Alkyl)carbonyl, (C₁-C₆-Alkoxy)carbonyl, (C₁-C₆-Alkoxy)carbonyl-C₁-C₆-alkyl,
(C₁-C₆-Alkoxy)carbonyl-C₂-C₆-alkenyl, worin die Alkenylkette zusätzlich ein bis drei Halogen- und/oder Cyano-Reste tragen kann, C₁-C₆-Alkylsulfonyl, (C₁-C₆-Alkoxy)carbonyl-C₁-C₆-alkylsulfonyl, Phenyl oder Phenylsulfonyl, wobei die Phenylringe der beiden letztgenannten Reste unsubstituiert sein oder ihrerseits einen bis drei Substituenten tragen können, jeweils ausgewählt unter Cyano, Nitro, Halogen, C₁-C₆-Alkyl, C₁-C₆-Halogenalkyl, C₃-C₆-Alkenyl, C₁-C₆-Alkoxy und (C₁-C₆-Alkoxy)carbonyl; oder
- R²⁷: und R²⁸ und/oder
- R²⁹: und R³⁰ zusammen mit dem jeweils gemeinsamen Stickstoffatom für einen gesättigten oder ungesättigten 4- bis 7-gliedrigen Azaheterocyclus, der neben Kohlenstoffringgliedern gewünschtenfalls eines der folgenden Glieder enthalten kann: -O-, -S-, -N=, -NH- oder -N(C₁-C₆-Alkyl)-.

Im Hinblick auf die Verwendung der substituierten Harnstoffe der Formel V als Herbizide haben die Variablen vorzugsweise folgende Bedeutungen, und zwar jeweils für sich allein oder in Kombination:
- Z: O oder S
- R^{A}: CO₂R¹, Halogen, Cyano, OR² oder C₁-C₃-Alkyl;
- Q: Q₁, Q₂ oder Q₄;
- X, Y und Y': unabhängig voneinander O oder S;
- T: eine chemische Bindung oder O;
- U: eine chemische Bindung, C₁-C₄-Alkylen, O oder S;
- R: C(O)OR³, C(O)SR³, C(S)OR³, C(S)SR³, CHO, CN, C(O)R², C(O)NR⁴R⁵, C(S)NR⁴R⁵, C(O)NHC(O)₂OR⁶, C(O)NHS(O)₂R⁶, C(O)NHS(O)₂OR⁶;
- R¹: Wasserstoff oder C₁-C₃-Alkyl;
- R²: C₁-C₃-Alkyl, C₃-C₆-Cycloalkyl, C₃-C₆-Alkenyl, C₃-C₆-Alkinyl, C₁-C₃-Haloalkyl, C₁-C₃-Alkoxycarbonyl-C₁-C₃-alkyl, Cyano-C₁-C₃-alkyl, Benzyl, welches mit Halogen, C₁-C₄-Alkyl oder Trifluormethyl substituiert sein kann, oder
Phenyl, welches mit Halogen, C₁-C₄-Alkyl, Trifluormethyl oder C₁-C₄-Alkoxy substituiert sein kann;
- R³: C₁-C₁₅-Alkyl, C₃-C₈-Cycloalkyl, C₂-C₁₀-Alkenyl, C₃-C₁₀-Alkinyl, C₁-C₆-Haloalkyl, C₁-C₆-Alkoxy-C₁-C₆-alkyl, C₁-C₆-Alkoxycarbonyl-C₁-C₆-alkyl, C₃-C₆-Alkenyloxy-C₁-C₆-alkyl, C₃-C₆-Alkinyloxy-C₁-C₆-alkyl, Cyano-C₁-C₆-alkyl,
Phenyl oder Benzyl, welche jeweils ein- bis fünffach durch Halogen, C₁-C₄-Alkyl, C₁-C₄-Halogenalkyl, C₁-C₄-Alkoxy, C₁-C₄-Halogenalkoxy, Amino, C₂-C₄-Monoalkylamino, C₁-C₄-Dialkylamino, C₁-C₄-Alkoxycarbonyl, Nitro oder Cyano substituiert sein können;
- R⁴: Wasserstoff, C₁-C₆-Alkyl oder C₁-C₆-Alkoxy;
- R⁵,: R⁶ unabhängig voneinander Wasserstoff, C₁-C₆-Alkyl, C₂-C₆-Alkenyl, C₂-C₆-Alkinyl, C₁-C₆-Halogenalkyl, C₁-C₆-Alkoxy-C₁-C₆-alkyl, Phenyl oder Benzyl, welche ein- bis fünffach durch Halogen, C₁-C₄-Alkyl, C₁-C₄-Halogenalkyl, C₁-C₄-Alkoxy, C₁-C₄-Dialkylamino, C₁-C₄-Alkoxycarbonyl, Nitro oder Cyano substituiert sein können; oder
- R⁴ und R⁵: stehen zusammen mit dem gemeinsamen Stickstoffatom für einen gesättigten oder ungesättigten 4- bis 7-gliedrigen Azaheterocyclus;
- R⁸: Wasserstoff, Fluor oder Chlor;
- R⁹: Chlor, Trifluormethyl oder Cyano;
- R¹⁰: Wasserstoff, Hydroxy, Mercapto, Cyano, Nitro, Halogen, C₁-C₆-Alkyl, C₁-C₆-Halogenalkyl, C₁-C₆-Halogenalkoxy, C₁-C₆-Halogenalkylthio, C₁-C₆-Alkoxy-(C₁-C₆-alkyl)carbonyl, C₁-C₆-Alkylthio-(C₁-C₆-alkyl)carbonyl, (C₁-C₆-Alkyl)-iminooxycarbonyl, C₁-C₆-Alkoxy-C₁-C₆-alkyl, C₁-C₆-Alkoxyamino-C₁-C₆-alkyl, C₁-C₆-Alkoxy-C₁-C₆-alkylamino-C₁-C₆-alkyl,
C₁-C₆-Alkoxy, C₁-C₆-Alkylthio, C₃-C₆-Cycloalkoxy, C₃-C₆-Cycloalkylthio, C₂-C₆-Alkenyloxy, C₂-C₆-Alkenylthio, C₂-C₆-Alkinyloxy, C₂-C₆-Alkinylthio, (C₁-C₆-Alkyl) carbonyloxy, (C₁-C₆-Alkyl)carbonylthio, (C₁-C₆-Alkoxy)carbonyloxy, (C₂-C₆-Alkenyl)carbonyloxy, (C₂-C₆-Alkenyl)carbonylthio, (C₂-C₆-Alkinyl)-carbonyloxy, (C₂-C₆-Alkinyl)carbonylthio, C₁-C₆-Alkylsulfonyloxy oder C₁-C₆-Alkylsulfonyl, wobei jeder dieser 17 Reste gewünschtenfalls einen zwei oder drei Substituenten tragen kann, ausgewählt unter:
- Halogen, Nitro, Cyano, Hydroxy, C₃-C₆-Cycloalky, C₁-C₆-Alkoxy, C₃-C₆-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy, Oxo, =N-OR¹³
- Phenyl, Phenoxy oder Phenylsulfonyl, wobei die drei letztgenannten Substituenten ihrerseits einen, zwei oder drei Substituenten tragen können, jeweils ausgewählt unter Halogen, Nitro, Cyano, C₁-C₆-Akyl, C₁-C₆-Halogenalkyl, C₁-C₆-Alkoxy und (C₁-C₆-Alkoxy)carbonyl;
- -CO-R¹⁴, -CO-OR¹⁴, -CO-SR¹⁴, -CO-N(R¹⁴)-R¹⁵, -OCO-R¹⁴, -OCO-OR^{14'}, -OCO-SR^{14'}, -OCO-N(R¹⁴)-R¹⁵, -N(R¹⁴)-R¹⁵, und -C(R¹⁶)=N-OR¹³;
C(Z¹)-R¹⁷, -C(=NR¹⁸)R¹⁷, C(R¹⁷)(Z²R¹⁹)(Z³R²⁰), C(R¹⁷)=C(R²¹)-CN, C(R¹⁷)=C(R²¹)-CO-R²², -CH(R¹⁷)-CH(R²¹)-COR²², -C(R¹⁷)=C(R²¹)-CH₂-CO-R²², -C(R¹⁷)=C(R²¹)-C(R²³)=C(R²⁴)-CO-R²², -C(R¹⁷)=C(R²¹)-CH₂-CH(R²⁵)-CO-R²², -CO-OR²⁶, -CO-SR²⁶, -CON(R²⁶)-OR¹³, -C≡C-CO-NHOR¹³, -C≡C-CO-N(R²⁶)-OR¹³, -C≡C-CS-NH-OR¹³, -C≡C-CS-N(R²⁶)-OR¹³, -C(R¹⁷)=C(R²¹)-CO-NHOR¹³, -C(R¹⁷)=C(R²¹)-CO-N(R²⁶)-OR¹³, -C(R¹⁷)=C(R²¹)-CS-NHOR¹³, -C(R¹⁷)=C(R²¹)-CS-N(R²⁶)-OR¹³, -C(R¹⁷)=C(R²¹)-C(R¹⁶)=N-OR¹³, C(R¹⁶)=N-OR¹³, -C≡C-C(R¹⁶)=NOR¹³, C(Z²R¹⁹)(Z³R²⁰)-OR²⁶, -C(Z²R¹⁹)(Z²R²⁰)SR²⁶, Q(Z²R¹⁹)(Z³R²⁰)-N(R²⁷)R²⁸, -N(R²⁷)-R²⁸, -CO-N(R²⁷)-R²⁸ oder -C(R¹⁷)=C(R²¹)CO-N(R²⁷)R²⁸; worin Z¹, Z², Z³ unabhängig voneinander für Sauerstoff oder Schwefel stehen;
- R¹¹: Wasserstoff, C₁-C₆-Alkyl, C₁-C₆-Haloalkyl, C₃-C₆-Alkenyl, C₃-C₆-Alkinyl, C₃-C₇-Cycloalkyl, oder 3- bis 7-gliedriges gesättigtes Heterocyclyl, ein oder mehrere Sauerstoff und/oder Schwefel-Atome enthaltend;
- R^{11'}: Wasserstoff oder C₁-C₆-Alkyl;
- R¹²: Wasserstoff oder C₁-C₃-Alkyl;
- R^{12'}: Wasserstoff, C₁-C₃-Alkyl
- R¹² und R^{12'}: gemeinsam C=O;
- R¹³: Wasserstoff, C₁-C₆-Alkyl, C₁-C₆-Halogenalkyl, C₃-C₆-Cycloalkyl, C₃-C₆-Alkenyl, C₃-C₆-Alkinyl, C₁-C₆-Alkoxy-C₁-C₆-alkyl, Cyano-C₁-C₆-alkyl, (C₁-C₆-Alkoxy)carbonyl-C₁-C₆-alkyl oder Phenylalkyl, wobei der Phenylring ein- bis dreifach durch Halogen, Cyano, Nitro, C₁-C₃-Alkyl, C₁-C₃-Halogenalkyl oder C₁-C₃-Alkoxy substituiert sein kann;
- R¹⁴: Wasserstoff, C₁-C₆-Alkyl, C₃-C₆-Cycloalkyl, C₃-C₆-Alkenyl, C₃-C₆-Alkinyl, C₁-C₆-Alkoxy-C₁-C₆-alkyl, (C₁-C₆-Alkoxy)carbonyl-C₁-C₆-alkyl, (C₁-C₆-Alkenyloxy)carbonyl-C₁-C₆-alkyl,
Phenyl oder Benzyl, welche am Phenylring unsubstituiert oder ein- bis dreifach durch Halogen, Cyano, Nitro, C₁-C₃-Alkyl, C₁-C₃-Halogenalkyl oder C₁-C₃-Alkoxy substituiert sein können;
- R^{14'}: die für R¹⁴ genannten Bedeutungen, ausgenommen Wasserstoff;
- R¹⁵: Wasserstoff, Hydroxy, C₁-C₆-Alkyl, C₁-C₆-Alkoxy, (C₁-C₃-Alkoxy)carbonyl-C₁-C₃-alkoxy, C₃-C₆-Alkenyl oder C₃-C₆-Alkinyl;
- R¹⁶: Wasserstoff, Halogen, C₁-C₆-Alkyl, C₁-C₆-Halogenalkyl, C₁-C₆-Alkoxy, C₃-C₆-Alkenyloxy, (C₁-C₆-Alkoxy)carbonylalkoxy, C₂-C₆-Alkenyl, (C₂-C₆-Alkenyl)carbonyloxy, C₃-C₆-Alkinyl, (C₂-C₆-Alkinyl)carbonyloxy,
Phenyl, Phenoxy oder Benzyl, wobei die Phenylringe der letztgenannten 3 Reste unsubstituiert oder ein- bis dreifach durch Halogen, Cyano, Nitro, C₁-C₃-Alkyl, C₁-C₃-Halogenalkyl, C₁-C₃-Alkoxy oder (C₁-C₃-Alkoxy)carbonyl substituiert sein können;
- R¹⁷: Wasserstoff, Cyano, C₁-C₆-Alkyl, C₁-C₆-Halogenalkyl, C₂-C₆-Alkenyl, C₂-C₆-Alkinyl, C₁-C₆-Alkoxy-C₁-C₆-alkyl oder (C₁-C₆-Alkoxy)carbonyl;
- R¹⁸: Wasserstoff, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkoxy-C₁-C₆-alkyl, C₁-C₆-Alkoxy, (C₁-C₆-Alkoxy)carbonyl-C₁-C₆-alkyl,
Phenyl oder Phenyl-(C₁-C₆-alkyl), wobei die beiden letztgenannten Phenylreste durch Halogen, Cyano, Nitro, C₁-C₃-Alkyl, C₁-C₃-Halogenalkyl, C₁-C₃-Alkoxy oder (C₁-C₃-Alkoxy)carbonyl substituiert sein können;
- R¹⁹,: R²⁰ unabhängig voneinander C₁-C₆-Alkyl, C₁-C₆-Halogenalkyl, C₃-C₆-Alkenyl, C₃-C₆-Alkinyl, C₁-C₆-Alkoxy-C₁-C₆-alkyl, oder
- R¹⁹ und R²⁰: zusammen für eine gesättigte 2- bis 4-gliedrige Kohlenstoffkette, die einen Oxosubstituenten tragen kann, wobei ein den Variablen Z² und Z³ nicht benachbartes Kohlenstoffatom dieser Kette durch -O-, -S-, -N=, -NH- oder -N(C₁-C₆-Alkyl)- ersetzt sein kann, und wobei die Kohlenstoffkette noch ein- bis dreimal durch Halogen oder C₁-C₆-Alkyl substituiert sein kann;
- R²¹: Wasserstoff, Cyano, Halogen, C₁-C₆-Alkyl, C₁-C₆-Halogenalkyl oder C₁-C₆-Alkoxy;
- R²²: Wasserstoff, OR³¹, S-R³¹, C₁-C₆-Alkyl, das noch einen oder zwei C₁-C₆-Alkoxysubstituenten tragen kann, C₂-C₆-Alkenyl, C₂-C₆-Alkinyl, C₁-C₆-Halogenalkyl oder C₃-C₆-Cycloalkyl;
- R²³: Wasserstoff, Cyano, Halogen, C₁-C₆-Alkyl, C₃-C₆-Alkenyl oder C₃-C₆-Alkinyl;
- R²⁴: Wasserstoff, Cyano, Halogen, C₁-C₆-Alkyl, C₁-C₆-Alkoxy oder C₁-C₆-Halogenalkyl
- R²⁵: Wasserstoff, Cyano oder C₁-C₆-Alkyl;
- R²⁶,: R³¹ unabhänging voneinander Wasserstoff, C₁-C₆-Alkyl, C₁-C₆-Halogenalkyl, C₂-C₆-Alkenyl oder C₂-C₆-Alkinyl, wobei die letztgenannten 4 Gruppen jeweils einen oder zwei der folgenden Reste tragen können: Cyano, Halogen, C₁-C₆-Alkoxy, C₁-C₆-Alkylcarbonyl, (C₁-C₆-Alkoxy)carbonyl; oder (C₁-C₆-Alkyl)carbonyl, (C₁-C₆-Alkoxy)carbonyl, Phenyl oder Phenyl-C₁-C₆-alkyl;
- R²⁷, R²⁸, R²⁹, R³⁰: unabhängig voneinander Wasserstoff, C₁-C₆-Alkyl, C₃-C₆-Alkenyl, C₂-C₆-Alkinyl, C₃-C₆-Cycloalkyl, C₁-C₆-Halogenalkyl, C₁-C₆-Alkoxy-C₁-C₆-alkyl, C₁-C₆-Alkylcarbonyl, (C₁-C₆-Alkoxy)carbonyl,oder
- R²⁷ und R²⁸: und/oder R²⁹ und R³⁰ zusammen mit dem jeweils gemeinsamen Stickstoffatom für einen gesättigten oder ungesättigten 4- bis 7-gliedrigen Azaheterocyclus, der neben Kohlenstoffringgliedern gewünschtenfalls ein Sauerstoffatom oder eine -NH-Gruppe enthalten kann;
- m: 0, 1, 2 oder 3;

R⁹ steht insbesondere für Halogen, und speziell für Fluor oder Chlor. R^{A} steht insbesondere für Wasserstoff, d.h. m steht für 0.

Insbesondere steht R¹⁰ in Q1 für:
C₁-C₆-Alkoxy, C₂-C₆-Alkenyloxy oder C₂-C₆-Alkinyloxy, wobei jeder der letztgenannten 3 Reste gewünschtenfalls ein bis drei Substituenten tragen kann, jeweils ausgewählt aus der Gruppe bestehend aus Halogen, C₁-C₆-Alkoxy, C₃-C₆-Alkenyloxy, C₃-C₆-Alkinyloxy, C₁-C₆-Alkylsulfonyl, -CO-R¹⁴, -CO-OR¹⁴, -CO-N(R¹⁴)-R¹⁵, -N(R¹⁴)-R¹⁵, und -C(R¹⁶)=N-OR¹³;
-CO-R¹⁷, -C(NR¹⁸)-R¹⁷, -C(R¹⁷)(OR¹⁹)(OR²⁰), -C(R¹⁷)=C(R²¹)-CO-R²², -CH(R¹⁷)-CH(R²¹)-CO-R²², -CO-OR²⁶, -CO-N(R²⁶)-OR¹³, -C(R¹⁷)=C(R²¹)-CO-N(R²⁶)-OR¹³, -C(R¹⁶)=N-OR¹³, -C(OR¹⁹)(OR²⁰)-OR²⁶, -N(R²⁷)R²⁸, -CON(R²⁷)R²⁸ oder -C(R¹⁷)=C(R²⁷)CO-N(R²⁷)R²⁸;
worin R¹³ bis R²² und R²⁶ bis R²⁸ die zuvor genannten Bedeutungen aufweisen,
und speziell für C₂-C₆-Alkenyloxy, C₂-C₆-Alkinyloxy, -C(R¹⁷)(OR¹⁹)(OR²⁰), -C(R¹⁷)=C(R²¹)-C(O)R²¹, -CH(R¹⁷)-CH(R²¹)-C(O)R²², C(O)OR²⁶, -C(O)-N(R²⁶)-OR¹³, -C(R¹⁶)=N-OR¹³ und C(O)N(R²⁷)R²⁸, worin R¹³, R¹⁶, R¹⁷, R¹⁹, R²⁰, R²¹, R²², R²⁶, R²⁷ und R²⁸ die zuvor genannten und insbesondere die im folgenden genannten Bedeutungen haben:
- R¹³: C₁-C₆-Alkyl, C₁-C₆-Haloalkyl, C₃-C₆-Alkenyl, C₃-C₆-Haloalkenyl, C₃-C₆-Alkinyl, C₁-C₆-Cyanoalkyl und C₁-C₆-Alkoxycarbonyl-C₁-C₆-alkyl;
- R¹⁶: Wasserstoff, C₁-C₆-Alkyl, C₁-C₆-Alkoxy, C₁-C₆-Alkoxycarbonyl-C₁-C₆-alkyl, C₁-C₆-Alkoxycarbonyl-C₁-C₆-alkoxy und Phenoxycarbonyl-C₁-C₆-alkoxy;
- R¹⁷: Wasserstoff, C₁-C₆-Alkyl;
- R¹⁹ und R²⁰: unabhängig voneinander C₁-C₆-Alkyl;
- R²¹: Wasserstoff, Halogen, C₁-C₆-Alkyl;
- R²²: Hydroxy, C₁-C₆-Alkoxy, C₁-C₆-Alkylthio, C₁-C₆-Alkoxycarbonyl-C₁-C₆-alkyl;
- R²⁶: C₁-C₆-Alkyl, C₃-C₆-Haloalkyl, C₃-C₆-Alkenyl, C₃-C₆-Alkinyl, C₁-C₆-Alkoxycarbonyl-C₁-C₆-alkyl, C₃-C₆-Alkenyloxycarbonyl-C₁-C₆-alkyl, C₃-C₆-Alkinyloxycarbonyl-C₁-C₆-alkyl, C₁-C₆-Alkoxyalkyl;
- R²⁷: Wasserstoff, C₁-C₆-Alkyl;
- R²⁸: Wasserstoff, C₁-C₆-Alkyl, C₁-C₆-Alkoxy,
- oder R²⁷ und R²⁸: gemeinsam einen 6-gliedrigen, gesättigten Azaheterocyclus, der gegebenenfalls ein oder zwei nicht benachbarte Sauerstoffatome im Ring aufweist.

Zur Herstellung der Verbindungen der Formel V aus den Verbindungen der Formeln I und VI gibt man vorzugsweise das Isocyanat bzw. das Isothiocyanat der allgemeinen Formel VI innerhalb 5 bis 120 min, vorzugsweise innerhalb 10 bis 30 min zu der Verbindung I, die vorzugsweise als Mischung mit einem der in Schritt 1 bei der Herstellung von I genannten Lösungsmittel vorgelegt wird. Man kann jedoch auch die Verbindung VI vorlegen, vorzugsweise in einem der vorgenannten Lösungsmittel, und hierzu die Verbindung I geben.

Die Reaktionsbedingungen entsprechen im Wesentlichen den bei Schritt 1 der Herstellung von I genannten Reaktionsbedingungen.

Vorzugsweise erfolgt die Zugabe der Verbindung VI zu der Verbindung I, bzw. die Zugabe der Verbindung I zu der Verbindung VI bei Temperaturen im Bereich von 0 bis 50°C, insbesondere 10 bis 25°C. Zur Vervollständigung der Umsetzung läßt man die Komponenten vorzugsweise bei Temperaturen im Bereich von 20 bis 80°C etwa 0,5 bis 20 h, insbesondere 1 bis 10 h nachreagieren.

Vorzugsweise führt man die Reaktion unter inerten Bedingungen, d.h. unter Ausschluß von Sauerstoff, z.B. unter inerten Gasen, in einem trockenen, d.h. von Wasser und Alkoholen weitgehend befreitem Lösungsmittel durch.

Man kann die Umsetzung durch Zugabe einer Lewissäure zur Aktivierung des Isocyanats bzw. des Isothiocyanats der Formel VI beschleunigen. Übliche Lewissäuren sind für diesen Zweck zinnorganische Verbindungen, z.B. Di-C₂-C₈-alkylzinn(IV)dicarboxylate, wie Di-n-butylzinn(IV)diacetat. Üblicherweise wird die Lewissäure in einer Menge von bis zu 15 Gew.-%, vorzugsweise 1 bis 10 Gew.-% und insbesondere 2 bis 5 Gew.-%, bezogen auf das Isocyanat bzw. Isothiocyanat der Formel VI eingesetzt.

Die Umsetzung kann auch durch Zugabe von nucleophilen Basen, beispielsweise Pyridin, α-, β- oder γ-Picolin, Chinolin, 4-Dimethylaminopyridin oder 4-Pyrrolidinopyridin beschleunigt werden. Die nucleophile Base wird üblicherweise in einer Menge von bis zu 15 Gew.-%, vorzugsweise 0,5 bis 10 Gew.-% und insbesondere 0,5 bis 5 Gew.-%, bezogen auf die Verbindung VI eingesetzt.

Die so erhältlichen substituierten Harnstoffe der allgemeinen Formel V sind einerseits wirksame Herbizide. Sie sind aber auch geeignete Ausgangsstoffe für die Herstellung von Herbiziden mit Triazolin-2,5-dion-Struktur (Verbindungen der allgemeinen Formel VIII), sofern R für einen Rest steht, der aus den Verbindungen V - beispielsweise durch Hydrolyse - abgespalten werden kann.

Beispiele für hydrolysierbare Reste R sind COOH, CHO, C(O)OR³, C(S)OR³, C(S)SR³ und C(O)SR³.

Die Hydrolyse der N-substituierten Harnstoffe mit Diazinstruktur (Verbindungen V) zu den Verbindungen VII mit unsubstituiertem Stickstoff in der 4-Position gelingt in einfacher Weise durch Hydrolyse mit Wasser.

Die Umsetzung erfolgt zweckmäßigerweise in Gegenwart einer Hilfsbase. Die Reaktionstemperatur liegt üblicherweise im Bereich von 10 bis 100°C, vorzugsweise im Bereich von 20 bis 80°C.

Vorzugsweise wird die Umsetzung in einem Lösungsmittel, beispielsweise einem mit Wasser mischbaren Lösungsmittel, z.B. einem C₁-C₄-Alkohol, wie Ethanol oder Methanol, einem cyclischen Ether, wie Tetrahydrofuran oder Dioxan, einem Amid, wie Dimethylformamid, Dimethylacetamid, N-Methylpyrrolidon; Dimethylsulfoxid, Acetonitril, einer Mischung der vorgenannten Lösungsmittel untereinander oder einer Mischung mit Wasser oder in Wasser selber durchgeführt.

Als Basen sind alle vorgenannten Basen geeignet. Bevorzugt Alkali- oder Erdalkalihydroxid, besonders bevorzugt Natriumhydroxid.

Die Base wird vorteilhaft in einem Molverhältnis von 0,9 bis 1,4, vorzugsweise 0,95 bis 1,2, besonders bevorzugt 0,98 bis 1,15 für das Verhältnis von substituierten Harnstoff Ib zu Base eingesetzt.

Die so gewonnenen Verbindungen VII können gemäß dem nachfolgenden Schema zu den Verbindungen VIII cyclisiert werden. Verbindungen VII und VIII weisen herbizide Aktivitäten auf.

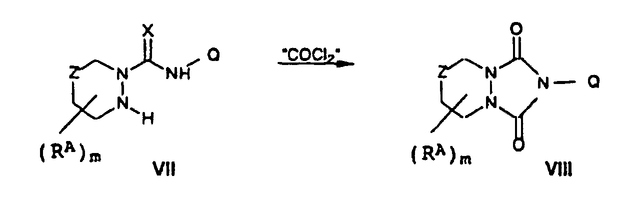

Die Cyclisierung der 3-(Arylcarbamoyl)-tetrahydro-4H-1,3,4-ox (bzw. thia)diazine VII wird mittels Phosgen oder einem Phosgenersatz, z.B. Diphosgen (ClC(=O)OCCl₃), vorteilhaft in Gegenwart eines der vorgenannten wasserfreien Lösungsmittel bei Temperaturen im Bereich von -10 bis 120°C, vorzugsweise 0 bis 80°C, besonders bevorzugt 10 bis 60°C durchgeführt.

Vorteilhaft leitet man das Phosgen bei 10 bis 60°C unter Rühren in eine Mischung von VII und einer Menge von 0,5 bis 5 Gew.-%, bezogen auf den Einsatzstoff, Aktivkohle als Katalysator in einem der vorgenannten wasserfreien Lösungsmittel während 0,5 bis 20 Stunden, bevorzugt 1 bis 12 Stunden.

Die Reaktion kann zusätzlich durch einen basischen Amid-Katalysator beschleunigt werden, z.B. DMF, das üblicherweise in einer Menge von 0,3 bis 10 Gew.-% bezogen auf den Eingangsstoff eingesetzt werden kann. Als basischen Katalysator kann man auch organische Basen wie Triethylamin, Tri-n-propylamin, N,N-Dimethylanilin oder N,N-Dimethylcyclohexylamin verwenden. vorzugsweise kann man auch Pyridin, ggf. direkt als Lösungsmittel einsetzen.

An Stelle von Phosgen kann man auch Diphosgen verwenden. Vorteilhaft gibt man das Diphosgen während 2 bis 20 min unter Rühren bei 0 bis -5°C zu der Mischung des Ausgangsstoffes und einem der vorgenannten Lösungsmittel, ggf. unter Zusatz von Aktivkohle, DMF oder der organischen Base, läßt innerhalb 1 Stunde auf 10°C erwärmen und rührt dann noch 1 bis 12 Stunden bei 10 bis 60°C. Die molare Menge an Phosgen bzw. Diphosgen beträgt 0,98 bis 5, vorzugsweise 1 bis 3, besonders bevorzugt 1 bis 1,3 mol pro mol Ausgangsstoff.

Die Konzentration der Edukte im Lösungsmittel beträgt im Allgemeinen 0,1 bis 5 mol/l, bevorzugt 0,2 bis 2 mol/l.

Die Reaktion kann drucklos oder unter Druck, kontinuierlich oder diskontinuierlich durchgeführt werden.

Die im folgenden aufgeführten Beispiele sollen die Erfindung verdeutlichen.

### Beispiel 1

### Tetrahydro-4H-1,3,4-oxdiazin-4-carbonsäuremethylester (erfindungsgemäße Verbindung)

### a) N-Amino-N-2-hydroxyethyl-carbamidsäuremethylester (Verbindung IV.1a - vergleiche Tabelle 3)

248,4 g (2,628 mol) Methylchlorformiat wurden bei 0 bis 5°C innerhalb 30 min unter Rühren zu einer Mischung von 200 g (2,628 mol) 2-Hydrazionethanol und 266 g (2,628 mol) Triethylamin in 1600 ml Methylenchlorid gegeben. Nach 3 h Rühren bei 3 bis 22°C wurde das entstandene Hydrochlorid durch Filtration abgetrennt und mit THF gewaschen. Die Filtrate wurden vereinigt. Der Filterrückstand wurde in 800 ml THF suspendiert und erneut filtriert. Auch dieses Filtrat wurde mit dem vorhergehenden Filtrat vereinigt. Die vereinigten Filtrate engte man im Vakuum ein. Man erhielt 366 g der Titelverbindung als farbloses Öl mit einer HPLC-Reinheit von 95,3% entsprechend einer Ausbeute von 98,9% d.Th. Nach dem GC betrug die Reinheit 85,2%.
¹H-NMR (400 MHZ, d₆-DMSO) δ(ppm) : 4,4 - 4,8 (breit/3H) NH₂/OH; 3,6 (s/3H) CH₃O; 3,52 (t/2H) und 3,35 (t/2H) CH₂-CH₂

### b) Tetrahydro-4H-1,3,4-oxdiazin-4-carbonsäuremethylester (Verbindung Ib.1b, vergleiche Tabelle 4)

22,4 g (0,746 mol) Paraformaldehyd wurden innerhalb 2 min unter Rühren bei 22°C zu einer Mischung von 100 g (0,746 mol) N-Amino-N-2-hydroxyethylcarbamidsäuremethylester in 1500 ml Methylenchlorid gegeben. Nach Zugabe von 8,5 g (0,045 mol) p-Toluolsulfonsäure rührte man 21 h bei 42°C, bis sich der Niederschlag gelöst hatte, kühlte dann auf 20°C ab und gab Magnesiumsulfat zu. Nach Filtration engte man im Vakuum ein. Man erhielt 111,8 g der Titelverbindung als farbloses Harz mit einer GC-Reinheit von 85% entsprechend einer Ausbeute von 85,8% d.Th.
¹H-NMR (500 MHZ, CF₃CO₂D) δ(ppm): 5,09 (s/2H) CH₂; 4,02 (s/3H) CH₃O; 3,8 - 4,25 (m/4H) CH₂CH₂
IR ν (C=O) 1703 cm⁻¹

Analog Beispiel 1 a wurden die in Tabelle 3 aufgeführten Vorstufen 2a bis 15a erhalten. In Tabelle 3 sind Brechungsindex (n_{D}²³) und/oder charakteristische IR-Banden (NH, CO) angegeben.

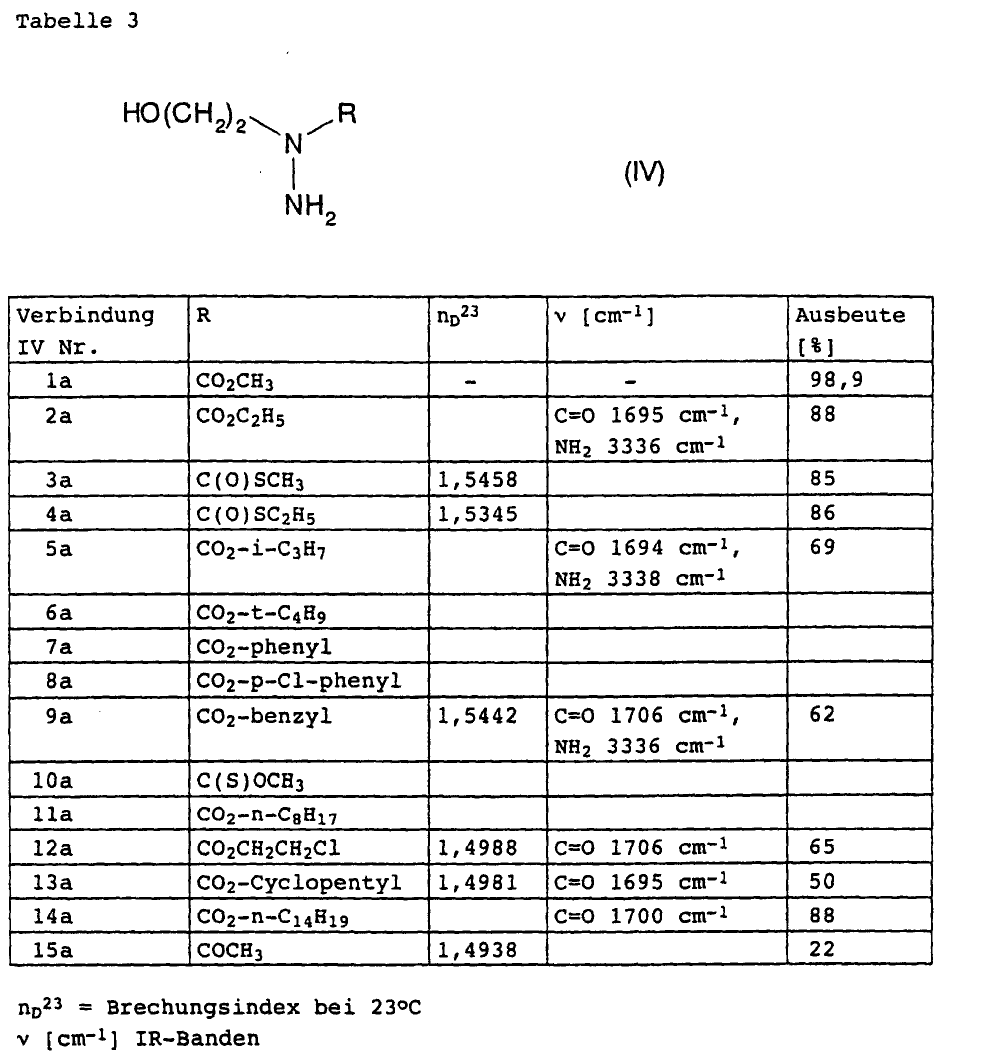

Analog Beispiel 1 b wurden die in Tabelle 4 aufgeführten Zielverbindungen der Formel Ib (Verbindungen Ib.2b bis Ib.15b) erhalten. In Tabelle 4 sind Brechungsindex, charakteristische IR-Banden (NH, CO) und/oder ¹H-NMR-Daten angegeben.

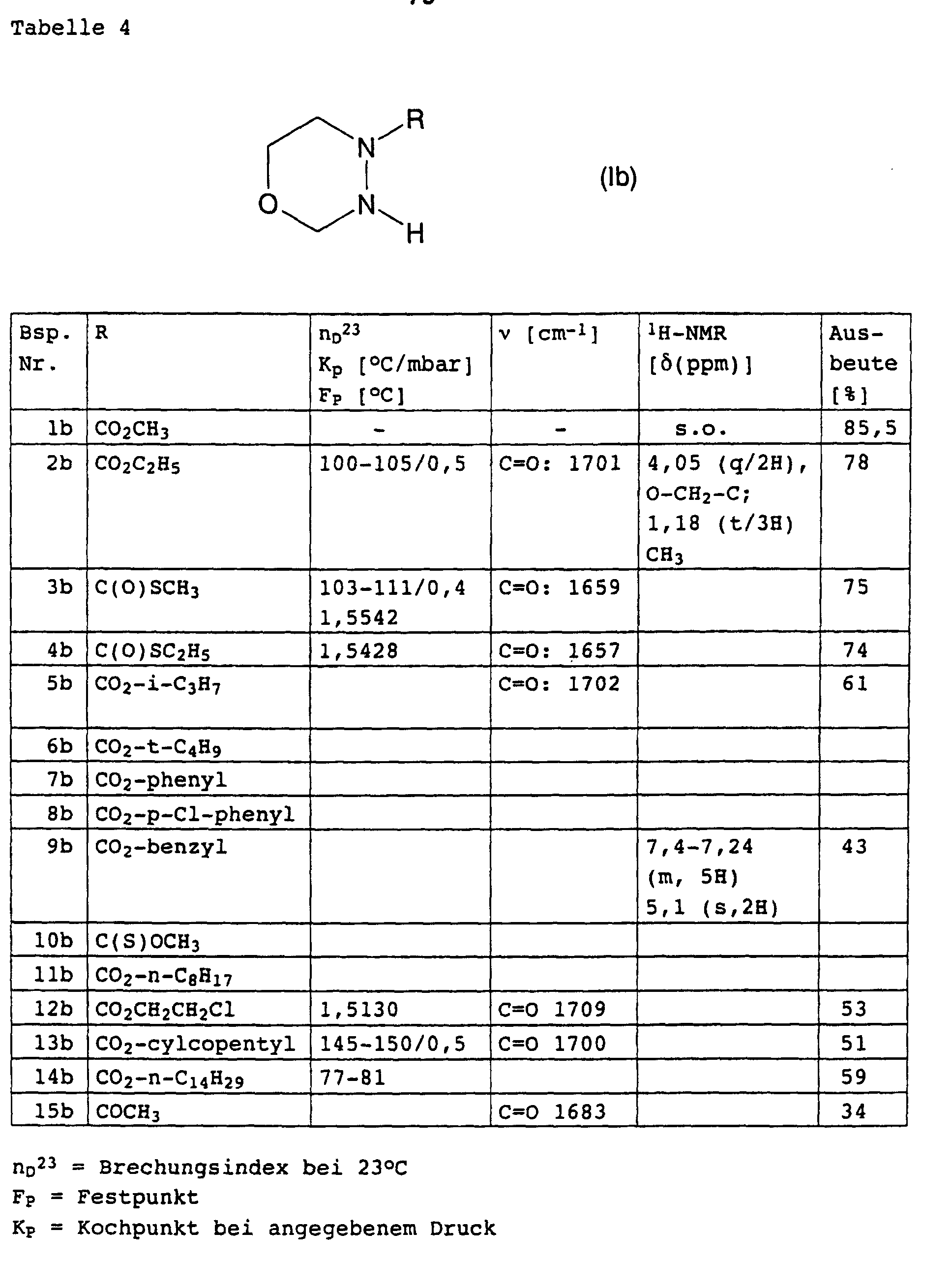

### Anwendungsbeispiel 1 (Verfahren)

### 1 a) 3-[N-(4'-Chlor-2'-fluor-5'-propargyloxyphenyl)aminocarbonyl]-tetrahydro-4H-1,3,4-oxdiazin-4-carbonsäuremethylester

5,7 g (25,25 mmol) 4-Chlor-2-fluor-5-propargyloxyphenylisocyanat in 50 ml THF wurden innerhalb 10 min unter Rühren zu einer Mischung von 3,7 g (25,25 mmol) der Verbindung aus Beispiel 1 b in 150 ml THF bei 22°C gegeben, wobei sich die Reaktionslösung innerhalb 1 h auf 25°C erwärmte.

Nach 12 h bei 22°C wurde die Reaktionslösung eingeengt und über Kieselgel mit Methylenchlorid/Ether 2:1 chromatographiert. Nach dem Einengen erhielt man 7,4 g (79% d.Th.) der Titelverbindung vom Fp. 130°C.

### 1 b) 3-[N-(4-Chlor-2-fluor-5-propargyloxyphenyl)aminocarbonyl]-tetrahydro-4H-1,3,4-oxdiazin

0,41 g (10,35 mmol) Natriumhydroxid, gelöst in 25 ml Wasser wurden innerhalb 20 min unter Rühren bei 60 bis 70°C zu einer Lösung von 3,5 g (9,42 mmol) der Verbindung aus Beispiel 1 a in 75 ml Ethanol gegeben. Nach der dünnschichtchromatographischen Untersuchung war vollständige Umsetzung eingetreten. Man kühlte ab und engte im Vakuum weitgehend ein. Nach Zugabe von 50 ml Wasser wurde mit 1 n Salzsäure angesäuert, wobei unter leichtem Schäumen Gasentwicklung eintrat und sich ein Niederschlag bildete. Die wässrige Phase wurde mit Methylenchlorid dreimal extrahieert, die vereinigten Extrakte über Magnesiumsulfat getrocknet und im Vakuum zur Trockene eingeengt. Man erhielt 3,0 g eines farblosen Pulvers mit dem F.P. 138-142°C. Das Pulver enthielt laut GC/MS-Untersuchung 1,71 g der Titelverbindung (Ausbeute: 58% d. Theorie).

Die Verbindung 1 b und das folgende Vergleichsprodukt lassen sic.. nach dem Stand der Technik (WO 94/10173) mit Phosgen oder einem Phosgenersatz zu herbiziden, anellierten Triazolen cyclisieren.

### Vergleichsbeispiel (WO 94/10173)

### Tetrahydro-4-[N-(4-chlor-2-fluor-5-propargyloxy-phenyl)aminocarbonyl-4H-1,3,4-oxdiazin

5,0 g (16,57 mmol) N-Amino-N-2-hydroxyethyl-N'-(4-chlor-2-fluor-5-propargyloxy-phenyl)harnstoff, 1,5 g (12,2 mmol) 37%iges Formalin und 0,5 g p-Toluolsulfonsäure wurden 17 h bei 42°C in Methylenchlorid am Wasserauskreiser gerührt. Nach dem Abkühlen wurde das Reaktionsgemisch im Vakuum teilweise eingeengt und dann über 200 ml Kieselgel chromatographiert. Es wurde mit Methylenchlorid, später mit Essigester chromatographiert. Man erhielt 3,6 g eines Substanzgemisches, das nach DC, HPLC und NMR aus 6 bis 7 Substanzen bestand. Nach der HPLC-Untersuchung mit unabhängig hergestelltem Vergleichsmaterial war die Titelverbindung mit 0,74 g (14,2% d.Th.) vertreten.

Beim Nachspülen der Säule mit Methanol konnte nur p-Toluolsulfonsäure isoliert werden.

Das erfindungsgemäße Verfahren führt somit zu Verbindungen VII in deutlich besseren Ausbeuten und stellt somit einen besseren Zugang zu den Herbiziden der allgemeinen Formel VIII dar.

## Patentansprüche

1. N-Substituierte Perhydro-3,4-diazine der Formel I 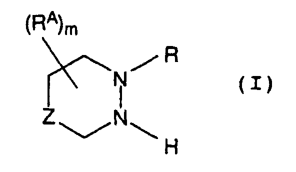 worin die Variablen Z, R, m und R^{A} die folgenden Bedeutungen haben:
Z O, S, S=O oder SO₂;
R^{A} Hydroxy, CO₂R¹, Halogen, Cyano, C(O)NR¹₂, OR², C₁-C₆-Alkyl, C₁-C₆-Halogenalkyl, C₃-C₆-Alkenyl, C₃-C₆-Alkinyl, COR¹, S(O)ₙR¹ mit n = 0, 1 oder 2, oder C(O)SR¹;
R CO₂H, CHO, CN, C(O)OR³, C(S)OR³, C(O)SR³, C(S)SR³, C(O)NR⁴R⁵, C(S)NR⁴R⁵, C(O)NHCO₂R⁶, C(O)NHSO₂R⁶, C(O)NHSO₃R⁶, C(O)R², P(O)R¹OR¹, P(O)(OR¹)₂, S(O)ₙR² mit n = 0, 1, oder 2 oder SO₂NHR¹;
m den Wert 0, 1, 2 oder 3;
und worin die Variablen R¹ bis R⁷ die folgenden Bedeutungen aufweisen:
R¹ Wasserstoff, C₁-C₆-Alkyl, C₁-C₆-Halogenalkyl, C₁-C₃-Alkoxy-C₁-C₃-alkyl, C₃-C₆-Alkenyl oder C₃-C₆-Alkinyl;
R² C₁-C₆-Alkyl, C₃-C₆-Cycloalkyl, C₃-C₆-Alkenyl, C₃-C₆-Allkinyl, C₁-C₆-Halogenalkyl, C₁-C₆-Alkoxy-C₁-C₆-alkyl, Hydroxycarbonyl-C₁-C₆-alkyl, C₁-C₆-Alkoxycarbonyl-C₁-C₆-alkyl, C₁-C₆-Akylthio-C₁-C₆-alkyl, C₁-C₆-Alkylsulfinyl-C₁-C₆-alkyl, C₁-C₆-Alkylsulfonyl-C₁-C₆-alkyl, C₃-C₆-Cycloalkyl-C₁-C₆-alkyl, C₃-C₆-Alkenyloxycarbonyl-C₁-C₆-alkyl, C₃-C₆-Alkinyloxycarbonyl-C₁-C₆-alkyl, C₃-C₆-Cycloalkoxy-C₁-C₆-alkyl, C₃-C₆-Alkenyloxy-C₁-C₆-alkyl, C₃-C₆-Alkinyloxy-C₁-C₆-alkyl, C₁-C₆-Halogenalkoxy-C₁-C₆-alkyl, C₃-C₆-Halogenalkenyloxy-C₁-C₆-alkyl, C₃-C₆-Halogenalkinyloxy-C₁-C₆-alkyl, C₃-C₆-Cycloalkyl-C₁-C₆-thioalkyl, C₃-C₆-Alkenylthio-C₁-C₆-alkyl, C₃-C₆-Alkinylthio-C₁-C₆-alkyl, Cyano-C₁-C₆-alkyl, C₃-C₆-Halogencycloalkyl-C₁-C₆-alkyl, Halogen-C₂-C₆-alkenyl, C₁-C₆-Alkoxy-C₃-C₆-alkenyl, C₁-C₆-Halogenalkoxy-C₃-C₆-alkenyl, C₁-C₆-Alkylthio-C₃-C₆-alkenyl, C₃-C₆-Halogenalkinyl, C₁-C₆-Alkoxy-C₃-C₆-alkinyl, C₁-C₆-Haloalkoxy-C₃-C₆-alkinyl, C₁-C₆-Alkylthio-C₃-C₆-alkinyl, C₁-C₆-Alkylcarbonyl, CHR¹COR⁷, CHR¹P(O)(OR⁷)₂, P(O)(OR⁷)₂, CHR¹P(S)(OR⁷)₂, CHR¹C(O)NR⁴R⁵, CHR¹C(O)NH₂,
Phenyl, Pyridyl, Benzyl, Phenoxy-C₁-C₆-alkyl, Benzyloxy-C₁-C₆-alkyl, wobei die Phenyl- bzw. Pyridylgruppen der fünf zuletzt genannten Substituenten mit Halogen, C₁-C₄-Alkyl, C₁-C₄-Alkoxy oder C₁-C₄-Halogenalkyl substituiert sein können,
R³ C₁-C₁₅-Alkyl, C₃-C₈-Cycloalkyl, C₂-C₁₀-Alkenyl, C₃-C₁₀-Alkinyl, C₁-C₆-Halogenalkyl, C₁-C₆-Alkoxy-C₁-C₆-alkyl, C₁-C₆-Alkylthio-C₁-C₆-alkyl, C₁-C₆-Alkylsulfinyl-C₁-C₆-alkyl, C₁-C₆-Alkylsulfonyl-C₁-C₆-alkyl, C₁-C₃-Alkoxy-C₁-C₃-alkoxy-C₁-C₃-alkyl, C₃-C₆-Cycloalkyl-C₁-C₆-alkyl, Carboxyl-C₁-C₆-alkyl, C₁-C₆-Alkoxycarbonyl-C₁-C₆-alkyl, C₁-C₆-Alkoxycarbonyl-C₂-C₆-alkenyl, C₃-C₆-Alkenyloxycarbonyl-C₁-C₆-alkyl, C₃-C₆-Alkinyloxycarbonyl-C₁-C₆-alkyl, C₃-C₈-Cycloalkoxy-C₁-C₆-alkyl, C₃-C₆-Alkenyloxy-C₁-C₆-alkyl, C₃-C₆-Alkinyloxy-C₁-C₆-alkyl, C₁-C₆-Halogenalkoxy-C₁-C₆-alkyl, C₃-C₆-Halogenalkenyloxy-C₁-C₆-alkyl, C₃-C₆-Halogenalkinyloxy-C₁-C₆-alkyl, C₃-C₆-Cycloalkyl-C₁-C₆-thioalkyl, C₃-C₆-Alkenylthio-C₁-C₆-alkyl, C₃-C₆-Alkinylthio-C₁-C₆-alkyl, Cyano-C₁-C₆-alkyl, C₃-C₆-Halogencycloalkyl-C₁-C₆-alkyl, Halogen-C₃-C₆-alkenyl, C₁-C₆-Alkoxy-C₃-C₆-alkenyl, C₁-C₆-Halogenalkoxy-C₃-C₆-alkenyl, C₁-C₆-Alkylthio-C₃-C₆-alkenyl, C₃-C₆-Halogenalkinyl, C₁-C₆-Alkoxy-C₃-C₆-alkinyl, C₁-C₆-Halogenalkoxy-C₃-C₆-alkinyl, C₁-C₆-Alkylthio-C₃-C₆-alkinyl,
CHR¹COR⁷, CHR¹P(O)(OR⁷)₂, P(O)(OR⁷)₂, CHR¹P(S)(OR⁷)₂, CHR¹C(O)NR⁴R⁵, CHR¹C(O)NH₂, Phenoxy-C₁-C₆-alkyl, Benzyloxy-C₁-C₆-alkyl, wobei die Phenylgruppen der zwei zuletzt genannten Substituenten mit Halogen, C₁-C₄-Alkyl, C₁-C₄-Alkoxy oder C₁-C₄-Halogenalkyl substituiert sein können;
Phenyl, Pyridyl, Naphthyl, Chinolyl, Chinazolyl, Chinoxalyl, 1-Methylindolyl, 1-Methylbenzimidazolyl, 2-Methylindazolyl, Benzofuranyl, Benzothienyl, Benzoxazolyl, Benzthiazolyl, Benzyl, wobei die 14 zuletzt genannten Substituenten in benachtbarten Positionen einen zweiwertigen Substituenten wie Methylendioxy, Difluormethylendioxy, Chlorfluormethylendioxy, Dichlormethylendioxy tragen können oder jeweils ein- bis fünffach durch Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Amino, C₁-C₄-Monoalkylamino, C₁-C₄-Dialkylamino, C₃-C₆-Cycloalkyl, C₁-C₄-Alkoxycarbonyl, C₁-C₄-Alkoxycarbonyl-C₁-C₄-alkyl, Hydroxy, Nitro oder Cyano substituiert sein können;
R⁴ Wasserstoff, C₁-C₆-Alkyl oder C₁-C₆-Alkoxy;
R⁵, R⁶ unabhängig voneinander Wasserstoff, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy-C₁-C₆-alkyl, C₁-C₆-Alkoxycarbonyl-C₁-C₆-alkyl,
C₁-C₆-Alkoxycarbonyl-C₂-C₆-alkenyl, wobei die Alkenylkette zusätzlich ein bis drei Halogen- und/oder Cyanoreste tragen kann,
Benzyl, welches in benachbarten Positionen des Phenylrings einen zweiwertigen Substituenten wie Methylendioxy, Difluormethylendioxy, Chorfluormethylendioxy, Dichlormethylendioxy tragen kann oder ein- bis fünffach durch Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₃-C₆-Cycloalkyl, C₁-C₄-Alkoxycarbonyl, C₁-C₄-Alkoxycarbonyl-C₁-C₄-alkyl, Hydroxy, Nitro oder Cyano substitituiert sein kann, oder
R⁴ und R⁵ zusammen mit dem Stickstoffatom, an das sie gebunden sind einen gesättigten oder ungesättigten 4- bis 7-gliedrigen Azaheterocyclus, der neben Kohlenstoffringgliedern gewünschtenfalls eines der folgenden Glieder enthalten kann: -O-, -S-, -N=, -NH- oder N-(C₁-C₆-Alkyl)-;
R⁷ Wasserstoff, C₁-C₆-Alkyl, C₁-C₆-Halogenalkyl, C₁-C₃-Alkoxy-C₁-C₃-alkyl, C₂-C₆-Cyanoalkyl, C₃-C₆-Alkenyl oder C₃-C₆-Alkinyl;

2. Substituierte Perhydrodiazine der allgemeinen Formel I gemäß Anspruch 1, worin die Variablen R¹ bis R⁷ die folgenden Bedeutungen haben:
R¹ Wasserstoff, C₁-C₆-Alkyl, C₁-C₆-Halogenalkyl, C₁-C₃-Alkoxy-C₁-C₃-alkyl, C₃-C₆-Alkenyl oder C₃-C₆-Alkinyl;
R² C₁-C₆-Alkyl, C₃-C₆-Cycloalkyl, C₃-C₆-Alkenyl, C₃-C₆-Alkinyl, C₁-C₆-Halogenalkyl, C₁-C₆-Alkoxy-C₂-C₆-alkyl, C₁-C₆-Alkoxycarbonyl-C₁-C₆-alkyl, C₁-C₆-Alkylthio-C₁-C₆-alkyl, C₃-C₆-Alkenyloxycarbonyl-C₁-C₆-alkyl, C₃-C₆-Alkinyloxycarbonyl-C₁-C₆-alkyl, C₃-C₆-Alkenyloxy-C₁-C₆-alkyl, C₃-C₆-Alkinyloxy-C₁-C₆-alkyl, C₁-C₆-Halogenalkoxy-C₁-C₆-alkyl, Cyano-C₁-C₆-alkyl, Halogen-C₂-C₆-alkenyl, Halogen-C₃-C₆-alkinyl, C₁-C₆-Alkylcarbonyl, CHR¹COR⁷, P(O)(OR⁷)₂, CHR¹P(S)(OR⁷)₂, CHR¹C(O)NR⁴R⁵, CHR¹C(O)NH₂,
Benzyl, Phenyl, Phenoxy-C₁-C₆-alkyl oder Benzyloxy-C₁-C₆-alkyl, wobei die vier letztgenannten Substituenten mit Halogen, C₁-C₄-Alkyl oder C₁-C₄-Halogenalkyl substituiert sein können;
R³ C₁-C₁₅-Alkyl, C₃-C₈-Cycloalkyl, C₂-C₁₀-Alkenyl, C₃-C₁₀-Alkinyl, C₁-C₆-Halogenalkyl, C₁-C₆-Alkoxy-C₁-C₆-alkyl, C₁-C₆-Alkylthioalkyl, C₁-C₆-Alkoxycarbonyl-C₁-C₆-alkyl, C₃-C₆-Alkenyloxycarbonyl-C₁-C₆-alkyl, C₃-C₆-Alkinyloxycarbonyl-C₁-C₆-alkyl, C₃-C₆-Alkenyloxy-C₁-C₆-alkyl, C₁-C₆-Halogenalkoxy-C₁-C₆-alkyl, Cyano-C₁-C₆-alkyl, Halogen-C₃-C₆-alkenyl, C₁-C₆-Alkoxy-C₃-C₆-alkenyl, C₁-C₆-Alkoxy-C₃-C₆-alkinyl, CHR¹COR⁷, P(O)(OR⁷)₂, CHR¹P(S)(OR⁷)₂, CHR¹C(O)NR⁴R⁵, CHR¹C(O)NH₂,
Benzyl, Phenyl, Phenoxy-C₁-C₆-alkyl oder Benzyloxy-C₁-C₆-alkyl, wobei die vier letztgenannten Substituenten mit Halogen, C₁-C₄-Alkyl oder C₁-C₄-Halogenalkyl substi-tuiert sein können;
R⁴ Wasserstoff, C₁-C₆-Alkyl oder C₁-C₆-Alkoxy;
R⁵, R⁶ unabhängig voneinander Wasserstoff, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy-C₁-C₆-alkyl, C₁-C₆-Alkoxycarbonyl-C₁-C₆-alkyl, C₁-C₆-Alkoxycarbonyl-C₂-C₆-alkenyl,
Benzyl, welche ein- bis fünffach durch Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxycarbonyl, C₁-C₄-Alkoxycarbonyl-C₁-C₄-alkyl, Nitro oder Cyano substituiert sein können, oder
R⁴ und R⁵ zusammen mit dem gemeinsamen Stickstoffatom für einen gesättigten oder ungesättigten 4- bis 7gliedrigen Azaheterocyclus stehen, der neben den Kohlenstoffringgliedern gewünschtenfalls eines der folgenden Glieder enthalten kann: -O-, -S-, -N=, -NH- oder -N(C₁-C₆-Alkyl)-;
R⁷ Wasserstoff, C₁-C₆-Alkyl, C₁-C₆-Halogenalkyl, C₁-C₃-Alkoxy-C₁-C₃-alkyl, C₂-C₆-Cyanoalkyl, C₃-C₆-Alkenyl oder C₃-C₆-Alkinyl;

3. Substituierte Perhydrodiazine der allgemeinen Formel I nach einem der vorhergehenden Ansprüche, worin R ausgewählt ist unter C(O)OR³, C(S)OR³, C(O)SR³, C(S)SR³ mit den für R³ genannten Bedeutungen.

4. Substituierte Perhydrodiazine der allgemeinen Formel I nach einem der vorhergehenden Ansprüche, worin Z für O steht und m den Wert 0 hat.

5. Verfahren zur Herstellung N-substituierter Perhydrodiazine der allgemeinen Formel I, wie in einem der vorhergehenden Ansprüche definiert, umfassend wenigstens zwei aufeinanderfolgende Reaktionsschritte, wobei man in einem ersten Reaktionsschritt substituierte Hydrazine der Formel II, 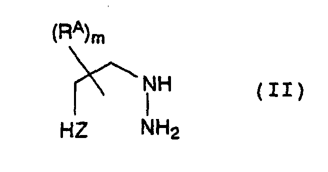 worin Z für 0 oder S steht und R^{A} und m die vorgenannte Bedeutung haben, mit einem Säurederivat der Formel III,
R-G (III)
worin R die vorgenannte Bedeutung hat und G eine nucleophil verdrängbare Abgangsgruppe bedeutet, oder R-G eine Verbindung der allgemeinen Formel R⁵-N=C=X oder der allgemeinen Formel R^{2a}(R^{2b})C=C=X bedeutet, worin X für O oder S steht, und R^{2a}(R^{2b})C-H für den Rest R² steht, wobei R² und R⁵ die obengenannten Bedeutungen aufweisen, umsetzt, und das so erhaltene Hydrazidderivat der Formel IV 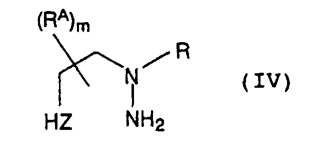 in einem zweiten Schritt mit Formaldehyd in Gegenwart einer Säure zu den substituierten Perhydrodiazinen der allgemeinen Formel I mit Z = O oder S cyclisiert, und gegebenenfalls in einem weiteren Reaktionsschritt für Z = S zu den Sulfoxiden Z = SO oder Sulfonen Z = SO₂ oxidiert oder die Reste R derivatisiert.

6. Verfahren zur Herstellung substituierter Harnstoffe der allgemeinen Formel V 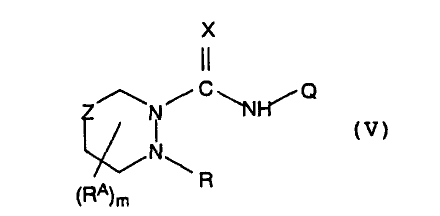 worin R, R^{A}, Z und m die in den Ansprüchen 1 bis 4 genannten Bedeutungen aufweisen,
X für O oder S steht, und
Q einen C₆-C₁₄-aromatischen Rest bedeutet, der wenigstens einfach substituiert ist und/oder über zwei benachbarte Kohlenstoffatome mit einen gesättigten oder ungesättigten 4- bis 7-gliedrigen Cyclus verbunden ist, der neben den Kohlenstoffringgliedern gewünschtenfalls eines, zwei oder drei der folgenden Glieder enthalten kann: -O-, -S-, -S(O)₂- -N=, -NH- oder -N(C₁-C₆-Alkyl)-;
**dadurch gekennzeichnet, daß** man ein N-substituiertes Perhydrodiazin der allgemeinen Formel I, wie in einem der vorhergehenden Ansprüche definiert, mit einem Isocycanat oder einem Isothiocycanat der allgemeinen Formel VI,
Q-N=C=X (VI)
worin Q und X die zuvor genannten Bedeutungen aufweisen, umsetzt.

## Claims

1. An N-substituted perhydro-3,4-diazine of the formula I 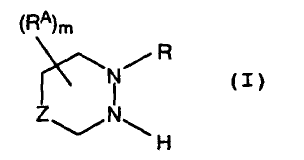 in which the variables Z, R, m and R^{A} are as defined below:
z is O, S, S=O or SO₂;
R^{A} is hydroxyl, CO₂R¹, halogen, cyano, C(O)NR¹₂, OR², C₁-C₆-alkyl, C₁-C₆-haloalkyl, C₃-C₆-alkenyl, C₃-C₆-alkynyl, COR¹, S(O)ₙR¹ where n= 0, 1 or 2, or C(O)SR¹;
R is CO₂H, CHO, CN, C(O)OR³, C(S)OR³, C(O)SR³, C(S)SR³, C(O)NR⁴R⁵, C(S)NR⁴R⁵, C(O)NHCO₂R⁶, C(O)NHSO₂R⁶, C(O)NHSO₃R⁶, C(O)R², P(O)R¹OR¹, P(O)(OR¹)₂, S(O)ₙR² where n = 0, 1, or 2 or SO₂NHR¹;
m has the value 0, 1, 2 or 3
and in which the variables R¹ to R⁷ are as defined below:
R¹ is hydrogen, C₁-C₆-alkyl, C₁-C₆-haloalkyl, C₁-C₃-alkoxy-C₁-C₃-alkyl, C₃-C₆-alkenyl or C₃-C₆-alkynyl;
R² is C₁-C₆-alkyl, C₃-C₆-cycloalkyl, C₃-C₆-alkenyl, C₃-C₆-alkynyl, C₁-C₆-haloalkyl, C₁-C₆-alkoxy-C₁-C₆-alkyl, hydroxycarbonyl-C₁-C₆-alkyl, C₁-C₆-alkoxycarbonyl-C₁-C₆-alkyl, C₁-C₆-alkylthio-C₁-C₆-alkyl, C₁-C₆-alkylsulfinyl-C₁-C₆-alkyl, C₁-C₆-alkylsulfonyl-C₁-C₆-alkyl, C₃-C₆-cycloalkyl-C₁-C₆-alkyl, C₃-C₆-alkenyloxycarbonyl-C₁-C₆-alkyl, C₃-C₆-alkynyloxycarbonyl-C₁-C₆-alkyl, C₃-C₆-cycloalkoxy-C₁-C₆-alkyl, C₃-C₆-alkenyloxy-C₁-C₆-alkyl, C₃-C₆-alkynyloxy-C₁-C₆-alkyl, C₁-C₆-haloalkoxy-C₁-C₆-alkyl, C₃-C₆-haloalkenyloxy-C₁-C₆-alkyl, C₃-C₆-haloalkynyloxy-C₁-C₆-alkyl, C₃-C₆-cycloalkyl-C₁-C₆-thioalkyl, C₃-C₆-alkenylthio-C₁-C₆-alkyl, C₃-C₆-alkynylthio-C₁-C₆-alkyl, cyano-C₁-C₆-alkyl, C₃-C₆-halocycloalkyl-C₁-C₆-alkyl, halo-C₂-C₆-alkenyl, C₁-C₆-alkoxy-C₃-C₆-alkenyl, C₁-C₆-haloalkoxy-C₃-C₆-alkenyl, C₁-C₆-alkylthio-C₃-C₆-alkenyl, C₃-C₆-haloalkynyl, C₁-C₆-alkoxy-C₃-C₆-alkynyl, C₁-C₆-haloalkoxy-C₃-C₆-alkynyl, C₁-C₆-alkylthio-C₃-C₆-alkynyl, C₁-C₆-alkylcarbonyl, CHR¹COR⁷, CHR¹P(O)(OR⁷)₂, P(O)(OR⁷)₂, CHR¹P(S)(OR⁷)₂, CHR¹C(O)NR⁴R⁵, CHR¹C(O)NH₂,
is phenyl, pyridyl, benzyl, phenoxy-C₁-C₆-alkyl, benzyloxy-C₁-C₆-alkyl, where the phenyl or pyridyl groups of the five last-mentioned substituents may be substituted by halogen, C₁-C₄-alkyl, C₁-C₄-alkoxy or C₁-C₄-haloalkyl,
R³ is C₁-C₁₅-alkyl, C₃-C₈-cycloalkyl, C₂-C₁₀-alkenyl, C₃-C₁₀-alkynyl, C₁-C₆-haloalkyl, C₁-C₆-alkoxy-C₁-C₆-alkyl, C₁-C₆-alkylthio-C₁-C₆-alkyl, C₁-C₆-alkylsulfinyl-C₁-C₆-alkyl, C₁-C₆-alkylsulfonyl-C₁-C₆-alkyl, C₁-C₃-alkoxy-C₁-C₃-alkoxy-C₁-C₃-alkyl, C₃-C₆-cycloalkyl-C₁-C₆-alkyl, carboxyl-C₁-C₆-alkyl, C₁-C₆-alkoxycarbonyl-C₁-C₆-alkyl, C₁-C₆-alkoxycarbonyl-C₂-C₆-alkenyl, C₃-C₆-alkenyloxycarbonyl-C₁-C₆-alkyl, C₃-C₆-alkynyloxycarbonyl-C₁-C₆-alkyl, C₃-C₈-cycloalkoxy-C₁-C₆-alkyl, C₃-C₆-alkenyloxy-C₁-C₆-alkyl, C₃-C₆-alkynyloxy-C₁-C₆-alkyl, C₁-C₆-haloalkoxy-C₁-C₆-alkyl C₃-C₆-haloalkenyloxy-C₁-C₆-alkyl, C₃-C₆-haloalkynyloxy-C₁-C₆-alkyl, C₃-C₆-cycloalkyl-C₁-C₆-thioalkyl, C₃-C₆-alkenylthio-C₁-C₆-alkyl, C₃-C₆-alkynylthio-C₁-C₆-alkyl, cyano-C₁-C₆-alkyl, C₃-C₆-halocycloalkyl-C₁-C₆-alkyl, halo-C₃-C₆-alkenyl, C₁-C₆-alkoxy-C₃-C₆-alkenyl, C₁-C₆-haloalkoxy-C₃-C₆-alkenyl, C₁-C₆-alkylthio-C₃-C₆-alkenyl, C₃-C₆-haloalkynyl, C₁-C₆-alkoxy-C₃-C₆-alkynyl, C₁-C₆-haloalkoxy-C₃-C₆-alkynyl, C₁-C₆-alkylthio-C₃-C₆-alkynyl,
is CHR¹COR⁷, CHR¹P(O)(OR⁷)₂, P(O)(OR⁷)₂, CHR¹P(S)(OR⁷)₂, CHR¹C(O)NR⁴R⁵, CHR¹C(O)NH₂, phenoxy-C₁-C₆-alkyl, benzyloxy-C₁-C₆-alkyl, where the phenyl groups of the two last-mentioned substituents may be substituted by halogen, C₁-C₄-alkyl, C₁-C₄-alkoxy or C₁-C₄-haloalkyl;
is phenyl, pyridyl, naphthyl, quinolyl, quinazolyl, quinoxalyl, 1-methylindolyl, 1-methylbenzimidazolyl, 2-methylindazolyl, benzofuranyl, benzothienyl, benzoxazolyl, benzthiazolyl, benzyl, where the 14 last-mentioned substituents may carry, in adjacent positions, a divalent substituent, such as methylenedioxy, difluoromethylenedioxy, chlorofluoromethylenedioxy, dichloromethylenedioxy, or may in each case be mono- to pentasubstituted by halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, amino, C₁-C₄-monoalkylamino, C₁-C₄-dialkylamino, C₃-C₆-cycloalkyl, C₁-C₄-alkoxycarbonyl, C₁-C₄-alkoxycarbonyl-C₁-C₄-alkyl, hydroxyl, nitro or cyano;
R⁴ is hydrogen, C₁-C₆-alkyl or C₁-C₆-alkoxy;
R⁵, R⁶ independently of one another are hydrogen, C₁-C₆-alkyl, C₂-C₆-alkenyl, C₂-C₆-alkynyl, C₃-C₆-cycloalkyl, C₁-C₆-haloalkyl, C₁-C₆-alkoxy-C₁-C₆-alkyl, C₁-C₆-alkoxycarbonyl-C₁-C₆-alkyl,
are C₁-C₆-alkoxycarbonyl-C₂-C₆-alkenyl, where the alkenyl chain may additionally carry one to three halogen and/or cyano radicals,
are benzyl which, in adjacent positions of the phenyl ring, may carry a divalent substituent, such as methylenedioxy, difluoromethylenedioxy, chlorofluoromethylenedioxy, dichloromethylenedioxy, or which may be mono- to pentasubstituted by halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₃-C₆-cycloalkyl, C₁-C₄-alkoxycarbonyl, C₁-C₄-alkoxycarbonyl-C₁-C₄-alkyl, hydroxyl, nitro or cyano, or
R⁴ and R⁵ together with the nitrogen atom to which they are attached are a saturated or unsaturated 4- to 7-membered azaheterocycle which, in addition to carbon ring members, may, if desired, contain one of the following members: -O-, -S-, -N=, -NH- or N-(C₁-C₆-alkyl)-;
R⁷ is hydrogen, C₁-C₆-alkyl, C₁-C₆-haloalkyl, C₁-C₃-alkoxy-C₁-C₃-alkyl, C₂-C₆-cyanoalkyl, C₃-C₆-alkenyl or C₃-C₆-alkynyl.

2. A substituted perhydrodiazine of the formula I as claimed in claim 1 in which the variables R¹ to R⁷ are as defined below:
R¹ is hydrogen, C₁-C₆-alkyl, C₁-C₆-haloalkyl, C₁-C₃-alkoxy-C₁-C₃-alkyl, C₃-C₆-alkenyl or C₃-C₆-alkynyl;
R² is C₁-C₆-alkyl, C₃-C₆-cycloalkyl, C₃-C₆-alkenyl, C₃-C₆-alkynyl, C₁-C₆-haloalkyl, C₁-C₆-alkoxy-C₂-C₆-alkyl, C₁-C₆-alkoxycarbonyl-C₁-C₆-alkyl, C₁-C₆-alkylthio-C₁-C₆-alkyl, C₃-C₆-alkenyloxycarbonyl-C₁-C₆-alkyl, C₃-C₆-alkynyloxycarbonyl-C₁-C₆-alkyl, C₃-C₆-alkenyloxy-C₁-C₆-alkyl, C₃-C₆-alkynyloxy-C₁-C₆-alkyl, C₁-C₆-haloalkoxy-C₁-C₆-alkyl, cyano-C₁-C₆-alkyl, halo-C₂-C₆-alkenyl, halo-C₃-C₆-alkynyl, C₁-C₆-alkylcarbonyl, CHR¹COR⁷, P(O)(OR⁷)₂, CHR¹P(S)(OR⁷)₂, CHR¹C(O)NR⁴R⁵, CHR¹C(O)NH₂,
is benzyl, phenyl, phenoxy-C₁-C₆-alkyl or benzyloxy-C₁-C₆-alkyl, where the four last-mentioned substituents may be substituted by halogen, C₁-C₄-alkyl or C₁-C₄-haloalkyl;
R³ is C₁-C₁₅-alkyl, C₃-C₈-cycloalkyl, C₂-C₁₀-alkenyl, C₃-C₁₀-alkynyl, C₁-C₆-haloalkyl, C₁-C₆-alkoxy-C₁-C₆-alkyl, C₁-C₆-alkylthioalkyl, C₁-C₆-alkoxycarbonyl-C₁-C₆-alkyl, C₃-C₆-alkenyloxycarbonyl-C₁-C₆-alkyl, C₃-C₆-alkynyloxycarbonyl-C₁-C₆-alkyl, C₃-C₆-alkenyloxy-C₁-C₆-alkyl, C₁-C₆-haloalkoxy-C₁-C₆-alkyl, cyano-C₁-C₆-alkyl, halo-C₃-C₆-alkenyl, C₁-C₆-alkoxy-C₃-C₆-alkenyl, C₁-C₆-alkoxy-C₃-C₆-alkynyl, CHR¹COR⁷, P(O)(OR⁷)₂, CHR¹P(S)(OR⁷)₂, CHR¹C(O)NR⁴R⁵, CHR¹C(O)NH₂,
is benzyl, phenyl, phenoxy-C₁-C₆-alkyl or benzyloxy-C₁-C₆-alkyl, where the four last-mentioned substituents may be substituted by halogen, C₁-C₄-alkyl or C₁-C₄-haloalkyl;
R⁴ is hydrogen, C₁-C₆-alkyl or C₁-C₆-alkoxy;
R⁵, R⁶ independently of one another are hydrogen, C₁-C₆-alkyl, C₂-C₆-alkenyl, C₂-C₆-alkynyl, C₃-C₆-cycloalkyl, C₁-C₆-haloalkyl, C₁-C₆-alkoxy-C₁-C₆-alkyl, C₁-C₆-alkoxycarbonyl-C₁-C₆-alkyl, C₁-C₆-alkoxycarbonyl-C₂-C₆-alkenyl,
are benzyl which may be mono- to pentasubstituted by halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkoxycarbonyl, C₁-C₄-alkoxycarbonyl-C₁-C₄-alkyl, nitro or cyano, or
R⁴ and R⁵ together with the common nitrogen atom are a saturated or unsaturated 4- to 7-membered azaheterocycle which, in addition to the carbon ring members may, if desired, contain one of the following members: -O-, -S-, -N=, -NH- or -N(C₁-C₆-alkyl)-;
R⁷ is hydrogen, C₁-C₆-alkyl, C₁-C₆-haloalkyl, C₁-C₃-alkoxy-C₁-C₃-alkyl, C₂-C₆-cyanoalkyl, C₃-C₆-alkenyl or C₃-C₆-alkynyl.

3. A substituted perhydrodiazine of the formula I as claimed in any of the preceding claims in which R is selected from C(O)OR³, C(S)OR³, C(O)SR³, C(S)SR³ where R³ is as defined.

4. A substituted perhydrodiazine of the formula I as claimed in any of the preceding claims in which Z is O and m has the value 0.

5. A process for preparing N-substituted perhydrodiazines of the formula I as defined in any of the preceding claims, which comprises at least two successive reaction steps, where, in a first reaction step, a substituted hydrazine of the formula II 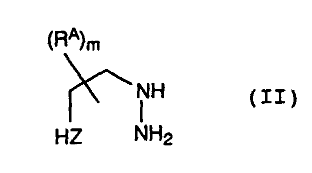 in which Z is O or S and R^{A} and m are as defined above is reacted with an acid derivative of the formula III
R-G (III)
in which R is as defined above and G is a nucleophilically displaceable leaving group, or R-G is a compound of the formula R⁵-N=C=X or the formula R^{2a}(R^{2b})C=C=X in which X is O or S and R^{2a}(R^{2b})C-H is the radical R², R² and R⁵ being as defined above, and the resulting hydrazide derivative of the formula IV 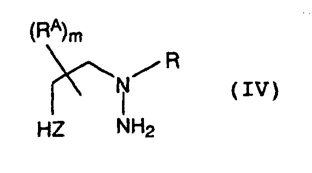 is, in a second step, cyclized with formaldehyde in the presence of an acid to give the substituted perhydrodiazines of the formula I where Z = O or S which are, if appropriate, in the case that Z = S, oxidized in a further reaction step to give the sulfoxide Z = SO or the sulfones Z = SO₂, or the radicals R are derivatized.

6. A process for preparing substituted ureas of the formula V 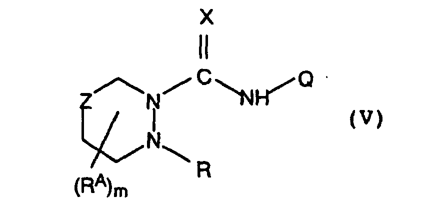 in which R, R^{A}, Z and m are as defined in claims 1 to 4,
X is O or S, and
Q is a C₆-C₁₄-aromatic radical which is at least monosubstituted and/or attached via two adjacent carbon atoms to a saturated or unsaturated 4- to 7-membered cycle which, in addition to the carbon ring members, may, if desired, contain one, two or three of the following members: -O-, -S-, -S(O)₂-, -N=, -NH- or -N(C₁-C₆-alkyl)-;
which comprises reacting a N-substituted perhydrodiazine of the formula I as defined in any of the preceding claims with an isocyanate or an isothiocyanate of the formula VI
Q-N=C=X (VI)
in which Q and X are as defined above.

## Revendications

1. Perhydro-3,4-diazines N-substituées répondant à la formule I : 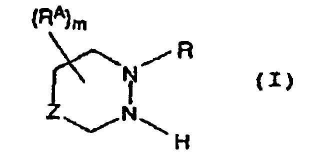 dans laquelle les variables Z, R, m et R^{A} ont les significations suivantes :
Z représente un groupe O, un groupe S, un groupe S=O, ou un groupe SO₂ ;
R^{A} représente un groupe hydroxy, un groupe CO₂R¹, un atome d'halogène, un groupe cyano, un groupe C(O)NR¹₂, un groupe OR², un groupe C₁-C₆-alkyle, un groupe C₁-C₆-halogénoalkyle, un groupe C₃-C₆-alcényle, un groupe C₃-C₆-alcinyle, un groupe COR¹, un groupe S(O)ₙR¹ avec n = 0, 1 ou 2, ou un groupe C(O)SR¹ ;
R représente un groupe CO₂H, un groupe CHO, un groupe CN, un groupe C(O)OR³, un groupe C(S)OR³, un groupe C(O)SR³, un groupe C(S)SR³, un groupe C(O)NR⁴R⁵, un groupe C(S)NR⁴R⁵, un groupe C(O)NHCO₂R⁶, un groupe C(O)NHSO₂R⁶, un groupe C(O)NHSO₃R⁶, un groupe C(O)R², un groupe P(O)R¹OR¹, un groupe P(O)(OR¹)₂, un groupe S(O)ₙR² avec n = 0, 1 ou 2, ou un groupe SO₂NHR¹ ;
m vaut 0,1, 2 ou 3 ;
et dans laquelle les variables R¹ à R⁷ ont les significations suivantes :
R¹ représente un atome d'hydrogène, un groupe C₁-C₆-alkyle, un groupe C₁-C₆-halogénoalkyle, un groupe C₃-C₆-alcoxy-C₃-C₆-alkyle, un groupe C₃-C₆-alcényle ou un groupe C₃-C₆-alcinyle ;
R² représente un groupe C₁-C₆-alkyle, un groupe C₃-C₆-cycloalkyle, un groupe C₃-C₆-alcényle, un groupe C₃-C₆-alcinyle, un groupe C₁-C₆-halogénoalkyle, un groupe C₁-C₆-alcoxy-C₁-C₆-alkyle, un groupe hydroxycarbonyl-C₁-C₆-alkyle, un groupe C₁-C₆-alcoxycarbonyl-C₁-C₆-alkyle, un groupe C₁-C₆-alkylthio-C₁-C₆-alkyle, un groupe C₁-C₆-alkylsulfinyl-C₁-C₆-alkyle, un groupe C₁-C₆-alkylsulfonyl-C₁-C₆-alkyle, un groupe C₃-C₆-cycloalkyle-C₁-C₆-alkyle, un groupe C₃-C₆-alcényloxycarbonyl-C₁-C₆-alkyle, un groupe C₃-C₆-alcinyloxycarbonyl-C₁-C₆-alkyle, un groupe C₃-C₆-cycloalcoxy-C₁-C₆-alkyle, un groupe C₃-C₆-alcényloxy-C₁-C₆-alkyle, un groupe C₃-C₆-alcinyloxy-C₁-C₆-alkyle, un groupe C₁-C₆-halogénoalcoxy-C₁-C₆-alkyle, un groupe C₃-C₆-halogénoalcényloxy-C₁-C₆-alkyle, un groupe C₃-C₆-halogénoalcinyloxy-C₁-C₆-alkyle, un groupe C₃-C₆-cycloalkyl-C₁-C₆-thioalkyle, un groupe C₃-C₆-alcénylthio-C₁-C₆-alkyle, un groupe C₃-C₆-alcinylthio-C₁-C₆-alkyle, un groupe cyano-C₁-C₆-alkyle, un groupe C₃-C₆-halogénocycloalkyl-C₁-C₆-alkyle, un groupe halogéno-C₂-C₆-alcényle, un groupe C₁-C₆-alcoxy-C₃-C₆-alcényle, un groupe C₁-C₆-halogénoalcoxy-C₃-C₆-alcényle, un groupe C₁-C₆-alkylthio-C₃-C₆-alcényle, un groupe C₃-C₆-halogénoalcinyle, un groupe C₁-C₆-alcoxy-C₃-C₆-alcinyle, un groupe C₁-C₆-haloalcoxy-C₃-C₆-alcinyle, un groupe C₁-C₆-alkylthio-C₃-C₆-alcinyle, un groupe C₁-C₆-alkylcarbonyle, un groupe CHR¹COR⁷, un groupe CHR¹P(O)(OR⁷)₂, un groupe P(O)(OR⁷)₂, un groupe CHR¹P(S)(OR⁷)₂, un groupe CHR¹C(O)NR⁴R⁵, un groupe CHR¹C(O)NH₂,
un groupe phényle, un groupe pyridyle, un groupe benzyle, un groupe phénoxy-C₁-C₆-alkyle, un groupe benzyloxy-C₁-C₆-alkyle, les groupes phényle et pyridyle des cinq derniers substituants cités pouvant être substitués par un atome d'halogène, un groupe C₁-C₄-alkyle, un groupe C₁-C₄-alcoxy ou un groupe C₁-C₄-halogénoalkyle,
R³ représente un groupe C₁-C₁₅-alkyle, un groupe C₃-C₈-cycloalkyle, un groupe C₂-C₁₀-alcényle, un groupe C₃-C₁₀-alcinyle, un groupe C₁-C₆-halogénoalkyle, un groupe C₁-C₆-alcoxy-C₁-C₆-alkyle, un groupe C₁-C₆-alkylthio-C₁-C₆-alkyle, un groupe C₁-C₆-alkylsulfinyl-C₁-C₆-alkyle, un groupe C₁-C₆-alkylsulfonyl-C₁-C₆-alkyle, un groupe C₁-C₃-alcoxy-C₁-C₃-alcoxy-C₁-C₃-alkyle, un groupe C₃-C₆-cycloalkyl-C₁-C₆-alkyle, un groupe carboxyl-C₁-C₆-alkyle, un groupe C₁-C₆-alcoxycarbonyl-C₁-C₆-alkyle, un groupe C₁-C₆-alcoxycarbonyl-C₂-C₆-alcényle, un groupe C₃-C₆-alcényloxycarbonyl-C₁-C₆-alkyle, un groupe C₃-C₆-alcinyloxycarbonyl-C₁-C₆-alkyle, un groupe C₃-C₈-cycloalcoxy-C₁-C₆-alkyle, un groupe C₃-C₆-alcényloxy-C₁-C₆-alkyle, un groupe C₃-C₆-alcinyloxy-C₁-C₆-alkyle, un groupe C₁-C₆-halogénoalcoxy-C₁-C₆-alkyle, un groupe C₃-C₆-halogénoalcényloxy-C₁-C₆-alkyle, un groupe C₃-C₆-halogénoalcinyloxy-C₁-C₆-alkyle, un groupe C₃-C₆-cycloalkyl-C₁-C₆-thioalkyle, un groupe C₃-C₆-alcénylthio-C₁-C₆-alkyle, un groupe C₃-C₆-alcinylthio-C₁-C₆-alkyle, un groupe cyano-C₁-C₆-alkyle, un groupe C₃-C₆-halogénocycloalkyl-C₁-C₆-alkyle, un groupe halogéno-C₃-C₆-alcényle, un groupe C₁-C₆-alcoxy-C₃-C₆-alcényle, un groupe C₁-C₆-halogénoalcoxy-C₃-C₆-alcényle, un groupe C₁-C₆-alkylthio-C₃-C₆-alcényle, un groupe C₃-C₆-halogénoalcinyle, un groupe C₁-C₆-alcoxy-C₃-C₆-alcinyle, un groupe C₁-C₆-halogénoalcoxy-C₃-C₆-alcinyle, un groupe C₁-C₆-alkylthio-C₃-C₆-alcinyle,
un groupe CHR¹COR⁷, un groupe CHR¹P(O)(OR⁷)₂, un groupe P(O)(OR⁷)₂, un groupe CHR¹P(S)(OR⁷)₂, un groupe CHR¹C(O)NR⁴R⁵, un groupe CHR¹C(O)NH₂, un groupe phénoxy-C₁-C₆-alkyle, un groupe benzyloxy-C₁-C₆-alkyle, les groupes phényle des deux demiers substituants cités pouvant être substitués par un atome d'halogène, un groupe C₁-C₄-alkyle, un groupe C₁-C₄-alcoxy ou un groupe C₁-C₄-halogénoalkyle ;
un groupe phényle, un groupe pyridyle, un groupe naphtyle, un groupe quinolyle, un groupe quinazolyle, un groupe quinoxalyle, un groupe 1-méthylindolyle, un groupe 1-méthylbenzimidazolyle, un groupe 2-méthylindazolyle, un groupe benzofuranyle, un groupe benzothiényle, un groupe benzoxazolyle, un groupe benzothiazolyle, un groupe benzyle, les 14 derniers substituants cités pouvant porter dans des positions voisines un substituant divalent comme un groupe méthylènedioxy, un groupe difluorométhylènedioxy, un groupe chlorofluorométhylènedioxy, un groupe dichlorométhylènedioxy ou pouvant être substitués chacun 1 à 5 fois par un atome d'halogène, un groupe C₁-C₄-alkyle, un groupe C₁-C₄-halogénoalkyle, un groupe C₁-C₄-alcoxy, un groupe C₁-C₄-halogénoalcoxy, un groupe C1-C4-alkylthio, un groupe C₁-C₄-halogénoalkylthio, un groupe amino, un groupe C₁-C₄-monoalkylamino, un groupe C₁-C₄-dialkylamino, un groupe C₃-C₆-cycloalkyle, un groupe C₁-C₄-alcoxycarbonyle, un groupe C₁-C₄-alcoxycarbonyle-C₁-C₄-alkyle, un groupe hydroxy, un groupe nitro ou un groupe cyano ;
R⁴ représente un atome d'hydrogène, un groupe C₁-C₆-alkyle ou un groupe C₁-C₆-alcoxy ;
R⁵, R⁶ représentent indépendamment l'un de l'autre un atome d'hydrogène, un groupe C₁-C₆-alkyle, un groupe C₂-C₆-alcényle, un groupe C₂-C₆-alcinyle, un groupe C₃-C₆-cycloalkyle, un groupe C₁-C₆-halogénoalkyle, un groupe C₁-C₆-alcoxy-C₁-C₆-alkyle, un groupe C₁-C₆-alcoxycarbonyl-C₁-C₆-alkyle ;
un groupe C₁-C₆-alcoxycarbonyl-C₂-C₆-alcényle, la chaîne alcényle pouvant porter, en outre, un à trois restes halogène et/ou cyano,
un groupe benzyle, qui, dans des positions voisines du noyau phényle, peut porter des substituants bivalents comme un groupe méthylènedioxy, un groupe difluorométhylènedioxy, un groupe chlorofluorométhylènedioxy, un groupe dichlorométhylénedioxy ou peut être substitué une à cinq fois par un atome d'halogène, un groupe C₁-C₄-alkyle, un groupe C₁-C₄-halogénoalkyle, un groupe C₁-C₄-alcoxy, un groupe C₁-C₄-halogénoalcoxy, un groupe C₁-C₄-alkylthio, un groupe C₁-C₄-halogénoalkylthio, un groupe C₃-C₆-cycloalkyle, un groupe C₁-C₄-alcoxycarbonyle, un groupe C₁-C₄-alcoxycarbonyl-C₁-C₄-alkyle, un groupe hydroxy, un groupe nitro ou un groupe cyano, ou
R⁴ et R⁵ représentent ensemble avec l'atome d'azote auquel ils sont liés un azahétérocycle à 4 à 7 membres saturé ou insaturé, qui peut contenir, si on le souhaite, à côté des membres du cycle carboné, l'un des membres suivants : -O-, -S-, -N=, -NH- ou N-( C₁-C₆- alkyle)- ;
R⁷ représente un atome d'hydrogène, un groupe C₁-C₆-alkyle, un groupe C1-C6-halogénoalkyle, un groupe C₁-C₃-alcoxy-C₁-C₃-alkyle, un groupe C₂-C₆-cyanoalkyle, un groupe C₃-C₆-alcényle ou un groupe C₃-C₆-alcinyle.

2. Perhydrodiazines substituées répondant à la formule générale 1 selon la revendication 1, dans lesquelles les variables R¹ à R⁷ ont les significations suivantes :
R¹ représente un atome d'hydrogène, un groupe C₁-C₆-alkyle, un groupe C₁-C₆-halogénoalkyle, un groupe C₁-C₃-alcoxy-C₁-C₃-alkyle, un groupe C₃-C₆-alcényle ou un groupe C₃-C₆-alcinyle ;
R² représente un groupe C₁-C₆-alkyle, un groupe C₃-C₆-cycloalkyle, un groupe C₃-C₆-alcényle, un groupe C₃-C₆-alcinyle, un groupe C₁-C₆-halogénoalkyle, un groupe C₁-C₆-alcoxy-C₂-C₆-alkyle, un groupe C₁-C₆-alcoxycarbonyl-C₁-C₆-alkyle, un groupe C₁-C₆-alkylthio-C₁-C₆-alkyle, un groupe C₃-C₆-alcényloxycarbonyl-C₁-C₆-alkyle, un groupe C₃-C₆-alcinyloxycarbonyl-C₁-C₆-alkyle, un groupe C₃-C₆-alcényloxy-C₁-C₆-alkyle, un groupe C₃-C₆-alcinyloxy-C₁-C₆-alkyle, un groupe C₁-C₆-halogénoalcoxy-C₁-C₆-alkyle, un groupe cyano-C₁-C₆-alkyle, un groupe halogéno-C₂-C₆-alcényle, un groupe halogéno-C₃-C₆-alcinyle, un groupe C₁-C₆-alkylcarbonyle, un groupe CHR¹COR⁷, un groupe P(O)(OR⁷)₂, un groupe CHR¹P(S)(OR⁷)₂, un groupe CHR¹C(O)NR⁴R⁵, un groupe CHR¹C(O)NH₂,
un groupe benzyle, un groupe phényle, un groupe phénoxy-C₁-C₆-alkyle ou un groupe benzyloxy-C₁-C₆-alkyle, les quatre derniers substituants cités pouvant être substitués par un atome d'halogène, un groupe C₁-C₄-alkyle ou un groupe C₁-C₄-halogénoalkyle ;
R³ représente un groupe C₁-C₁₅-alkyle, un groupe C₃-C₈-cycloalkyle, un groupe C₂-C₁₀-alcényle, un groupe C₃-C₁₀-alcinyle, un groupe C₁-C₆-halogénoalkyle, un groupe C₁-C₆-alcoxy-C₁-C₆-alkyle, un groupe C₁-C₆-alkylthioalkyle, un groupe C₁-C₆-alcoxycarbonyl-C₁-C₆-alkyle, un groupe C₃-C₆-alcényloxycarbonyl-C₁-C₆-alkyle, un groupe C₃-C₆-alcinyloxycarbonyl-C₁-C₆-alkyle, un groupe C₃-C₆-alcényloxy-C₁-C₆-alkyle, un groupe C₁-C₆-halogénoalcoxy-C₁-C₆-alkyle, un groupe cyano-C₁-C₆-alkyle, un groupe halogéno-C₃-C₆-alcényle, un groupe C₁-C₆-alcoxy-C₃-C₆-alcényle, un groupe C₁-C₆-alcoxy-C₃-C₆-alcinyle, un groupe CHR¹COR⁷, un groupe P(O)(OR⁷)₂, un groupe CHR¹P(S)(OR⁷)₂, un groupe CHR¹C(O)NR⁴R⁵, un groupe CHR¹C(O)NH₂,
un groupe benzyle, un groupe phényle, un groupe phénoxy-C₁-C₆-alkyle ou un groupe benzyloxy-C₁-C₆-alkyle, les quatre demiers substituants cités pouvant être substitués par un atome d'halogène, un groupe C₁-C₄-alkyle ou un groupe C₁-C₄-halogénoalkyle ;
R⁴ représente un atome d'hydrogène, un groupe C₁-C₆-alkyle ou un groupe C₁-C₆-alcoxy ;
R⁵, R⁶ représentent indépendamment l'un de l'autre un atome d'hydrogène, un groupe C₁-C₆-alkyle, un groupe C₂-C₆-alcényle, un groupe C₂-C₆-alcinyle, un groupe C₃-C₆-cycloalkyle, un groupe C₁-C₆-halogénoalkyle, un groupe C₁-C₆-alcoxy-C₁-C₆-alkyle, un groupe C₁-C₆-alcoxycarbonyl-C₁-C₆-alkyle, un groupe C₁-C₆-alcoxycarbonyl-C₂-C₆-alcényle,
un groupe benzyle, qui peut être substitué une à cinq fois par un atome d'halogène, un groupe C₁-C₄-alkyle, un groupe C₁-C₄-halogénoalkyle, un groupe C₁-C₄-alcoxy, un groupe C₁-C₄-halogénoalcoxy, un groupe C₁-C₄-alkylthio, un groupe C₁-C₄-halogénoalkylthio, un groupe C₁-C₄-alcoxycarbonyle, un groupe C₁-C₄-alcoxycarbonyl-C₁-C₄-alkyle, un groupe nitro ou un groupe cyano, ou
R⁴ et R⁵ représentent ensemble avec l'atome d'azote commun un azahétérocycle à 4 à 7 membres, saturé ou insaturé, qui peut contenir, à côté des membres du cycle carboné, si on le souhaite, l'un des membres suivants : -O-, -S-, -N=, -NH- ou -N (C₁-C₆-alkyle)- ;
R⁷ représente un atome d'hydrogène, un groupe C₁-C₆-alkyle, un groupe C₁-C₆-halogénoalkyle, un groupe C₁-C₃-alcoxy-C₁-C₃-alkyle, un groupe C₂-C₆-cyanoalkyle, un groupe C₃-C₆-alcényle ou un groupe C₃-C₆-alcinyle ;

3. Perhydrodiazines substituées répondant à la formule générale I selon l'une quelconque des revendications précédentes, dans lesquelles R est choisi parmi le groupe C(O)OR³, le groupe C(S)OR³, le groupe C(O)SR³, le groupe C(S)SR³ avec les significations indiquées pour R³.

4. Perhydrodiazines substituées répondant à la formule générale I selon l'une quelconque des revendications précédentes, dans lesquelles Z représente O et m vaut 0.

5. Procédé pour la préparation de perhydrodiazines N-substituées répondant à la formule générale I, selon l'une quelconque des revendications précédentes, comprenant au moins deux étapes de réaction successives, dans lequel, dans une première étape de réaction, on fait réagir des hydrazines substituées répondant à la formule II : 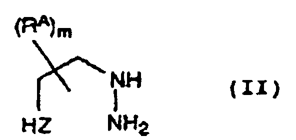 dans laquelle Z représente O ou S, et R^{A} et m ont les significations précitées, avec un dérivé acide répondant à la formule III
R - G (III)
dans laquelle R a la signification précitée et G représente un groupe partant déplaçable nucléophile, ou R-G représente une combinaison répondant à la formule générale R⁵ - N = C = X ou répondant à la formule générale R^{2A} R^{2A}(R^{2b}) C = C = X, dans laquelle X représente O ou S, et R^{2A}(R^{2B})C-H représente le reste R², R² et R⁵ présentant les significations précitées, et le dérivé hydrazide répondant à la formule IV ainsi obtenu : 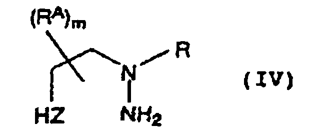 est cyclisé dans une deuxième étape avec du formaldéhyde en présence d'un acide en perhydrodiazines substituées répondant à la formule générale I avec Z = O ou S, et le cas échéant, oxydé dans une autre étape de réaction pour Z = S en sulfoxydes Z = SO ou sulfones Z = SO₂ ou les restes R sont dérivatisés.

6. Procédé pour la préparation d'urées substituées répondant à la formule générale V : 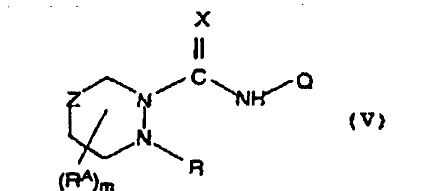 dans laquelle R, R^{A}, Z et m ont les significations indiquées dans les revendications 1 à 4,
X représente O ou S, et
Q représente un reste aromatique en C₆-C₁₄, qui est au moins une fois substitué et/ou est relié, par l'intermédiaire de deux atomes de carbone voisins, avec un cycle à 4 à 7 membres saturé ou insaturé, qui peut contenir, à côté des membres du cycle carboné, si on le souhaite, un, deux ou trois des membres suivants : -O-, -S-, -S(O)₂-, -N=, -NH- ou -N(C₁-C₆-alkyle)- ;
**caractérisé en ce que** l'on fait réagir une perhydrodiazine N-substituée répondant à la formule générale I, selon l'une quelconque des revendications précédentes, avec un isocyanate ou un isothiocyanate répondant à la formule générale VI,
Q-N=C=X (VI)
dans laquelle Q et X ont les significations indiquées précédemment.
